# EUROPEAN PATENT SPECIFICATION

(11) **EP 4 180 416 B1**
(45) Date of publication and mention of the grant of the patent: **27.11.2024**
(21) Application number: 21886831.3
(22) Date of filing: 28.10.2021
(51) Int. Cl.: C09K 11/06, C07C 211/61, C09D 7/63, H10K 85/60, C07C 217/94

(54) **NOVEL COMPOUND, COATING COMPOSITION COMPRISING SAME, ORGANIC LIGHT-EMITTING DEVICE USING SAME, AND MANUFACTURING METHOD THEREFOR**
NEUARTIGE VERBINDUNG, BESCHICHTUNGSZUSAMMENSETZUNG DAMIT, ORGANISCHE LICHTEMITTIERENDE VORRICHTUNG DAMIT UND HERSTELLUNGSVERFAHREN DAFÜR
NOUVEAU COMPOSÉ, COMPOSITION DE REVÊTEMENT LE COMPRENANT, DISPOSITIF ÉLECTROLUMINESCENT ORGANIQUE L'UTILISANT ET PROCÉDÉ DE FABRICATION

(30) Priority: 30.10.2020 KR 20200142781
(43) Date of publication of application: 17.05.2023
(73) Proprietor: LG Chem, Ltd., Seoul 07336 (KR)
(72) Inventor: SEO, Minseob, Daejeon 34122 (KR); LEE, Jiyoung, Daejeon 34122 (KR); LIM, Dowon, Daejeon 34122 (KR); CHOI, Doowhan, Daejeon 34122 (KR); KANG, Eunhye, Daejeon 34122 (KR); BAE, Jaesoon, Daejeon 34122 (KR); LEE, Jaechol, Daejeon 34122 (KR)
(74) Representative: Hoffmann Eitle
(86) International application number: PCT/KR2021/015282
(87) International publication number: WO 2022/092837

(56) References cited:
- WO-A1-2020/162156
- WO-A1-2020/162156
- JP-A- 2018 194 794
- KR-A- 20080 111 968
- KR-A- 20080 111 968
- KR-A- 20130 101 830
- KR-A- 20190 035 513
- US-A1- 2019 338 140

## Description

### [Technical Field]

The present disclosure claims the benefit of the filing date of Korean Patent No. 10-2020-0142781 filed with the Korean Intellectual Property Office on October 30, 2020, the entire contents of which are included in the present disclosure.

The present disclosure relates to a novel compound, a coating composition comprising the compound, an organic light emitting device formed using the coating composition, and a method for manufacturing the organic light emitting device.

### [Background Art]

The organic light emitting phenomenon is one of examples in which electric current is converted into visible light by an internal process of a specific organic molecule. The principle of the organic light emitting phenomenon is as follows. If a current is applied between the two electrodes when an organic material layer is positioned between an anode and a cathode, electrons and holes are respectively injected into the organic material layer from the cathode and the anode. The electrons and holes injected into the organic material layer recombine to form excitons, and light is emitted as these excitons are falling back to the ground state. An organic light emitting device using such a principle may generally comprise a cathode, an anode, and an organic material layer disposed therebetween, for example, a hole injection layer, a hole transport layer, a light emitting layer, an electron injection layer, and an electron transport layer.

Conventionally, a deposition process has been mainly used in order to manufacture an organic light emitting device. However, when manufacturing an organic light emitting device by the deposition process, there are problems in that a lot of material loss occurs and it is difficult to manufacture a device having a large area. In order to solve these problems, a device using a solution process is being developed.

Therefore, development of materials for the solution process is required.

(Patent Document 1) Korean Patent Publication No. 10-2017-089095.

US 2019/338140 A1 (SHIN HYEONAH [KR] ET AL) 7 November 2019 (2019-11-07) discloses compounds similar to Chemical Formula 1 of the present application.

### [Disclosure]

### [Technical Problem]

An object of the present disclosure is to provide a novel compound, a coating composition comprising the compound, an organic light emitting device formed using the coating composition, and a method for manufacturing the organic light emitting device.

### [Technical Solution]

A compound represented by Chemical Formula 1 below is provided. 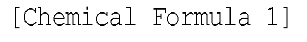 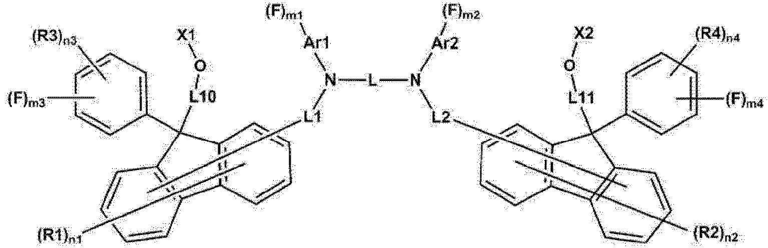 In Chemical Formula 1 above,
L is a substituted or unsubstituted divalent hydrocarbon ring group; or a substituted or unsubstituted divalent heterocyclic group,
L1 and L2 are the same as or different from each other, and are each independently a direct bond; a substituted or unsubstituted arylene group; or a substituted or unsubstituted heteroarylene group,
Ar1 and Ar2 are the same as or different from each other, and are each independently a substituted or unsubstituted aryl group; or a substituted or unsubstituted heteroaryl group,
L10 and L11 are the same as or different from each other, and are each independently a substituted or unsubstituted arylene group,
X1 and X2 are the same as or different from each other, and are each independently a curable group,
R1 to R4 are the same as or different from each other, and are each independently hydrogen; heavy hydrogen; a halogen group; a substituted or unsubstituted alkyl group; a substituted or unsubstituted alkoxy group; a substituted or unsubstituted aryl group; or a substituted or unsubstituted heteroaryl group,
n1 and n2 are each independently an integer of 0 to 7, and when n₁ and n₂ are each 2 or more, the substituents in two or more parentheses are the same as or different from each other,
m1 is 1 to the number of bonding positions at which a substituent of fluorine (F) can be bonded to Ar₁,
m2 is 1 to the number of bonding positions at which a substituent of fluorine (F) can be bonded to Ar₂,
n3 and n4 are each independently an integer of 0 to 4, and when n₃ and n₄ are each 2 or more, the substituents in two or more parentheses are the same as or different from each other,
m3 and m4 are each independently an integer of 1 to 5, n₃+m₃ is 5 or less, and n₄+m₄ is 5 or less.

The present disclosure provides a coating composition comprising the compound.

The present disclosure also provides an organic light emitting device comprising: a first electrode; a second electrode; and an organic material layer consisting of one or more layers provided between the first electrode and the second electrode, wherein one or more layers of the organic material layer contain the above-described coating composition or a cured product thereof.

Finally, the present disclosure provides a method for manufacturing an organic light emitting device, the method comprising the steps of: preparing a first electrode; forming an organic material layer consisting of one or more layers on the first electrode; and forming a second electrode on the organic material layer consisting of one or more layers, wherein the step of forming an organic material layer consisting of one or more layers comprises a step of forming one or more layers using the coating composition.

### [Advantageous Effects]

The compound of Chemical Formula 1 of the present disclosure can be used as a material for an organic material layer of an organic light emitting device, can obtain a device having a low driving voltage and excellent luminous efficiency and lifespan characteristics, and enables a large area of the device by enabling a solution process.

Further, since the compound of the present disclosure has excellent hole-mobility by containing four or more fluoro groups (-F) at a specific position, when it is applied to a device, a device having low voltage, high efficiency, and long lifespan characteristics can be obtained.

In addition, when the organic material layer of an upper layer part is formed through a solution process after forming an organic material layer containing the compound of Chemical Formula 1 of the present disclosure, the organic material layer containing the compound of Chemical Formula 1 above is not dissolved in a solution process solvent of the upper layer part so that there is an advantage in that the performance of the device is not deteriorated.

### [Description of Drawings]

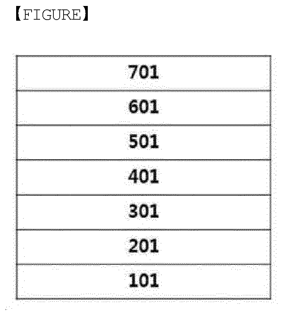

FIG. 1 shows an example of an organic light emitting device according to an embodiment of the present disclosure.

### <Reference Numeral>

101: Substrate
201: First Electrode
301: Hole Injection Layer
401: Hole Transport Layer
501: Light Emitting Layer
601: Electron Transport and Injection Layer
701: Second Electrode

### [Best Mode for Carrying Out the Invention]

Hereinafter, the present disclosure will be described in detail.

In the present disclosure, when a member (layer) is said to be located "on" other member (layer), this not only includes a case where a member (layer) is in contact with the other member, but also includes a case where another member (layer) exists between the two members (layers).

In the present disclosure, when a part "includes" a certain component, this means that other components may be further included, rather than excluding other components, unless there's a particularly opposing statement.

In the present disclosure, the term "curable group" may refer to a reactive substituent that cross-links compounds by exposing them to heat and/or light. Crosslinking may be generated by connecting radicals produced while decomposing carbon-carbon multiple bonds or cyclic structures by heat treatment or light irradiation.

Hereinafter, the substituents of the present disclosure will be described in detail.

In the present disclosure, "-----" refers to a site bonded to other substituent or bonding moiety.

In the present disclosure, the term "substitution" means that the hydrogen atom bonded to the carbon atom of the compound is changed to another substituent, and the position to be substituted is not limited if the position is a position at which the hydrogen atom is substituted, that is, a position where the substituent is substitutable. When two or more substituents are substituted, the two or more substituents may be the same as or different from each other.

In the present disclosure, the term "substituted or unsubstituted" means that it is substituted with one or more substituents selected from the group consisting of heavy hydrogen, a halogen group, a nitrile group, an alkyl group, a cycloalkyl group, an alkoxy group, an aryl group, and a heteroaryl group, or it is substituted with a substituent to which two or more substituents among the substituents exemplified above are connected, or unsubstituted. For example, "a substituent to which two or more substituents are connected" may be a biphenyl group. That is, the biphenyl group may be an aryl group, and may be interpreted as a substituent to which two phenyl groups are connected.

Further, in the present disclosure, the term "substituted or unsubstituted" means that it is substituted with one or more substituents selected from the group consisting of heavy hydrogen, a halogen group, a nitrile group, an alkyl group having 1 to 20 carbon atoms, a cycloalkyl group having 3 to 30 carbon atoms, an alkoxy group having 1 to 20 carbon atoms, an aryl group having 6 to 60 carbon atoms, and a heteroaryl group having 2 to 60 carbon atoms, or it is substituted with a substituent to which two or more substituents among the substituents exemplified above are connected, or unsubstituted.

In the present disclosure, the halogen group is a fluoro group (-F), a chloro group (-Cl), a bromo group (-Br) or an iodo group (-I).

In the present disclosure, the alkyl group may be linear or branched, and the number of carbon atoms is not particularly limited, but may be 1 to 20. According to another embodiment, the alkyl group has 1 to 10 carbon atoms. Specific examples of the alkyl group may include a methyl group, an ethyl group, an n-propyl group, an isopropyl group, an n-butyl group, an isobutyl group, a tert-butyl group, an n-pentyl group, an n-hexyl group, an n-heptyl group, etc., but the present disclosure is not limited thereto.

In the present disclosure, the cycloalkyl group is not particularly limited, but may have 3 to 60 carbon atoms, and according to an embodiment, the cycloalkyl group has 3 to 30 carbon atoms. According to another embodiment, the cycloalkyl group has 3 to 20 carbon atoms. Specific examples of the cycloalkyl group may include a cyclopropyl group, a cyclobutyl group, a cyclopentyl group, a cyclohexyl group, a cycloheptyl group, a cyclooctyl group, etc., but the present disclosure is not limited thereto.

In the present disclosure, the alkoxy group may be a straight chain or branched chain. The number of carbon atoms of the alkoxy group is not particularly limited, but may be 1 to 20. Specific examples of the alkoxy group may include a methoxy group, an ethoxy group, an n-propoxy group, an n-butoxy group, a tert-butoxy group, an n-pentyloxy group, an n-hexyloxy group, an n-octyloxy group, an n-nonyloxy group, an n-decyloxy group, etc., but the present disclosure is not limited thereto.

In the present disclosure, the aryl group is not particularly limited, but may have 6 to 60 carbon atoms, and may be a monocyclic aryl group or a polycyclic aryl group. According to an embodiment, the aryl group has 6 to 30 carbon atoms. According to an embodiment, the aryl group has 6 to 20 carbon atoms. The aryl group may include a phenyl group, a biphenyl group, a terphenyl group, or the like as a monocyclic aryl group, but the present disclosure is not limited thereto. The aryl group may include a naphthyl group, an anthracenyl group, a phenanthrenyl group, a pyrenyl group, a perylenyl group, a triphenylenyl group, a chrysenyl group, a fluorenyl group, or the like as a polycyclic aryl group, but the present disclosure is not limited thereto.

In the present disclosure, the fluorenyl group may be substituted, and two substituents may be bonded to each other to form a spiro structure.

When the fluorenyl group is substituted, the substituents may include substituted fluorenyl groups including a spirofluorenyl group such as 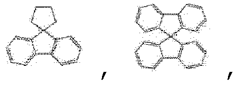 or the like, a 9,9-dimethylfluorenyl group 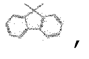 a 9,9-diphenylfluorenyl group 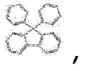 etc., but the present disclosure is not limited thereto.

In the present disclosure, the heterocyclic group is an aromatic ring group, an aliphatic ring group, or a condensed ring group thereof, containing one or more of N, O, P, and S as a heteroatom. The number of carbon atoms of the heterocyclic group is not particularly limited, but may be 2 to 60.

In the present disclosure, the heteroaryl group is an aromatic ring group containing one or more of N, O, P, and S as a heteroatom, and the number of carbon atoms of the heteroaryl group is not particularly limited, but may be 2 to 60. According to an embodiment, the heteroaryl group has 2 to 30 carbon atoms. Examples of the heteroaryl group may include a pyridine group, a pyrrole group, a pyrimidine group, a pyridazine group, a furan group, a thiophene group, a benzothiophene group, a benzofuran group, a dibenzothiophene group, a dibenzofuran group, a carbazole group, etc., but the present disclosure is not limited thereto.

In the present disclosure, the hydrocarbon ring group may be an aromatic ring group, an aliphatic ring group, or a condensed ring group thereof.

In the present disclosure, the description of the above-described aryl group may be applied to the aromatic hydrocarbon ring group.

In the present disclosure, the description of the above-described cycloalkyl group may be applied to the aliphatic hydrocarbon ring group.

In the present disclosure, the contents of the above-described aryl group are applied except that the arylene group is divalent.

In the present disclosure, the contents of the above-described heteroaryl group are applied except that the heteroarylene group is divalent.

In the present disclosure, "C_{Y1-Y2}" means "carbon number of Y1 to Y2".

According to an embodiment of the present disclosure, there is provided a compound represented by Chemical Formula 1 above.

The compound represented by Chemical Formula 1 above contains a fluoro group (-F) in the substituents Ar₁ and Ar₂ respectively so that the formation of radicals is inhibited at the positions of Ar₁ and Ar₂, thereby having high stability of the compound and high HOMO (highest occupied molecular orbital) energy level values. Due to this, according to an embodiment of the present disclosure, when the compound of Chemical Formula 1 above is contained in the hole injection layer within the organic light emitting device, it facilitates hole injection from the hole injection layer to the hole transport layer, thereby having a major effect on improving the lifespan of the device.

In the present disclosure, "energy level" means an energy magnitude. Therefore, the energy level is interpreted to mean an absolute value of the corresponding energy value. For example, a deep energy level means that the absolute value increases in the negative direction from the vacuum level.

In an embodiment of the present disclosure, the compound of Chemical Formula 1 above is preferably compounds having solubility in a suitable organic solvent.

Further, in the case of a compound according to an embodiment of the present disclosure, an organic light emitting device can be manufactured by a solution application method, thereby enabling the device to have a large area.

In an embodiment of the present disclosure, F in Chemical Formula 1 above means a fluoro group.

In the present disclosure, X1 and X2 are the same as or different from each other, and are each independently a curable group.

According to an embodiment of the present disclosure, the curable group is any one selected from structures below.

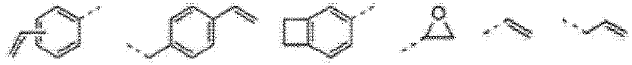

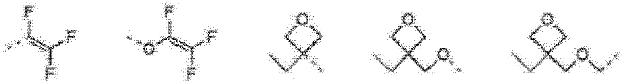

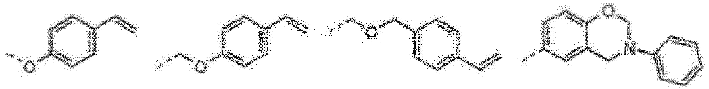

According to an embodiment of the present disclosure, X1 and X2 are the same as or different from each other, and are each independently 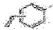 or

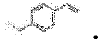

In an embodiment of the present disclosure, Chemical Formula 1 above is represented by Chemical Formula 2 below. 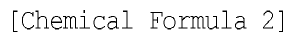 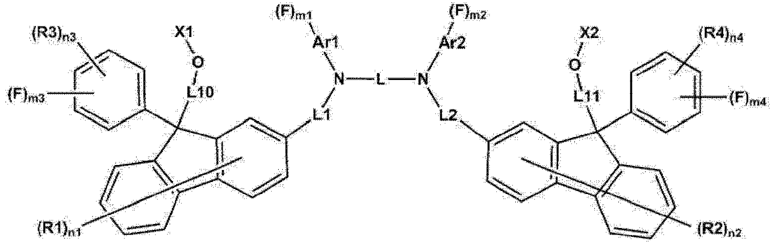 In Chemical Formula 2 above,
L, L1, L2, Ar1, Ar2, L10, L11, X1, X2, R1 to R4, n1 to n4, and m1 to m₄ are defined as in Chemical Formula 1.

In an embodiment of the present disclosure, L10 and L11 are the same as or different from each other, and are each independently a substituted or unsubstituted C₆-C₆₀ arylene group.

In another embodiment, L10 and L11 are the same as or different from each other, and are each independently a substituted or unsubstituted C₆-C₃₀ arylene group.

According to another embodiment, L10 and L11 are the same as or different from each other, and are each independently a substituted or unsubstituted phenylene group.

According to another embodiment, L10 and L11 are each a phenylene group.

In an embodiment of the present disclosure, Chemical Formula 1 above is represented by Chemical Formula 3 below. 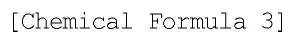 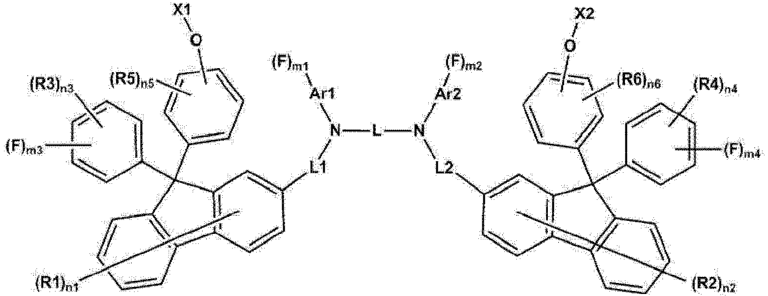 In Chemical Formula 3 above,
L, L1, L2, Ar1, Ar2, X1, X2, R1 to R4, n1 to n4, and m1 to m4 are defined as in Chemical Formula 1,
R5 and R6 are the same as or different from each other, and are each independently hydrogen; heavy hydrogen; a halogen group; a substituted or unsubstituted alkyl group; a substituted or unsubstituted alkoxy group; a substituted or unsubstituted aryl group; or a substituted or unsubstituted heteroaryl group,
n₅ and n₆ are each an integer of 0 to 4, and when n₅ and n₆ are each 2 or more, the substituents in two or more parentheses are the same as or different from each other.

According to an embodiment of the present disclosure, R₅ and R₆ are the same as or different from each other, and are each independently hydrogen; heavy hydrogen; a halogen group; a substituted or unsubstituted C₁-C₂₀ alkyl group; a substituted or unsubstituted C₁-C₂₀ alkoxy group; a substituted or unsubstituted C₆-C₃₀ aryl group; or a substituted or unsubstituted C₂-C₃₀ heteroaryl group.

In another embodiment, R5 and R6 are the same as or different from each other, and are each independently hydrogen; heavy hydrogen; a halogen group; a C₁-C₂₀ alkyl group; a C₁-C₂₀ alkoxy group; a C₆-C₃₀ aryl group; or a C₂-C₃₀ heteroaryl group.

In another embodiment, R5 and R6 are each hydrogen.

In an embodiment of the present disclosure, n5 is an integer of 0 to 4, and when n5 is 2 or more, 2 or more R5 are the same as or different from each other.

In an embodiment of the present disclosure, n6 is an integer of 0 to 4, and when n6 is 2 or more, 2 or more R6 are the same as or different from each other.

According to an embodiment of the present disclosure, n₅ and n₆ are each independently 0 or 1.

In an embodiment of the present disclosure, L is a substituted or unsubstituted C₆-C₆₀ divalent hydrocarbon ring group; or a substituted or unsubstituted C₂-C₆₀ divalent heterocyclic group.

In another embodiment, L is a substituted or unsubstituted C₆-C₃₀ divalent hydrocarbon ring group; or a substituted or unsubstituted C₂-C₃₀ divalent heterocyclic group.

According to another embodiment, L is a C₆-C₃₀ divalent hydrocarbon ring group unsubstituted or substituted with a C₁-C₂₀ alkyl group.

In an embodiment of the present disclosure, L is any one selected from structures below.

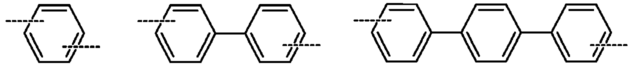

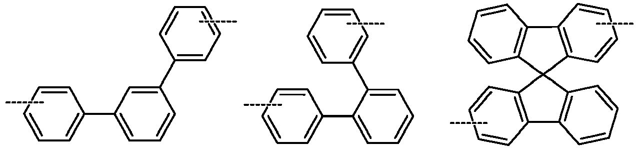

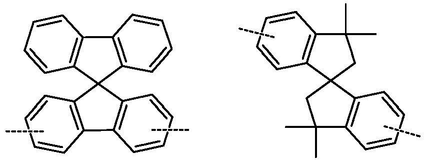

The structures are unsubstituted or substituted with one or more substituents selected from the group consisting of: heavy hydrogen; a halogen group; a substituted or unsubstituted alkyl group; a substituted or unsubstituted alkoxy group; a substituted or unsubstituted aryl group; and a substituted or unsubstituted heteroaryl group.

In the above structures, ... denotes a bonding position.

According to an embodiment of the present disclosure, the structures are unsubstituted or substituted with one or more substituents selected from the group consisting of: heavy hydrogen; a halogen group; a substituted or unsubstituted C₁-C₂₀ alkyl group; a substituted or unsubstituted C₁-C₂₀ alkoxy group; a substituted or unsubstituted C₆-C₃₀ aryl group; and a substituted or unsubstituted C₂-C₃₀ heteroaryl group.

In another embodiment, the structures are unsubstituted or substituted with a substituted or unsubstituted C₁-C₂₀ alkyl group.

According to another embodiment, the structures are unsubstituted or substituted with a substituted or unsubstituted C₁-C₁₀ alkyl group.

In another embodiment, the structures are unsubstituted or substituted with a methyl group.

In an embodiment of the present disclosure, L1 and L2 are the same as or different from each other, and are each independently a direct bond; a substituted or unsubstituted C₆-C₆₀ arylene group; or a substituted or unsubstituted C₂-C₆₀ heteroarylene group.

In another embodiment, L1 and L2 are each a direct bond.

According to an embodiment of the present disclosure, Ar1 and Ar2 are the same as or different from each other, and are each independently a substituted or unsubstituted C₆-C₆₀ aryl group; or a substituted or unsubstituted C₂-C₆₀ heteroaryl group.

According to another embodiment, Ar1 and Ar2 are the same as or different from each other, and are each independently a substituted or unsubstituted C₆-C₃₀ aryl group; or a substituted or unsubstituted C₂-C₃₀ heteroaryl group.

According to another embodiment, Ar1 and Ar2 are the same as or different from each other, and are each independently a C₆-C₃₀ aryl group unsubstituted substituted with a C₁-C₂₀ alkyl group.

In another embodiment, Ar1 and Ar2 are the same as or different from each other, and are each independently a substituted or unsubstituted phenyl group; a substituted or unsubstituted biphenyl group; or a substituted or unsubstituted terphenyl group.

According to another embodiment, Ar1 and Ar2 are the same as or different from each other, and are each independently a phenyl group unsubstituted or substituted with a C₁-C₂₀ alkyl group; a biphenyl group unsubstituted or substituted with a C₁-C₂₀ alkyl group; or a terphenyl group unsubstituted or substituted with a C₁-C₂₀ alkyl group.

In another embodiment, Ar1 and Ar2 are the same as or different from each other, and are each independently a phenyl group unsubstituted or substituted with a C₁-C₁₀ alkyl group; a biphenyl group unsubstituted or substituted with a C₁-C₁₀ alkyl group; or a terphenyl group unsubstituted or substituted with a C₁-C₁₀ alkyl group.

According to another embodiment, Ar1 and Ar2 are the same as or different from each other, and are each independently a phenyl group unsubstituted or substituted with a methyl group; a biphenyl group unsubstituted or substituted with a methyl group; or a terphenyl group unsubstituted or substituted with a methyl group.

According to an embodiment of the present disclosure, R1 to R4 are the same as or different from each other, and are each independently hydrogen; heavy hydrogen; a halogen group; a substituted or unsubstituted C₁-C₂₀ alkyl group; a substituted or unsubstituted C₁-C₂₀ alkoxy group; a substituted or unsubstituted C₆-C₃₀ aryl group; or a substituted or unsubstituted C₂-C₃₀ heteroaryl group.

In another embodiment, R1 to R4 are each hydrogen.

In an embodiment of the present disclosure, n₁ and n₂ are each independently 0 or 1.

According to an embodiment of the present disclosure, m₁ is 1 to the number of bonding positions at which a substituent of fluorine (F) may be bonded to Ar1, and m2 is 1 to the number of bonding positions at which a substituent of fluorine (F) may be bonded to Ar2. At this time, the number of bonding positions to which the substituent may be bonded means up to 5 to which hydrogen is bonded when Ar1 or Ar2 is a phenyl group, and up to 9 to which hydrogen is bonded when Ar1 or Ar2 is a biphenyl group.

In an embodiment of the present disclosure, m1 and m2 are each independently an integer of 1 to 5.

According to another embodiment, m1 is 1.

According to another embodiment, m1 is 2.

According to another embodiment, m1 is 3.

According to another embodiment, m1 is 4.

According to another embodiment, m1 is 5.

According to another embodiment, m2 is 1.

According to another embodiment, m2 is 2.

According to another embodiment, m2 is 3.

According to another embodiment, m2 is 4.

According to another embodiment, m2 is 5.

According to an embodiment of the present disclosure, m3 and m4 are each independently an integer of 1 to 5, n₃ and n₄ are each independently an integer of 0 to 4, n3+m3 is 5 or less, and n4+m4 is 5 or less.

In an embodiment of the present disclosure, when n3 and n4 are each 2 or more, the substituents in two or more parentheses are the same or different from each other.

According to another embodiment, m3 is 1.

According to another embodiment, m3 is 2.

According to another embodiment, m3 is 3.

According to another embodiment, m3 is 4.

According to another embodiment, m3 is 5.

According to another embodiment, m4 is 1.

According to another embodiment, m4 is 2.

According to another embodiment, m4 is 3.

According to another embodiment, m4 is 4.

According to another embodiment, m4 is 5.

In an embodiment of the present disclosure, Chemical Formula 1 above may be represented by any one of compounds below.

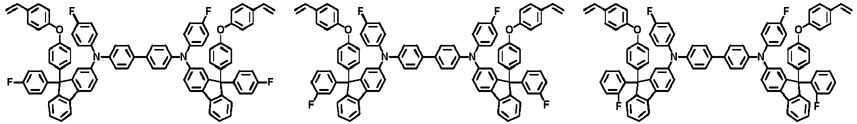

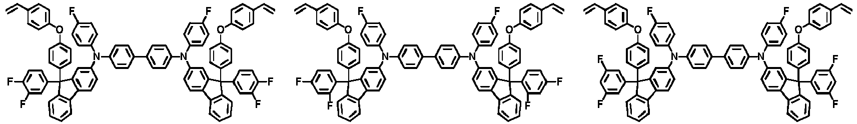

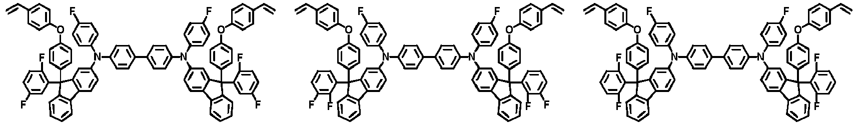

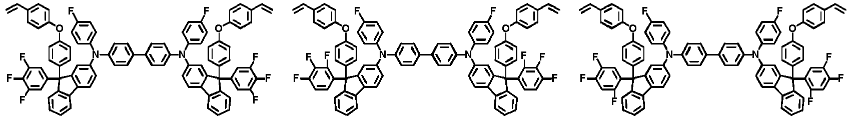

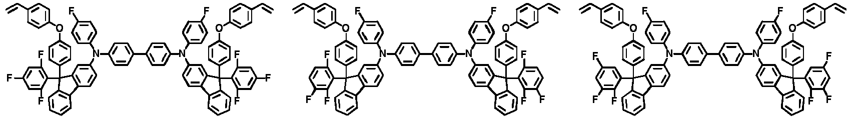

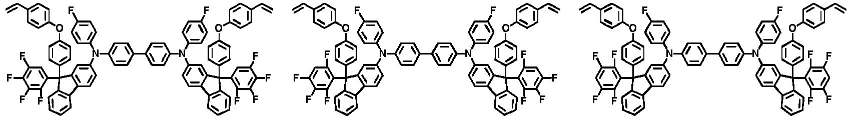

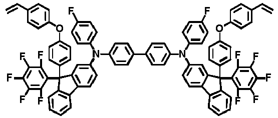

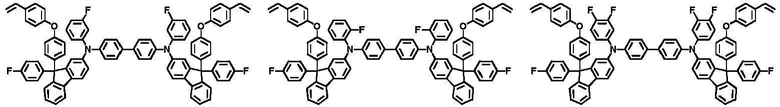

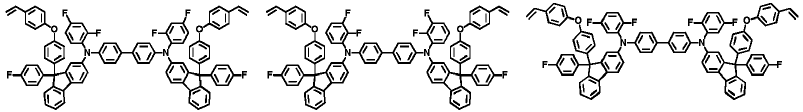

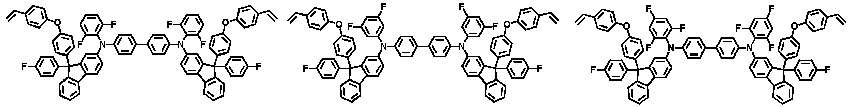

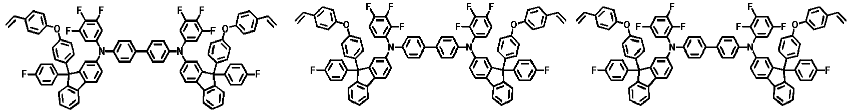

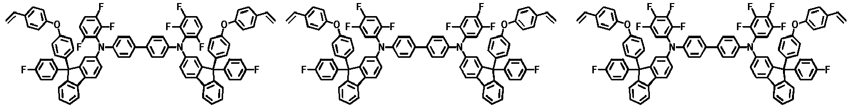

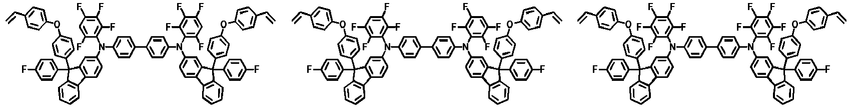

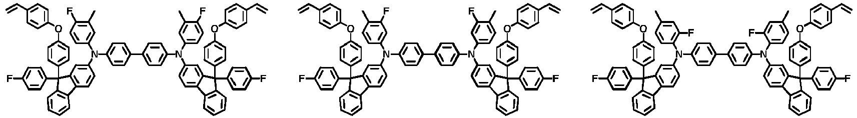

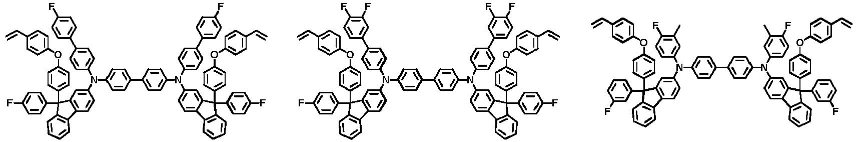

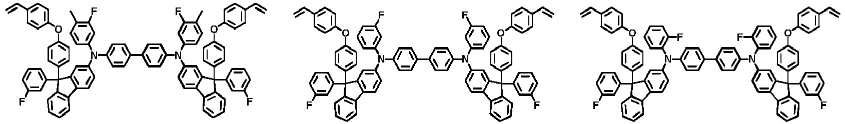

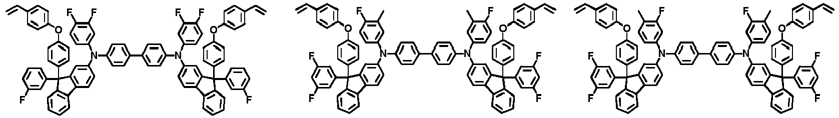

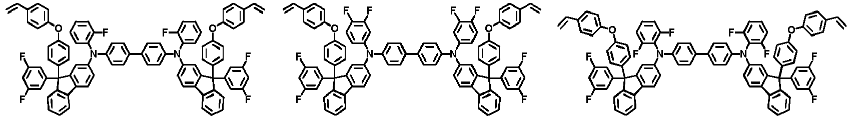

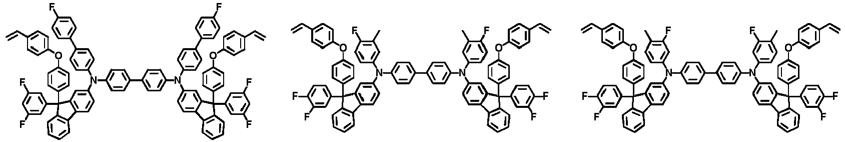

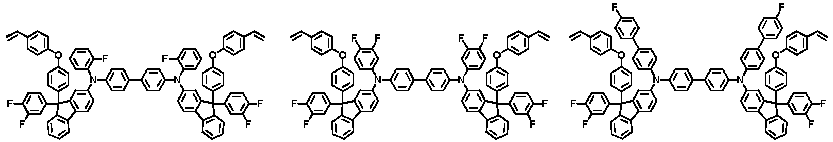

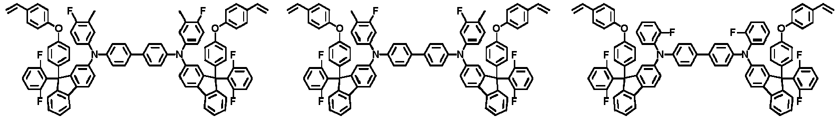

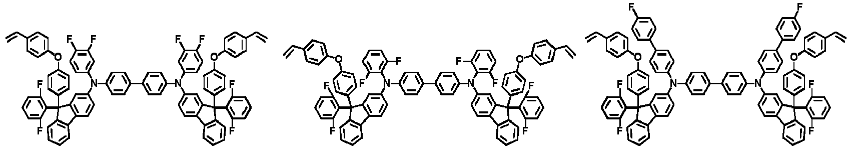

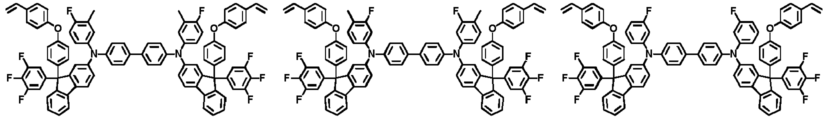

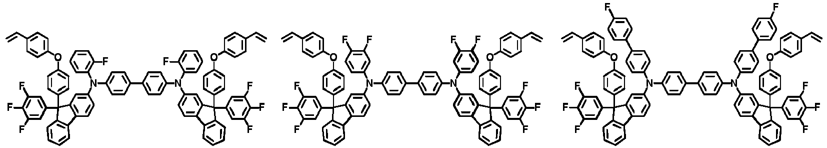

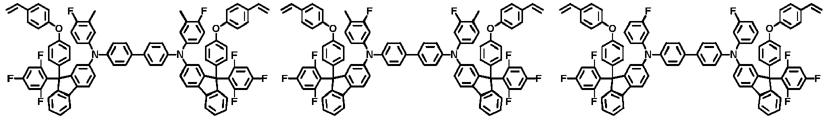

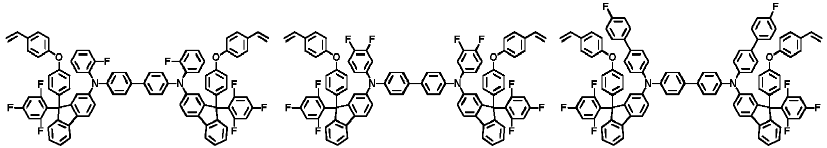

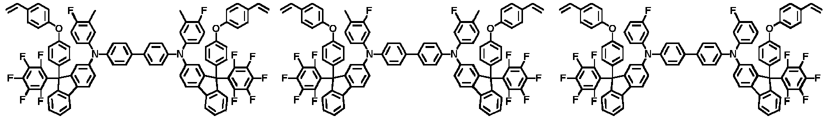

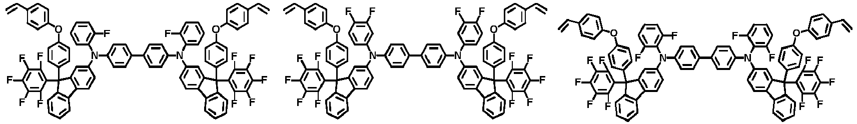

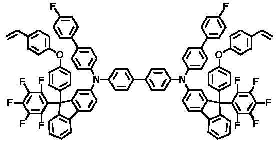

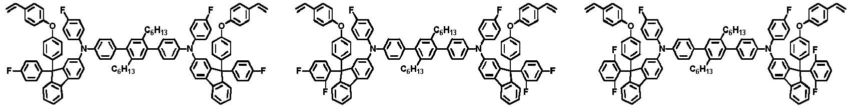

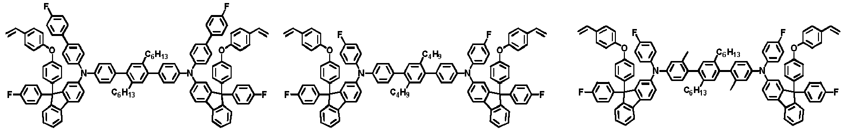

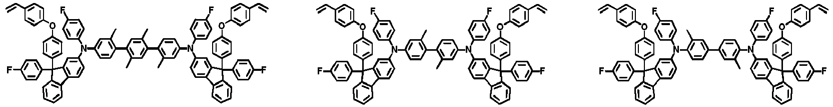

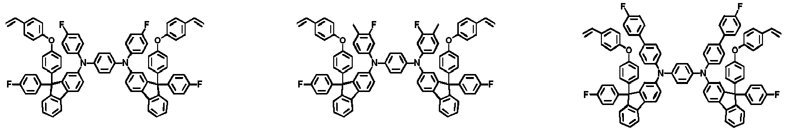

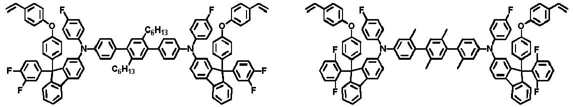

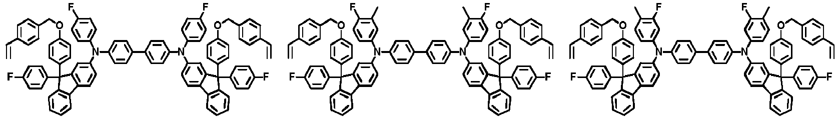

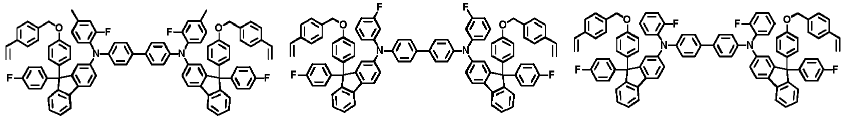

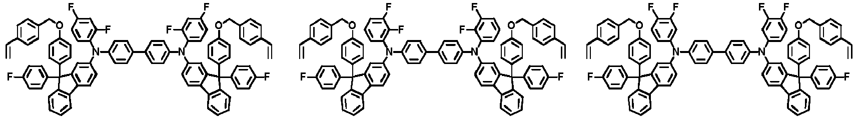

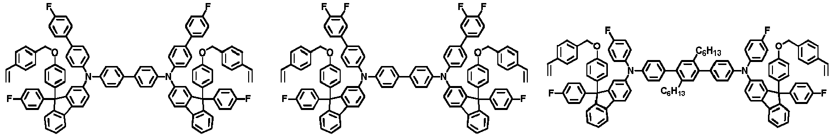

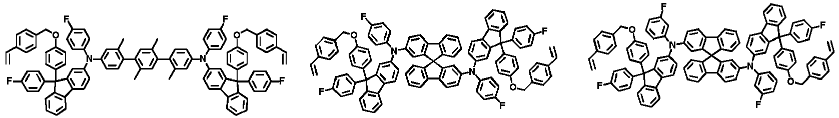

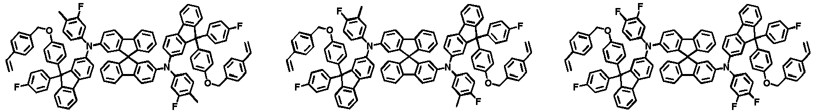

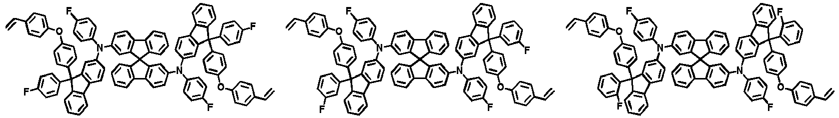

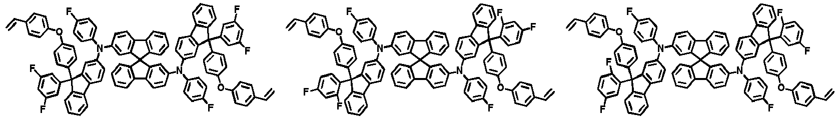

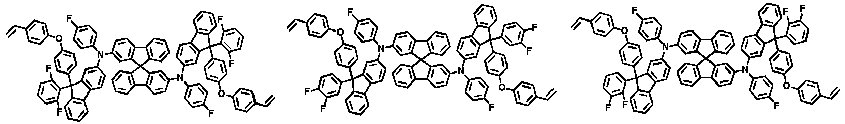

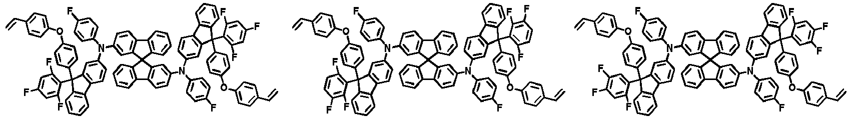

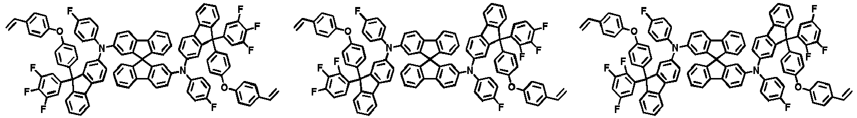

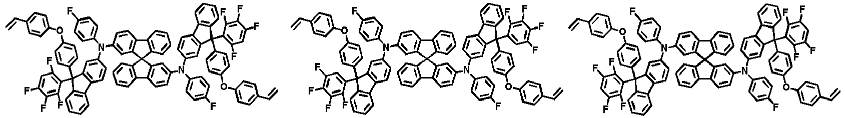

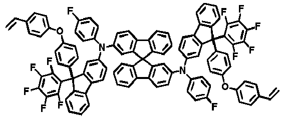

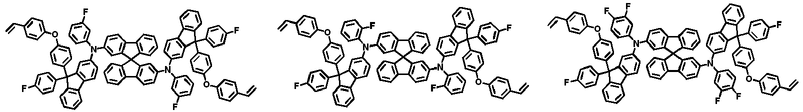

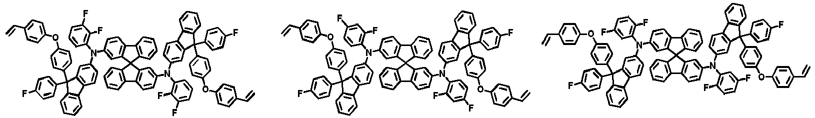

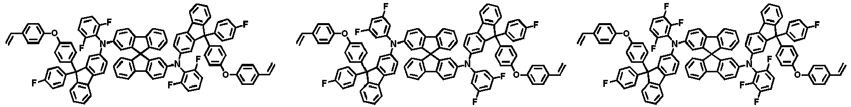

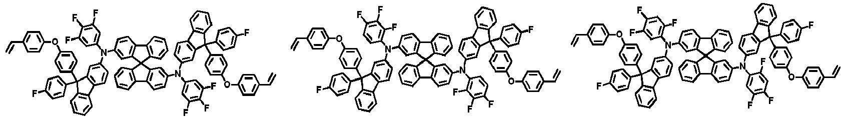

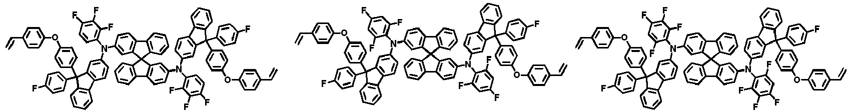

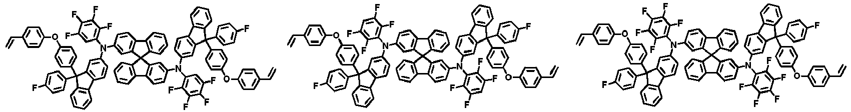

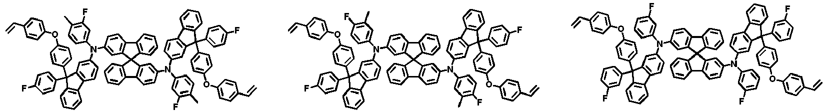

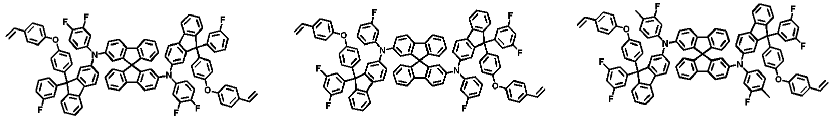

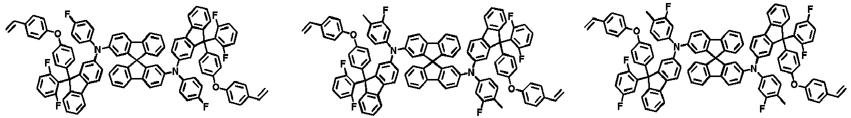

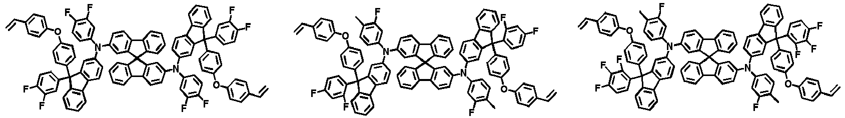

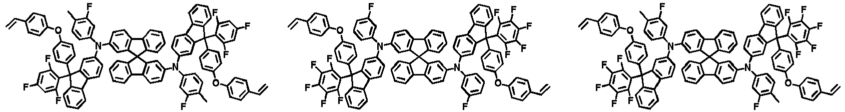

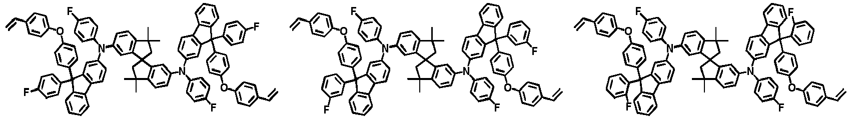

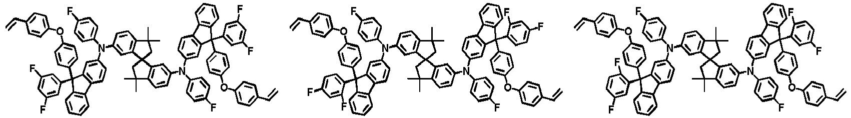

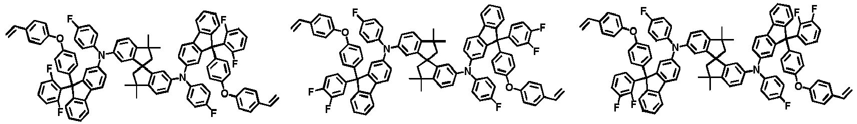

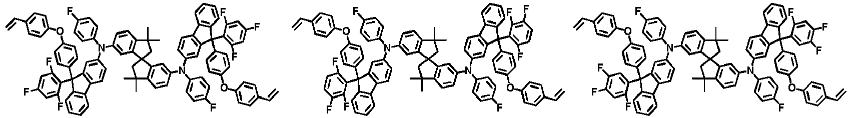

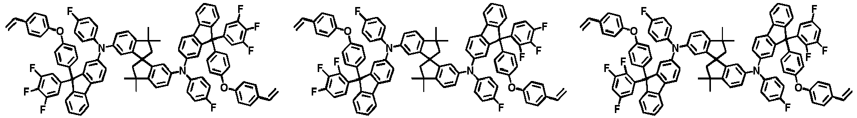

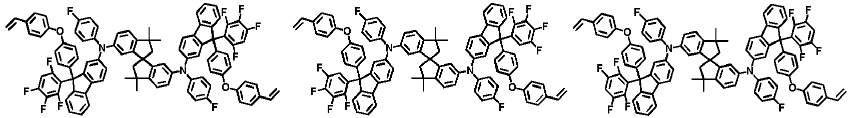

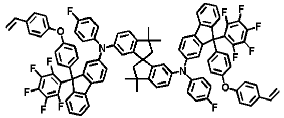

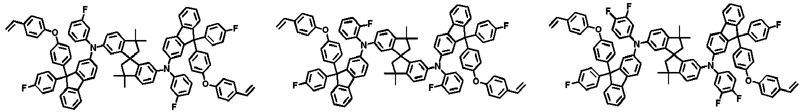

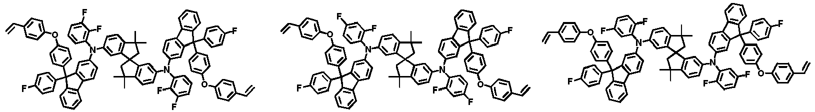

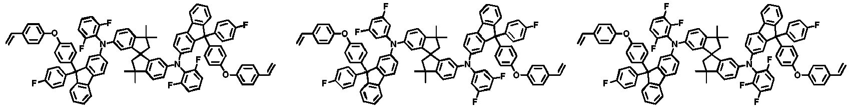

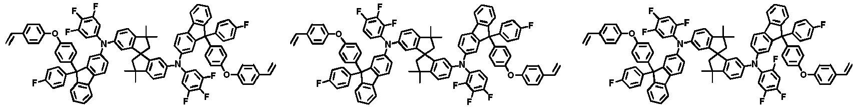

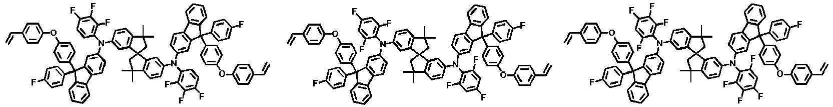

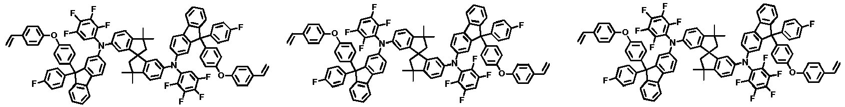

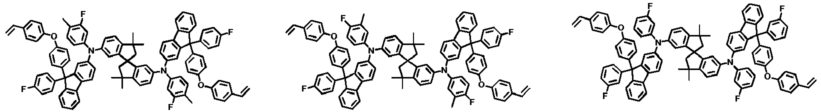

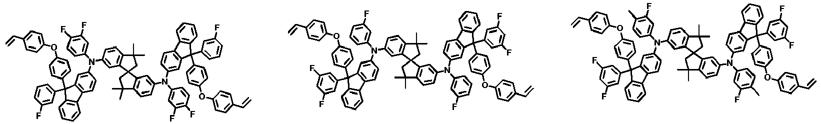

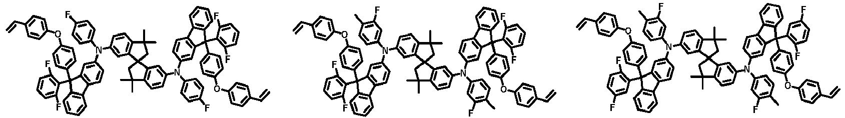

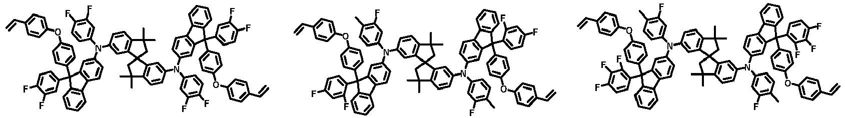

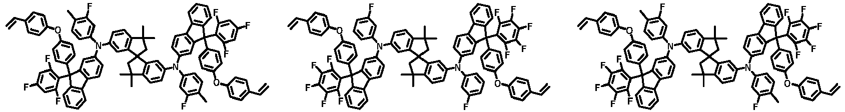

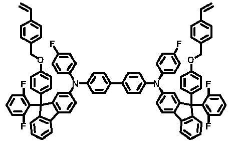

The compound according to an embodiment of the present disclosure may be prepared as in a preparation method described later. Further, the substituents may be bonded by methods known in the art, and the type, position or number of the substituents may be changed depending on techniques known in the art.

In an embodiment of the present disclosure, there is provided a coating composition comprising the above-described compound of Chemical Formula 1.

In an embodiment of the present disclosure, the coating composition comprises the compound of Chemical Formula 1 above and a solvent.

In an embodiment of the present disclosure, the coating composition may be a liquid phase. The "liquid phase" means a liquid state at room temperature and atmospheric pressure.

In an embodiment of the present disclosure, for example, chlorine-based solvents such as chloroform, methylene chloride, 1,2-dichloroethane, 1,1,2-trichloroethane, chlorobenzene, o-dichlorobenzene, etc.; ether-based solvents such as tetrahydrofuran, dioxane, etc.; aromatic hydrocarbon-based solvents such as toluene, xylene, trimethylbenzene, mesitylene, etc.; aliphatic hydrocarbon-based solvents such as cyclohexane, methylcyclohexane, n-pentane, n-hexane, n-heptane, n-octane, n-nonane, n-decane, etc.; ketone-based solvents such as acetone, methyl ethyl ketone, cyclohexanone, isophorone, tetralone, decalone, acetylacetone, etc.; ester-based solvents such as ethyl acetate, butyl acetate, ethyl cellosolve acetate, etc.; polyvalent alcohols and derivatives thereof such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, 1,2-hexanediol, etc.; alcohol-based solvents such as methanol, ethanol, propanol, isopropanol, cyclohexanol, etc.; sulfoxide-based solvents such as dimethyl sulfoxide, etc.; amide-based solvents such as N-methyl-2-pyrrolidone, N,N-dimethylformamide, etc.; and solvents such as Tetralin, etc. may be exemplified as the solvent, but any solvent capable of dissolving or dispersing the compound of Chemical Formula 1 according to an embodiment of the present disclosure is sufficient, and the present disclosure is not limited thereto.

In another embodiment, the solvent may be used alone or as a mixture of two or more solvents.

In an embodiment of the present disclosure, the coating composition does not further comprise a p-doping material.

In an embodiment of the present disclosure, the coating composition further comprises a p-doping material.

In the present disclosure, the p-doping material refers to a material that allows the host material to have p-semiconductor properties. The p-semiconductor properties refer to the properties of receiving or transporting holes to the highest occupied molecular orbital (HOMO) energy level, that is, the properties of a material having high conductivity of the holes.

The p-doping material may be any one of structures below, but the present disclosure is not limited thereto.

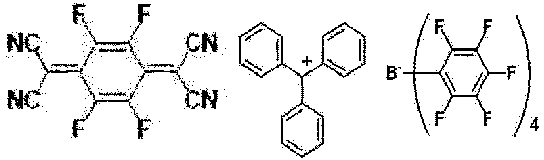

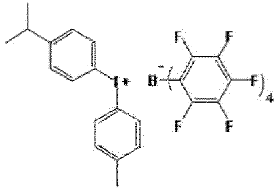

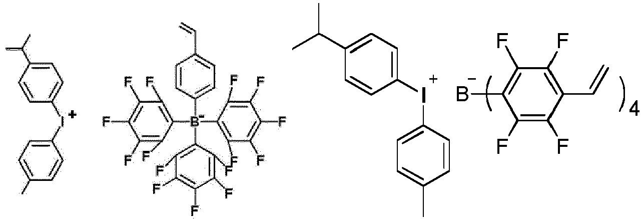

In an embodiment of the present disclosure, the p-doping material has a content of 0 to 50% by weight based on the compound of Chemical Formula 1 above.

In an embodiment of the present disclosure, the p-doping material is contained in an amount of 0 to 30% by weight based on the total solid content of the coating composition. In an embodiment of the present disclosure, the p-doping material is preferably contained in an amount of 1 to 30% by weight based on the total solid content of the coating composition.

In another embodiment, the coating composition may further comprise a single molecule containing a photocurable group and/or thermosetting group; or a single molecule containing a terminal group capable of forming a polymer by heat. The single molecule containing a photocurable group and/or thermosetting group; or the single molecule containing a terminal group capable of forming a polymer by heat as described above may be a compound with a molecular weight of 3,000 g/mol or less.

The single molecule containing a photocurable group and/or thermosetting group; or the single molecule containing a terminal group capable of forming a polymer by heat may refer to a single molecule in which aryl such as phenyl, biphenyl, fluorene, naphthalene, or the like; arylamine; or fluorene is substituted with a photocurable group and/or a thermosetting group, or a terminal group capable of forming a polymer by heat.

In another embodiment, the coating composition has a viscosity of 2 to 15 cP at room temperature.

When the above viscosity is satisfied, it is easy to manufacture a device. Specifically, a uniform film may be formed when an organic material layer is formed in the device.

The present disclosure also provides an organic light emitting device formed using the coating composition.

In an embodiment of the present disclosure, the organic light emitting device comprises: a first electrode; a second electrode; and an organic material layer consisting of one or more layers provided between the first electrode and the second electrode, wherein one or more layers of the organic material layer contain the coating composition or a cured product thereof, and the cured product of the coating composition is in a cured state due to heat treatment or light treatment.

In an embodiment of the present disclosure, the organic material layer containing the coating composition or a cured product thereof is a hole transport layer or a hole injection layer.

In an embodiment of the present disclosure, the organic material layer containing the coating composition or a cured product thereof is an electron transport layer or an electron injection layer.

In another embodiment, the organic material layer containing the coating composition or a cured product thereof is a light emitting layer.

In another embodiment, the organic material layer containing the coating composition or a cured product thereof is a light emitting layer, and the light emitting layer contains the compound of Chemical Formula 1 above as a host of the light emitting layer.

In another embodiment, the organic material layer containing the coating composition or a cured product thereof is a light emitting layer, and the light emitting layer contains the compound of Chemical Formula 1 above as a dopant of the light emitting layer.

In an embodiment of the present disclosure, the organic light emitting device further comprises one or more layers selected from the group consisting of a hole injection layer, a hole transport layer, an electron transport layer, an electron injection layer, an electron blocking layer, a hole blocking layer, a hole transport and injection layer, and an electron transport and injection layer.

In an embodiment of the present disclosure, the first electrode is an anode, and the second electrode is a cathode.

According to another embodiment, the first electrode is a cathode, and the second electrode is an anode.

In another embodiment, the organic light emitting device may be a normal type organic light emitting device in which an anode, an organic material layer consisting of one or more layers, and a cathode are sequentially stacked on a substrate.

In another embodiment, the organic light emitting device may be an inverted type organic light emitting device in which a cathode, an organic material layer consisting of one or more layers, and an anode are sequentially stacked on a substrate.

The organic material layer of the organic light emitting device of the present disclosure may be formed in a single-layer structure, but may be formed in a multilayer structure in which two or more layers of an organic material layer are stacked. For example, the organic light emitting device of the present disclosure may have a structure including a hole injection layer, a hole transport layer, a light emitting layer, an electron transport layer, an electron injection layer, a hole transport and injection layer, an electron transport and injection layer, etc. as an organic material layer. However, the structure of the organic light emitting device is not limited thereto and may include a smaller number of layers of the organic material layer.

For example, the structure of an organic light emitting device according to an embodiment of the present disclosure is exemplified in FIG. 1.

In FIG. 1, the structure of an organic light emitting device in which a first electrode 201, a hole injection layer 301, a hole transport layer 401, a light emitting layer 501, an electron transport and injection layer 601, and a second electrode 701 are sequentially stacked on a substrate 101 is exemplified. Here, the electron transport and injection layer means a layer that simultaneously performs electron transport and electron injection. The hole injection layer 301 and/or the hole transport layer 401 of FIG. 1 may be formed using a coating composition comprising the compound of Chemical Formula 1 described above.

FIG. 1 exemplifies an organic light emitting device, but the present disclosure is not limited thereto.

When the organic light emitting device comprises a plurality of layers of an organic material layer, the organic material layer may be formed of the same material or different materials.

An organic light emitting device of the present disclosure may be manufactured using materials and methods known in the art except that one or more layers of the organic material layer are formed using a coating composition comprising the compound of Chemical Formula 1 above.

For example, an organic light emitting device of the present disclosure may be manufactured by sequentially stacking an anode, an organic material layer, and a cathode on a substrate. At this time, an organic light emitting device may be manufactured by depositing a metal, a conductive metal oxide, or an alloy thereof on a substrate using a physical vapor deposition (PVD) method such as sputtering or e-beam evaporation to form an anode, forming an organic material layer including one or more layers of a hole injection layer, a hole transport layer, a light emitting layer, an electron injection layer, an electron transport layer, a hole transport and injection layer, or an electron transport and injection layer thereon through a solution process, a deposition process, etc., and depositing a material that can be used as a cathode thereon. In addition to such a method, an organic light emitting device may be manufactured by depositing a cathode material, an organic material layer, and an anode material in order on a substrate.

Furthermore, the present disclosure provides a method for manufacturing an organic light emitting device formed using the coating composition.

Specifically, in an embodiment of the present disclosure, a method for manufacturing an organic light emitting device comprises the steps of: preparing a first electrode; forming an organic material layer consisting of one or more layers on the first electrode; and forming a second electrode on the organic material layer consisting of one or more layers, wherein the step of forming of the organic material layer comprises a step of forming one or more layers using the coating composition.

In an embodiment of the present disclosure, the step of forming an organic material layer consisting of one or more layers using the coating composition uses a spin coating method.

In another embodiment, the step of forming an organic material layer consisting of one or more layers using the coating composition uses a printing method.

In an embodiment of the present disclosure, the printing method may include, for example, inkjet printing, nozzle printing, offset printing, transfer printing, screen printing, or the like, but the present disclosure is not limited thereto.

Since a coating composition according to an embodiment of the present disclosure is suitable for a solution process due to its structural characteristics so that it may be formed by a printing method, thereby having an economical effect in terms of time and cost when manufacturing a device.

In an embodiment of the present disclosure, the step of forming an organic material layer consisting of one or more layers using the coating composition comprises the steps of: coating the coating composition; and heat-treating or light-treating the coated coating composition.

In an embodiment of the present disclosure, the step of forming an organic material layer consisting of one or more layers using the coating composition comprises the steps of: coating the coating composition on the first electrode or the organic material layer consisting of one or more layers; and heat-treating or light-treating the coated coating composition.

In an embodiment of the present disclosure, the heat treatment step may be performed through heat treatment, and a heat treatment temperature in the heat treatment step may be 85 to 250°C, may be 100 to 250°C in an embodiment, and may be 150 to 250°C in another embodiment.

In another embodiment, a heat treatment time in the heat treatment step may be 1 minute to 2 hours, may be 1 minute to 1 hour in an embodiment, and may be 30 minutes to 1 hour in another embodiment.

When the step of forming an organic material layer consisting of one or more layers formed by using the coating composition comprises the heat treatment or light treatment step, a plurality of the compounds contained in the coating composition may provide an organic material layer including a thinned film structure by forming a crosslink. In this case, when another layer is stacked on the surface of the organic material layer formed by using the coating composition, it may be prevented from being dissolved, morphologically affected, or decomposed by a solvent.

Therefore, when the organic material layer formed by using the coating composition is formed by comprising a heat treatment or light treatment step, resistance to solvent increases so that a multilayer may be formed by repeatedly performing solution deposition and crosslinking method, and stability is increased so that lifespan characteristics of the device may be increased.

As the anode material, a material having a large work function is generally preferred to facilitate hole injection into the organic material layer. Specific examples of the anode material that can be used in the present disclosure may include metals such as vanadium, chromium, copper, zinc and gold, or alloys thereof; metal oxides such as zinc oxide, indium oxide, indium tin oxide (ITO), and indium zinc oxide (IZO); a combination of a metal and an oxide such as ZnO:Al or SnO₂:Sb; conductive polymers such as poly(3-methylthiophene), poly[3,4-(ethylene-1,2-dioxy)thiophene] (PEDOT), polypyrrole, and polyaniline; etc., but the present disclosure is not limited thereto.

As the cathode material, a material having a small work function is generally preferably to facilitate electron injection into the organic material layer. Specific examples of the cathode material may include metals such as barium, magnesium, calcium, sodium, potassium, titanium, indium, yttrium, lithium, gadolinium, aluminum, silver, tin and lead, or alloys thereof; a multilayered material such as LiF/Al or LiO₂/Al; etc., but the present disclosure is not limited thereto.

The hole injection layer is a layer for injecting holes from the electrode, and a compound is preferred as a hole injection material, the compound which has the ability to transport holes to have a hole injection effect on the anode and an excellent hole injection effect on the light emitting layer or the light emitting material, prevents the movement of excitons produced in the light emitting layer to the electron injection layer or an electron injection material, and is additionally excellent in the ability to form a thin film. It is preferable that the highest occupied molecular orbital (HOMO) of the hole injection material is between the work function of the anode material and the HOMO of the surrounding organic material layer. Specific examples of the hole injection material may include the compound of Chemical Formula 1 described above, metal porphyrin, oligothiophene, arylamine-based organic materials, hexanitrile hexaazatriphenylene-based organic materials, and quinacridone-based organic materials, perylene-based organic materials, anthraquinone, polyaniline- and polythiophene-based conductive polymers, etc., but the present disclosure is not limited thereto.

The hole transport layer is a layer that receives holes from the hole injection layer and transports the holes up to the light emitting layer. As a hole transport material, a material having high mobility to the holes is suitable as a material capable of receiving holes from the anode or hole injection layer to move them to the light emitting layer. Specific examples of the hole transport material may include arylamine-based organic materials, conductive polymers, block copolymers having a conjugated portion and a non-conjugated portion together, etc., but the present disclosure is not limited thereto.

The hole transport and injection layer may contain the materials of the hole transport layer and the hole injection layer described above.

As the light emitting material, a material having good quantum efficiency for fluorescence or phosphorescence is preferable as a material capable of emitting light in the visible ray region by receiving and combining holes and electrons from the hole transport layer and the electron transport layer respectively. Specific examples of the light emitting material may include 8-hydroxy-quinoline aluminum complex (Alq₃); carbazole-based compounds; dimerized styryl compounds; BAlq; 10-hydroxybenzo quinoline-metal compounds; benzoxazole-, benzthiazole- and benzimidazole-based compounds; poly(p-phenylenevinylene) (PPV)-based polymers; spiro compounds; polyfluorene, rubrene, etc., but the present disclosure is not limited thereto.

The light emitting layer may contain a host material and a dopant material. The host material includes condensed aromatic ring derivatives, heterocycle-containing compounds, or the like. Specifically, the condensed aromatic ring derivatives may include anthracene derivatives, pyrene derivatives, naphthalene derivatives, pentacene derivatives, phenanthrene compounds, fluoranthene compounds, etc., and the heterocycle-containing compounds may include carbazole derivatives, dibenzofuran derivatives, ladder-type furan compounds, pyrimidine derivatives, etc., but the present disclosure is not limited thereto.

Examples of the dopant material may include an aromatic amine derivative, a styrylamine compound, a boron complex, a fluoranthene compound, a metal complex, etc. Specifically, the aromatic amine derivative, as a condensed aromatic ring derivative having a substituted or unsubstituted arylamino group, may include pyrene, anthracene, chrysene, periflanthene, or the like, which has an arylamino group. The styrylamine compound is a compound in which at least one arylvinyl group is substituted in a substituted or unsubstituted arylamine, and one or more substituents selected from the group consisting of an aryl group, a silyl group, an alkyl group, a cycloalkyl group, and an arylamino group are substituted or unsubstituted in the styrylamine compound. Specifically, the styrylamine compound may include styrylamine, styryldiamine, styryltriamine, styryltetraamine, etc., but the present disclosure is not limited thereto. Further, the metal complex may include an iridium complex, a platinum complex, etc., but the present disclosure is not limited thereto.

The electron transport layer is a layer that receives electrons from the electron injection layer and transports them to the light emitting layer. As an electron transport material, a material having high mobility to the electrons is suitable as a material capable of receiving electrons well from the cathode and moving them to the light emitting layer. Specific examples of the electron transport material may include Al complexes of 8-hydroxyquinoline; complexes comprising Alq₃; organic radical compounds; hydroxyflavone-metal complexes; etc., but the present disclosure is not limited thereto. The electron transport layer may be used together with any desired cathode material as has been used in accordance with the conventional art. In particular, examples of appropriate cathode materials are conventional materials having a low work function and followed by an aluminum layer or a silver layer. Specifically, the cathode materials are cesium, barium, calcium, ytterbium, and samarium, followed in each case by an aluminum layer or a silver layer.

The electron injection layer is a layer that injects electrons from the electrode, and a compound is preferred as an electron injection material, the compound which has the ability to transport electrons, has an effect of injecting electrons from the cathode and an excellent electron injection effect on the light emitting layer or the light emitting material, prevents the movement of excitons produced in the light emitting layer to the hole injection layer, and is additionally excellent in the ability to form a thin film. Specifically, examples of the electron injection material may include fluorenone, anthraquinodimethane, diphenoquinone, thiopyran dioxide, oxazole, oxadiazole, triazole, imidazole, perylenetetracarboxylic acid, fluorenylidene methane, anthrone, and the like, derivatives thereof, metal complex compounds, nitrogen-containing 5-membered ring derivatives, etc., but the present disclosure is not limited thereto.

The electron transport and injection layer may contain the aforementioned materials of the electron transport layer and the electron injection layer.

Examples of the metal complex compounds may include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-crezolato)gallium, bis(2-methyl-8-quinolinato)(1-naphtolato)aluminum, bis(2-methyl-8-quinolinato)(2-naphtolato)gallium, etc., but the present disclosure is not limited thereto.

The hole blocking layer is a layer that blocks holes from reaching the cathode, and may be generally formed under the same conditions as the hole injection layer. Specifically, the hole blocking layer may contain oxadiazole derivatives or triazole derivatives, phenanthroline derivatives, BCP, aluminum complexes, etc., but the present disclosure is not limited thereto.

The electron blocking layer is a layer that blocks electrons from reaching the anode, and materials known in the art may be used.

The organic light emitting device according to the present disclosure may be a top emission type, a back emission type, or a double side emission type depending on the materials used.

### [Mode for Carrying Out the Invention]

Hereinafter, Examples will be given and described in detail in order to describe the present disclosure specifically.

Examples of the present disclosure are provided to more completely explain the present disclosure to those of ordinary skill in the art.

### <Preparation Example>

### Preparation Example 1. Preparation of Compound 1

### 1) Synthesis of Intermediate 1-1

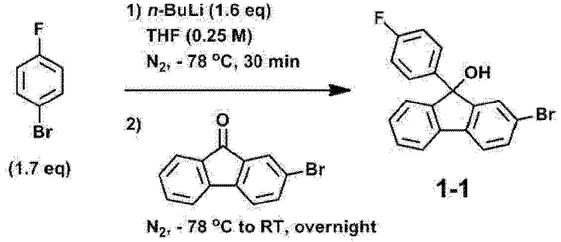

27.9 mL (255 mmol) of 1-bromo-4-fluorobenzene was put into 500 mL of tetrahydrofuran (THF). After nitrogen substitution, a reaction mixture was cooled to -78°C. 96 mL (240 mmol) of n-BuLi (2.5 M Hex) was put into a dropping funnel, and then slowly injected into the reaction mixture. The mixture was stirred at -78°C for 30 minutes. 38.9 g (150 mmol) of 2-bromofluorenone was injected into the stirred mixture. The mixture was stirred overnight while slowly raising the temperature to room temperature. After terminating the reaction by injecting distilled water, a reaction product was extracted with ethyl acetate and water. After collecting an organic layer, the organic layer was dried using MgSO₄ and filtered. The filtrate was dried with a vacuum rotary concentrator to remove an organic solvent and used for the next reaction.

### 2) Synthesis of Intermediate 1-2

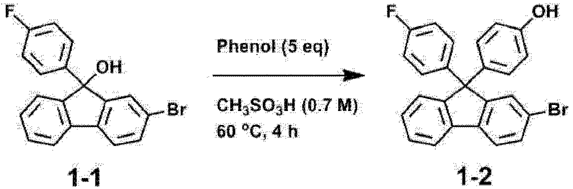

Up to 53 g (150 mmol) of Intermediate 1-1 and 70.6 g (750 mmol) of phenol were put into a round bottom flask (RBF). After putting 214 mL of CH₃SO₃H, the mixture was stirred at 60°C for 4 hours. After injecting ice water, the mixture was extracted with ethyl acetate and water. After collecting an organic layer, the organic layer was dried using MgSO₄ and filtered. The filtrate was dried with a vacuum rotary concentrator to remove an organic solvent. After column-purifying the dried filtrate, 40.6 g of Intermediate 1-2 was obtained by crystallizing the column-purified filtrate under dichloromethane/heptane conditions.

### 3) Synthesis of Intermediate 1-3

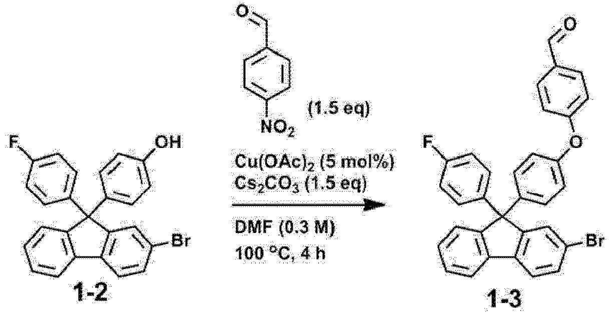

40 g (92.7 mmol) of Intermediate 1-2, 21.2 g (139 mmol) of 4-nitrobenzaldehyde, 842 mg (4.64 mmol) of Cu(OAc)₂, and 45.3 g (139 mmol) of Cs₂CO₃ were put into a round bottom flask (RBF). 310 mL of dimethylformamide (DMF) was put into the RBF and stirred at 100°C for 4 hours. After extracting a reaction product with ethyl acetate and water, and collecting an organic layer, the organic layer was dried using MgSO₄ and filtered. The filtrate was dried with a vacuum rotary concentrator to remove an organic solvent. After column-purifying the dried filtrate, 38.7 g of Intermediate 1-3 was obtained by crystallizing the column-purified filtrate under dichloromethane (DCM)/heptane conditions.

### 4) Synthesis of Intermediate 1-4

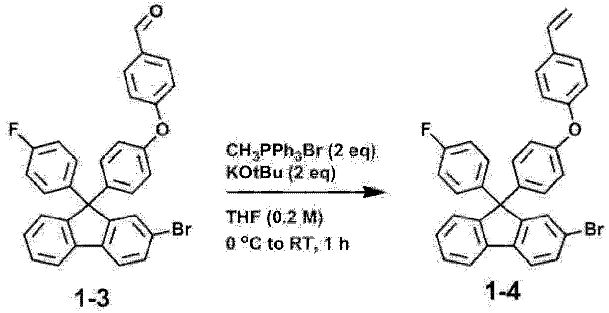

51.6 g (144.6 mmol) of CH₃PPh₃Br, 16.2 g (144.6 mmol) of KOtBu, and 217 mL of THF were contained in an RBF and then cooled to 0°C. A solution obtained by dissolving 38.7 g (72.3 mmol) of Intermediate 1-3 in 144 mL of tetrahydrofuran (THF) was injected into the reaction mixture. While raising the temperature to room temperature, the mixture was stirred for 1 hour. After extracting the stirred mixture with ethyl acetate and water and collecting an organic layer, the organic layer was dried using MgSO₄ and filtered. The filtrate was dried with a vacuum rotary concentrator to remove an organic solvent. After column-purifying the dried filtrate, 33.7 g of Intermediate 1-4 was obtained by crystallizing the column-purified filtrate under DCM/EtOH conditions.

### 5) Synthesis of Compound 1

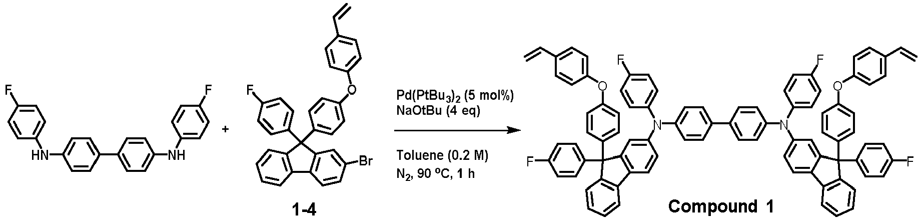

944 mg (2.5 mmol) of N,N'-bis(4-fluorophenyl)benzidine, 2.73 g (5.13 mmol) of Intermediate 1-4, 64 mg (0.125 mmol) of Pd(PtBu₃)₂, and 961 mg (10 mmol) of NaOtBu were put into an RBF. After nitrogen substitution, 12.5 mL of toluene was put into the RBF and stirred at 90°C for 1 hour. After extracting the stirred mixture with ethyl acetate and water and collecting an organic layer, the organic layer was dried using MgSO₄ and filtered. The filtrate was dried with a vacuum rotary concentrator to remove an organic solvent. After column-purifying the dried filtrate, 2.7 g of Compound 1 was obtained by crystallizing the column-purified filtrate under DCM/EtOH conditions. The synthesis of the compound was confirmed through LC-MS and NMR. MS: [M+H]⁺ = 1277

NMR measurement values of Compound 1: ¹H NMR (500 MHz, DMSO-d6) δ 7.83 (m, 4H), 7.45 - 7.36 (m, 12H), 7.27 (t, 2H), 7.17 - 6.86 (m, 36H), 6.65 - 6.57 (m, 2H), 5.65 (dd, 2H), 5.11 (dd, 2H)

### Preparation Example 2. Preparation of Compound 2

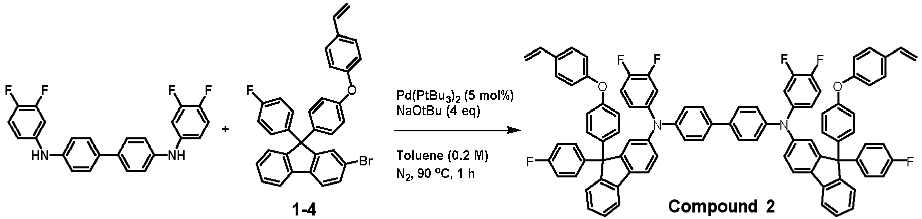

1.02 g (2.5 mmol) of N,N'-Bis(3,4-difluorophenyl)benzidine, 2.73 g (5.13 mmol) of Intermediate 1-4, 64 mg (0.125 mmol) of Pd(PtBu₃)₂, and 961 mg (10 mmol) of NaOtBu were put into an RBF. After nitrogen substitution, 12.5 mL of toluene was put into the RBF and stirred at 90°C for 1 hour. After extracting the stirred mixture with ethyl acetate and water and collecting an organic layer, the organic layer was dried using MgSO₄ and filtered. The filtrate was dried with a vacuum rotary concentrator to remove an organic solvent. After column-purifying the dried filtrate, 2.8 g of Compound 2 was obtained by crystallizing the column-purified filtrate under DCM/EtOH conditions. The synthesis of the compound was confirmed through LC-MS and NMR. MS: [M+H]⁺ = 1313

NMR measurement values of Compound 2: ¹H NMR (500 MHz, DMSO-d6) δ 7.87 (t, 4H), 7.50 (d, 4H), 7.44 - 7.27 (m, 12H), 7.11 - 7.00 (m, 22H) 6.94 - 6.83 (m, 10H), 6.66 - 6.59 (m, 2H), 5.66 (dd, 2H), 5.12 (dd, 2H)

### Preparation Example 3. Preparation of Compound 3

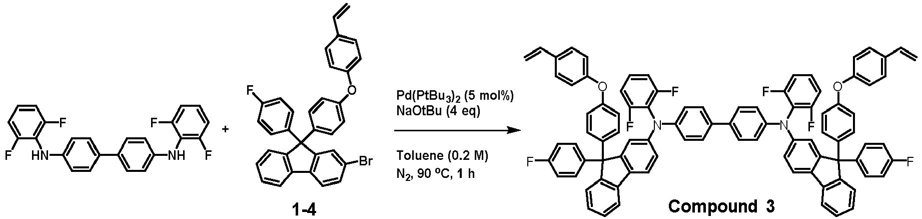

1.02 g (2.5 mmol) of N,N'-Bis(2,6-difluorophenyl)benzidine, 2.73 g (5.13 mmol) of Intermediate 1-4, 64 mg (0.125 mmol) of Pd(PtBu₃)₂, and 961 mg (10 mmol) of NaOtBu were put into an RBF. After nitrogen substitution, 12.5 mL of toluene was put into the RBF and stirred at 90°C for 1 hour. After extracting the stirred mixture with ethyl acetate and water and collecting an organic layer, the organic layer was dried using MgSO₄ and filtered. The filtrate was dried with a vacuum rotary concentrator to remove an organic solvent. After column-purifying the dried filtrate, 2.5 g of Compound 3 was obtained by crystallizing the column-purified filtrate under DCM/EtOH conditions. The synthesis of the compound was confirmed through LC-MS and NMR. MS: [M+H]⁺ = 1313

NMR measurement values of Compound 3: ¹H NMR (500 MHz, DMSO-d6) δ 7.86 - 7.83 (m, 4H), 7.46 - 7.37 (m, 14H), 7.30 - 7.22 (m, 6H), 7.10 - 6.87 (m, 28H), 6.65 - 6.56 (m, 2H), 5.66 (dd, 2H), 5.11 (dd, 2H)

### Preparation Example 4. Preparation of Compound 4

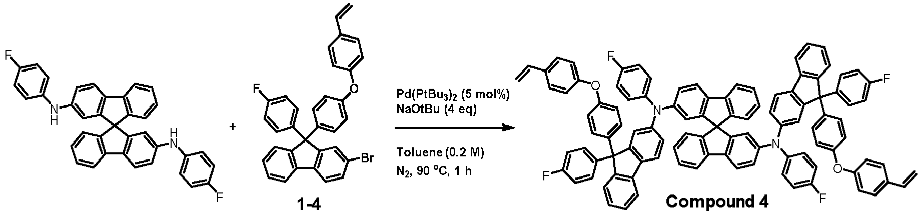

1.34 g (2.5 mmol) of 2,2'-Bis(4-fluorophenylamino)-9,9'-spirobi[9H-fluorene], 2.73 g (5.13 mmol) of Intermediate 1-4, 64 mg (0.125 mmol) of Pd(PtBu₃)₂, and 961 mg (10 mmol) of NaOtBu were put into an RBF. After nitrogen substitution, 12.5 mL of toluene was put into the RBF and stirred at 90°C for 1 hour. After extracting the stirred mixture with ethyl acetate and water and collecting an organic layer, the organic layer was dried using MgSO₄ and filtered. The filtrate was dried with a vacuum rotary concentrator to remove an organic solvent. After column-purifying the dried filtrate, 2.9 g of Compound 4 was obtained by crystallizing the column-purified filtrate under DCM/EtOH conditions. The synthesis of the compound was confirmed through LC-MS and NMR. MS: [M+H]⁺ = 1440

NMR measurement values of Compound 4: ¹H NMR (500 MHz, DMSO-d6) δ 7.80 - 7.74 (m, 6H), 7.69 (d, 2H), 7.44 - 7.33 (m, 8H), 7.30 - 7.24 (m, 4H), 7.06 - 6.80 (m, 36H), 6.71 - 6.63 (m, 2H), 6.56 (m, 2H), 6.30 - 6.28 (m, 2H), 5.70 (dd, 2H), 5.17 (dd, 2H)

### Preparation Example 5. Preparation of Compound 5

### 1) Synthesis of Intermediate 5-1

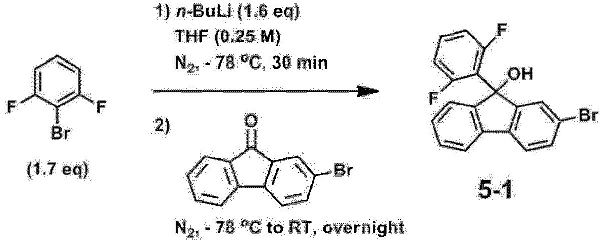

29.4 mL (255 mmol) of 1-bromo-2,6-difluorobenzene was put into 500 mL of THF. After nitrogen substitution, a reaction mixture was cooled to -78°C. 96 mL (240 mmol) of n-BuLi (2.5 M Hex) was put into a dropping funnel, and then slowly injected into the reaction mixture. The mixture was stirred at -78°C for 30 minutes. 38.9 g (150 mmol) of 2-bromofluorenone was injected into the stirred mixture and stirred overnight while slowly raising the temperature to room temperature. After terminating the reaction by injecting distilled water, a reaction product was extracted with ethyl acetate and water. After collecting an organic layer, the organic layer was dried using MgSO₄ and filtered. The filtrate was dried with a vacuum rotary concentrator to remove an organic solvent and used for the next reaction.

### 2) Synthesis of Intermediate 5-2

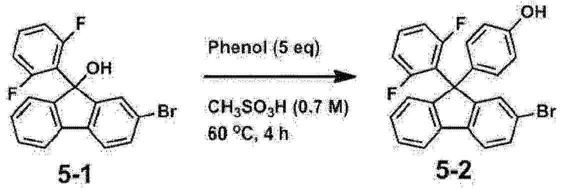

56 g (150 mmol) of Intermediate 5-1 and 70.6 g (750 mmol) of phenol were put into an RBF. 214 mL of CH₃SO₃H was put into the RBF and stirred at 60°C for 4 hours. After injecting ice water, the mixture was extracted with ethyl acetate and water. After collecting an organic layer, the organic layer was dried using MgSO₄ and filtered. The filtrate was dried with a vacuum rotary concentrator to remove an organic solvent. After column-purifying the dried filtrate, 45.2 g of Intermediate 5-2 was obtained by crystallizing the column-purified filtrate under DCM/Heptane conditions.

### 3) Synthesis of Intermediate 5-3

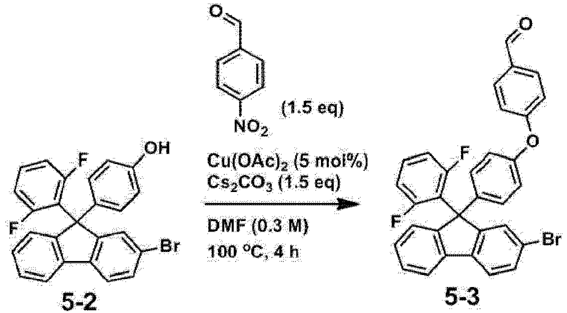

45.2 g (100 mmol) of Intermediate 5-2, 22.8 g (150 mmol) of 4-nitrobenzaldehyde, 908 mg (5 mmol) of Cu(OAc)₂, and 48.9 g (150 mmol) of Cs₂CO₃ were put into an RBF. 333 mL of DMF was put into the RBF and stirred at 100°C for 4 hours. After extracting the stirred mixture with ethyl acetate and water and collecting an organic layer, the organic layer was dried using MgSO₄ and filtered. The filtrate was dried with a vacuum rotary concentrator to remove an organic solvent. After column-purifying the dried filtrate, 39.8 g of Intermediate 5-3 was obtained by crystallizing the column-purified filtrate under DCM/Heptane conditions.

### 4) Synthesis of Intermediate 5-4

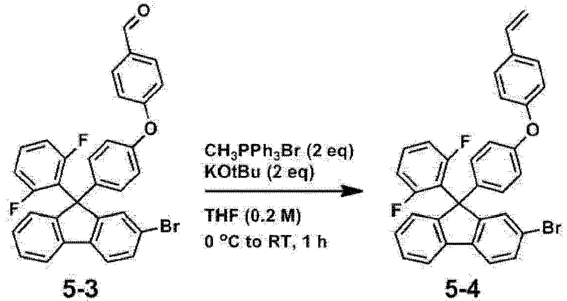

51.4 g (144 mmol) of CH₃PPh₃Br, 16.2 g (144 mmol) of KOtBu, and 216 mL of THF were contained in an RBF and then cooled to 0°C. A solution obtained by dissolving 39.8 g (72 mmol) of Intermediate 5-3 in 144 mL of THF was injected into a reaction mixture, the mixture was stirred for 1 hour while raising the temperature to room temperature. After extracting the stirred mixture with ethyl acetate and water and collecting an organic layer, the organic layer was dried using MgSO₄ and filtered. The filtrate was dried with a vacuum rotary concentrator to remove an organic solvent. After column-purifying the dried filtrate, 34.8 g of Intermediate 5-4 was obtained by crystallizing the column-purified filtrate under DCM/EtOH conditions.

### 5) Synthesis of Compound 5

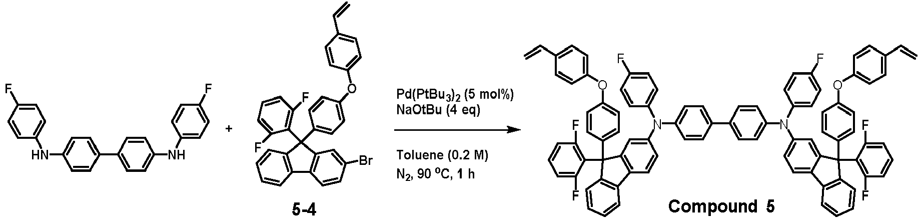

944 mg (2.5 mmol) of N,N'-Bis(4-fluorophenyl)benzidine, 2.83 g (5.13 mmol) of Intermediate 5-4, 64 mg (0.125 mmol) of Pd(PtBu₃)₂, and 961 mg (10 mmol) of NaOtBu were put into an RBF. After nitrogen substitution, 12.5 mL of toluene was put into the RBF and stirred at 90°C for 1 hour. After extracting the stirred mixture with ethyl acetate and water and collecting an organic layer, the organic layer was dried using MgSO₄ and filtered. The filtrate was dried with a vacuum rotary concentrator to remove an organic solvent. After column-purifying the dried filtrate, 2.7 g of Compound 5 was obtained by crystallizing the column-purified filtrate under DCM/EtOH conditions. The synthesis of the compound was confirmed through LC-MS and NMR. MS: [M+H]⁺ = 1331

NMR measurement values of Compound 5: ¹H NMR (500 MHz, DMSO-d6) δ 7.84 - 7.81 (m, 4H), 7.46 (d, 2H), 7.43 - 7.34 (m, 12H), 7.27 (t, 2H), 7.18 - 7.12 (m, 6H), 7.09 - 6.95 (m, 14H), 6.95 - 6.87 (m, 8H), 6.83 (t, 4H), 6.64 - 6.55 (m, 2H), 5.64 (dd, 2H), 5.10 (dd, 2H)

### Preparation Example 6. Preparation of Compound 6

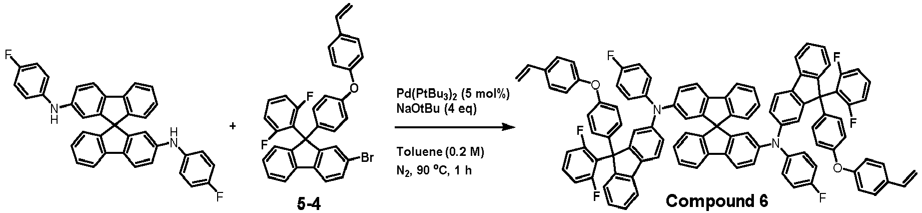

1.34 g (2.5 mmol) of 2,2'-Bis(4-fluorophenylamino)-9,9'-spirobi[9H-fluorene], 2.83 g (5.13 mmol) of Intermediate 5-4, 64 mg (0.125 mmol) of Pd(PtBu₃)₂, and 961 mg (10 mmol) of NaOtBu were put into an RBF. After nitrogen substitution, 12.5 mL of toluene was put into the RBF and stirred at 90°C for 1 hour. After extracting the stirred mixture with ethyl acetate and water and collecting an organic layer, the organic layer was dried using MgSO₄ and filtered. The filtrate was dried with a vacuum rotary concentrator to remove an organic solvent. After column-purifying the dried filtrate, 2.9 g of Compound 6 was obtained by crystallizing the column-purified filtrate under DCM/EtOH conditions. The synthesis of the compound was confirmed through LC-MS and NMR. MS: [M+H]⁺ = 1475

NMR measurement values of Compound 6: ¹H NMR (500 MHz, DMSO-d6) δ 7.79 - 7.73 (m, 6H), 7.69 - 7.66 (m, 2H), 7.44 - 7.40 (m, 6H), 7.36 - 7.22 (m , 8H), 7.08 - 6.85 (m, 22H), 6.85 - 6.67 (m, 10H), 6.71 - 6.63 (m, 2H), 6.58 - 6.55 (m, 2H), 6.26 (m, 2H), 5.70 (dd , 2H), 5.17 (dd, 2H)

### Preparation Example 7. Preparation of Compound 7

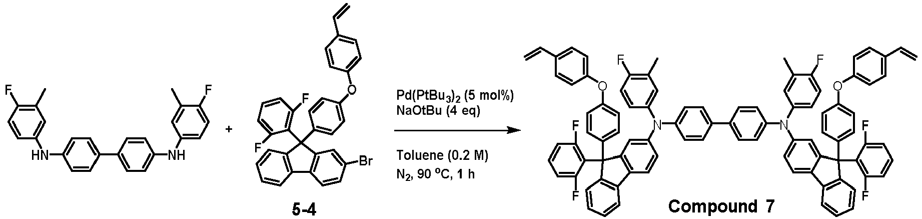

1.0 g (2.5 mmol) of N,N'-Bis(4-fluoro-3-methylphenyl)benzidine, 2.83 g (5.13 mmol) of Intermediate 5-4, 64 mg (0.125 mmol) of Pd(PtBu₃)₂, and 961 mg (10 mmol) of NaOtBu were put into an RBF. After nitrogen substitution, 12.5 mL of toluene was put into the RBF and stirred at 90°C for 1 hour. After extracting the stirred mixture with ethyl acetate and water and collecting an organic layer, the organic layer was dried using MgSO₄ and filtered. The filtrate was dried with a vacuum rotary concentrator to remove an organic solvent. After column-purifying the dried filtrate, 3.1 g of Compound 7 was obtained by crystallizing the column-purified filtrate under DCM/EtOH conditions. The synthesis of the compound was confirmed through LC-MS and NMR. MS: [M+H]⁺ = 1341

NMR measurement values of Compound 7: ¹H NMR (500 MHz, DMSO-d6) δ 7.84 - 7.81 (m, 4H), 7.46 (d, 2H), 7.43 - 7.34 (m, 10H), 7.27 (t, 2H), 7.18 - 7.12 (m, 6H), 7.09 - 6.95 (m, 14H), 6.95 - 6.87 (m, 8H), 6.83 (t, 4H), 6.64 - 6.55 (m, 2H), 5.64 (dd, 2H), 5.10 (dd, 2H), 2.33 (s, 6H)

### Preparation Example 8. Preparation of Compound 8

### 1) Synthesis of Intermediate 8-1

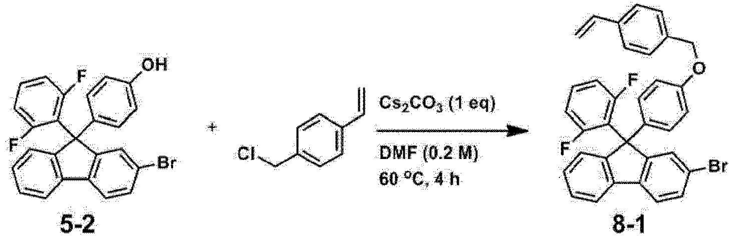

4.5 g (10 mmol) of Intermediate 5-2 and 3.3 g (10 mmol) of Cs₂CO₃ were put into an RBF. 50 mL of DMF and 1.7 mL (12 mmol) of 4-vinylbenzyl chloride were put into the RBF and stirred at 60°C for 4 hours. After extracting the stirred mixture with ethyl acetate and water and collecting an organic layer, the organic layer was dried using MgSO₄ and filtered. The filtrate was dried with a vacuum rotary concentrator to remove an organic solvent. After column-purifying the dried filtrate, 3.9 g of Intermediate 8-1 was obtained by crystallizing the column-purified filtrate under DCM/Heptane conditions.

### 2) Synthesis of Compound 8

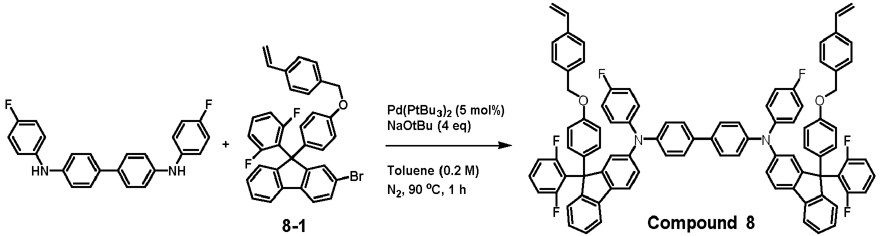

944 mg (2.5 mmol) of N,N'-Bis(4-fluorophenyl)benzidine, 2.9 g (5.13 mmol) of Intermediate 8-1, 64 mg (0.125 mmol) of Pd(PtBu₃)₂, and 961 mg (10 mmol) of NaOtBu were put into an RBF. After nitrogen substitution, 12.5 mL of toluene was put into the RBF and stirred at 90°C for 1 hour. After extracting the stirred mixture with ethyl acetate and water and collecting an organic layer, the organic layer was dried using MgSO₄ and filtered. The filtrate was dried with a vacuum rotary concentrator to remove an organic solvent. After column-purifying the dried filtrate, 2.8 g of Compound 8 was obtained by crystallizing the column-purified filtrate under DCM/EtOH conditions. The synthesis of the compound was confirmed through LC-MS and NMR. MS: [M+H]⁺ = 1341

NMR measurement values of Compound 8: ¹H NMR (500 MHz, DMSO-d6) δ 7.84 - 7.81 (m, 4H), 7.46 (d, 2H), 7.43 - 7.33 (m, 12H), 7.26 (t, 2H), 7.19 - 7.13 (m, 6H), 7.09 - 6.96 (m, 14H), 6.94 - 6.87 (m, 8H), 6.83 (t, 4H), 6.64 - 6.55 (m, 2H), 5.64 (dd, 2H), 5.16 (s, 4H), 5.10 (dd, 2H)

### Preparation Example 9. Preparation of Compound 9

### 1) Synthesis of Intermediate 9-1

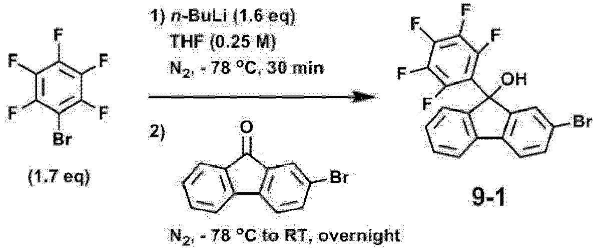

31.8 mL (255 mmol) of 1-bromo-2,3,4,5,6-pentafluorobenzene was put into 500 mL of THF, and after nitrogen substitution, a reaction mixture was cooled to -78°C. 96 mL (240 mmol) of n-BuLi (2.5 M Hex) was put into a dropping funnel, and then slowly injected into the reaction mixture, followed by stirring at -78°C for 30 minutes. 38.9 g (150 mmol) of 2-bromofluorenone was injected into the stirred mixture. The mixture was stirred overnight while slowly raising the temperature to room temperature. After terminating the reaction by injecting distilled water, a reaction product was extracted with ethyl acetate and water. After collecting an organic layer, the organic layer was dried using MgSO₄ and filtered. The filtrate was dried with a vacuum rotary concentrator to remove an organic solvent and used for the next reaction.

### 2) Synthesis of Intermediate 9-2

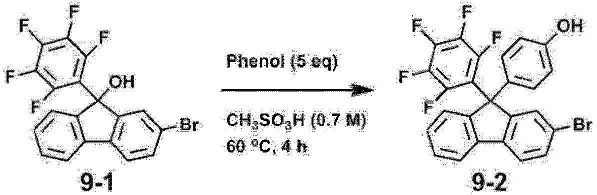

64.1 g (150 mmol) of Intermediate 9-1 and 70.6 g (750 mmol) of phenol were put into an RBF. 214 mL of CH₃SO₃H was put into the RBF and stirred at 60°C for 4 hours. After injecting ice water, the mixture was extracted with ethyl acetate and water. After collecting an organic layer, the organic layer was dried using MgSO₄ and filtered. The filtrate was dried with a vacuum rotary concentrator to remove an organic solvent. After column-purifying the dried filtrate, 52.8 g of Intermediate 9-2 was obtained by crystallizing the column-purified filtrate under DCM/Heptane conditions.

### 3) Synthesis of Intermediate 9-3

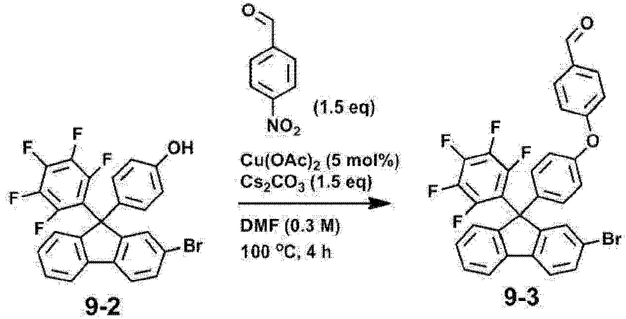

50.3 g (100 mmol) of Intermediate 9-2, 22.8 g (150 mmol) of 4-nitrobenzaldehyde, 908 mg (5 mmol) of Cu(OAc)₂, and 48.9 g (150 mmol) of Cs₂CO₃ were put into an RBF. 333 mL of DMF was put into the RBF and stirred at 100°C for 4 hours. After extracting the stirred mixture with ethyl acetate and water and collecting an organic layer, the organic layer was dried using MgSO₄ and filtered. The filtrate was dried with a vacuum rotary concentrator to remove an organic solvent. After column-purifying the dried filtrate, 48.6 g of Intermediate 9-3 was obtained by crystallizing the column-purified filtrate under DCM/Heptane conditions.

### 4) Synthesis of Intermediate 9-4

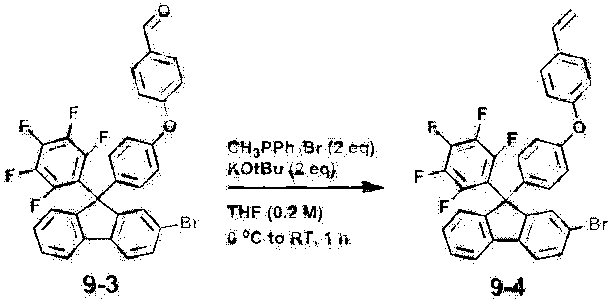

51.4 g (144 mmol) of CH₃PPh₃Br, 16.2 g (144 mmol) of KOtBu, and 216 mL of THF were contained in an RBF and then cooled to 0°C. A solution obtained by dissolving 43.7 g (72 mmol) of Intermediate 9-3 in 144 mL of THF was injected into a reaction mixture, the mixture was stirred for 1 hour while raising the temperature to room temperature. After extracting the stirred mixture with ethyl acetate and water and collecting an organic layer, the organic layer was dried using MgSO₄ and filtered. The filtrate was dried with a vacuum rotary concentrator to remove an organic solvent. After column-purifying the dried filtrate, 31 g of Intermediate 9-4 was obtained by crystallizing the column-purified filtrate under DCM/EtOH conditions.

### 5) Synthesis of Compound 9

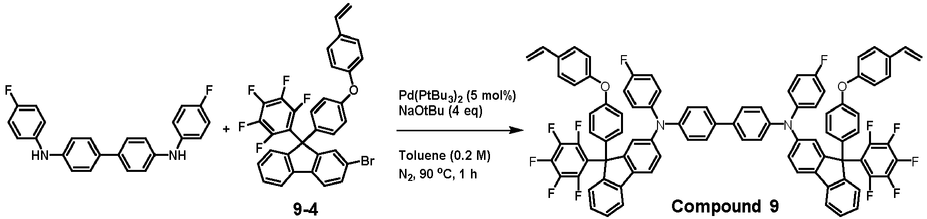

944 mg (2.5 mmol) of N,N'-Bis(4-fluorophenyl)benzidine, 2.73 g (5.13 mmol) of Intermediate 9-4, 64 mg (0.125 mmol) of Pd(PtBu₃)₂, and 961 mg (10 mmol) of NaOtBu were put into an RBF. After nitrogen substitution, 12.5 mL of toluene was put into the RBF and stirred at 90°C for 1 hour. After extracting the stirred mixture with ethyl acetate and water and collecting an organic layer, the organic layer was dried using MgSO₄ and filtered. The filtrate was dried with a vacuum rotary concentrator to remove an organic solvent. After column-purifying the dried filtrate, 2.7 g of Compound 9 was obtained by crystallizing the column-purified filtrate under DCM/EtOH conditions. The synthesis of the compound was confirmed through LC-MS and NMR. MS: [M+H]⁺ = 1421

NMR measurement values of Compound 9: ¹H NMR (500 MHz, DMSO-d6) δ 7.83 (m, 4H), 7.45 - 7.36 (m, 12H), 7.27 (t, 2H), 7.17 - 6.86 (m, 28H), 6.65 - 6.57 (m, 2H), 5.65 (dd, 2H), 5.11 (dd, 2H)

### <Device Example>

### Experimental Example 1.

After putting a glass substrate coated with an indium tin oxide (ITO) thin film to a thickness of 1,500 Å into distilled water in which a detergent had been dissolved, it was cleaned with ultrasonic waves. At this time, a product manufactured by Fischer Co. was used as the detergent, and distilled water that had been secondarily filtered with a filter manufactured by Millipore Co. was used as distilled water. After washing ITO for 30 minutes, ultrasonic cleaning was performed for 10 minutes by repeating it twice with distilled water. After washing with distilled water was finished, ultrasonic cleaning was performed with a solvent of isopropyl alcohol or acetone, and the substrate was dried. After cleaning the substrate for 5 minutes, the substrate was transported to a glove box.

2% by weight of cyclohexanone ink containing Compound 1 prepared in Preparation Example 1 above and Compound G below at a weight ratio of 8:2 was spin-coated on the ITO surface of an ITO transparent electrode and heat-treated at 220°C for 30 minutes to form a hole injection layer with a thickness of 400 Å.

A 2% by weight toluene ink of Compound A below was spin-coated on the hole injection layer and heat-treated at 120°C for 10 minutes to form a hole transport layer having a thickness of 200 Å. Compound B and Compound C below were vacuum-deposited at a weight ratio of 92:8 on the hole transport layer to form a light emitting layer having a thickness of 200 Å. Compound D below was vacuum-deposited on the light emitting layer to form an electron transport and injection layer having a thickness of 350 Å. A cathode was formed by sequentially depositing LiF to a thickness of 10 Å and aluminum to a thickness of 1,000 Å on the electron transport and injection layer.

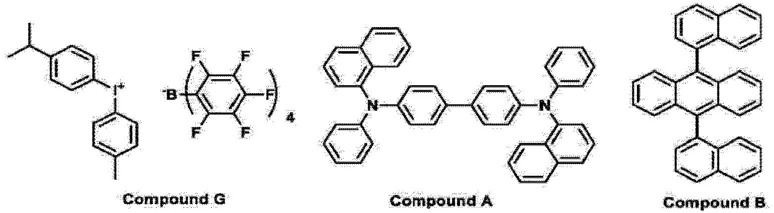

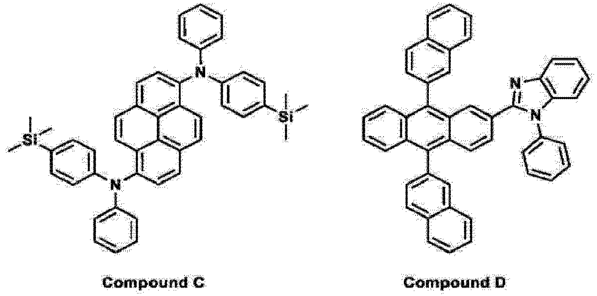

In the above process, the deposition rate of the organic material was maintained at 0.4 to 0.7 Å/sec, the deposition rate of lithium fluoride of the cathode was maintained at 0.3 Å/sec, the deposition rate of aluminum of the cathode was maintained at 2 Å/sec, and the vacuum degree during deposition was maintained at 2 × 10⁻⁷ to 5 × 10⁻⁸ torr.

### Experimental Examples 2 to 9.

Organic light emitting devices were manufactured in the same manner as in Experimental Example 1 above except that the compounds shown in Table 1 below were used instead of Compound 1 above when manufacturing a hole injection layer (HIL).

### Comparative Experimental Examples 1 to 3.

Organic light emitting devices were manufactured in the same manner as in Experimental Example 1 above except that any one compound of Compounds CE1 to CE3 below as shown in Table 1 below was used instead of Compound 1 above when manufacturing a hole injection layer.

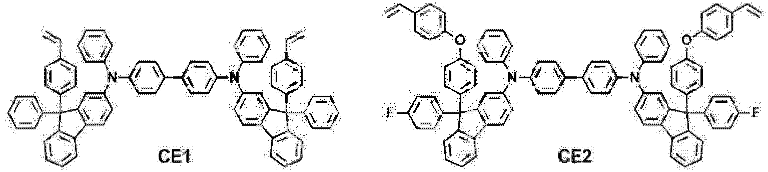

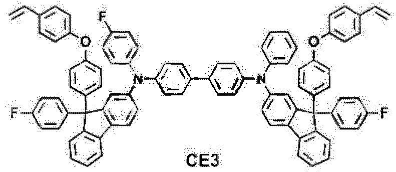

Table 1 below shows the results of measuring driving voltage, external quantum efficiency, luminance, and lifespan of the organic light emitting devices manufactured in Experimental Examples and Comparative Experimental Examples above at a current density of 10 mA/cm². The external quantum efficiency was obtained by (number of emitted photons)/(number of injected charge carriers). T95 refers to a time (hr) required for the luminance to decrease from the initial luminance (500 nit) to 95%.

**[Table 1]**

| | HIL host | Driving voltage (V) | External quantum efficiency (%) | Luminance (cd/m²) | T95(hr) @500 nit |
|---|---|---|---|---|---|
| Experimental Example 1 | Compound 1 | 4.58 | 5.6 | 576 | 183 |
| Experimental Example 2 | Compound 2 | 4.63 | 5.5 | 603 | 147 |
| Experimental Example 3 | Compound 3 | 4.59 | 5.7 | 610 | 153 |
| Experimental Example 4 | Compound 4 | 4.64 | 5.5 | 586 | 177 |
| Experimental Example 5 | Compound 5 | 4.71 | 5.2 | 590 | 157 |
| Experimental Example 6 | Compound 6 | 4.81 | 5.6 | 543 | 163 |
| Experimental Example 7 | Compound 7 | 4.73 | 5.2 | 527 | 154 |
| Experimental Example 8 | Compound 8 | 4.66 | 5.3 | 552 | 161 |
| Experimental Example 9 | Compound 9 | 4.52 | 5.4 | 571 | 170 |
| Comparative Experimental Example 1 | CE1 | 5.68 | 4.8 | 432 | 62 |
| Comparative Experimental Example 2 | CE2 | 5.55 | 4.9 | 470 | 85 |
| Comparative Experimental Example 3 | CE3 | 4.78 | 5.1 | 503 | 98 |

Experimental Examples 1 to 9 use the compounds represented by Chemical Formula 1 according to the present disclosure as a host for the hole injection layer, and Comparative Experimental Examples 1 to 3 use Compound CE1 to which a fluoro group (-F) is not bonded, Compound CE2 in which a fluoro group (-F) is bonded only to fluorene, or Compound CE3 in which a fluoro group (-F) is not bonded to Ar₂ of Chemical Formula 1.

As shown in Table 1 above, it could be confirmed that the organic light emitting devices using the compounds represented by Chemical Formula 1 according to the present disclosure as a host for the hole injection layer have greatly reduced driving voltages and greatly improved lifespans compared to the organic light emitting devices of Comparative Experimental Examples.

Further, the organic light emitting devices of Experimental Examples were shown to increase the efficiency and luminance compared to the organic light emitting devices of Comparative Experimental Examples. Through this, it could be confirmed that the performances of the organic light emitting devices were improved by improving the film and interfacial properties by the fluoro group (-F) introduced into the molecule, and improving the hole mobility and the hole/electron balance due to the change in the HOMO level.

## Claims

1. A compound represented by the following Chemical Formula 1: 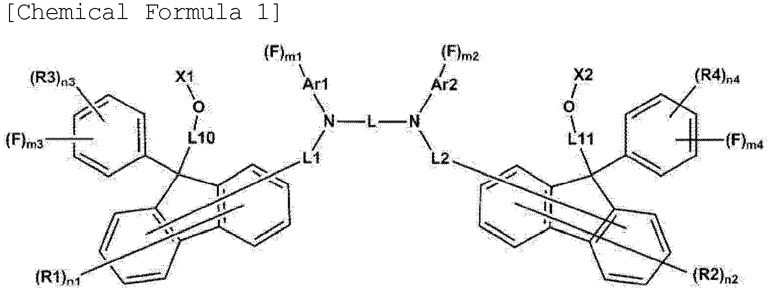 in Chemical Formula 1,
L is a substituted or unsubstituted divalent hydrocarbon ring group; or a substituted or unsubstituted divalent heterocyclic group,
L1 and L2 are the same as or different from each other, and are each independently a direct bond; a substituted or unsubstituted arylene group; or a substituted or unsubstituted heteroarylene group,
Ar1 and Ar2 are the same as or different from each other, and are each independently a substituted or unsubstituted aryl group; or a substituted or unsubstituted heteroaryl group,
L10 and L11 are the same as or different from each other, and are each independently a substituted or unsubstituted arylene group,
X1 and X2 are the same as or different from each other, and are each independently a curable group,
R1 to R4 are the same as or different from each other, and are each independently hydrogen; heavy hydrogen; a halogen group; a substituted or unsubstituted alkyl group; a substituted or unsubstituted alkoxy group; a substituted or unsubstituted aryl group; or a substituted or unsubstituted heteroaryl group,
n1 and n2 are each independently an integer of 0 to 7, and when n1 and n2 are each 2 or more, each occurrence of R1 and R2 is the same as or different from each other,
m1 is 1 to the number of bonding positions at which a fluorine (F) is bonded to Ar1,
m2 is 1 to the number of bonding positions at which a fluorine (F) is bonded to Ar2,
n3 and n4 are each independently an integer of 0 to 4, and when n3 and n4 are each 2 or more, each occurrence of R3 and R4 is the same as or different from each other,
m3 and m4 are each independently an integer of 1 to 5, n3+m3 is 5 or less, and n4+m₄ is 5 or less.

2. The compound of claim 1, wherein the curable group is any one selected from the following structures, and --- in the following structures means a bonding position:

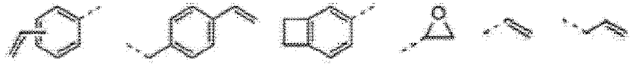

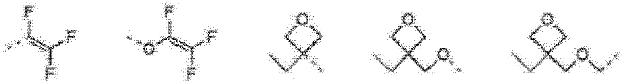

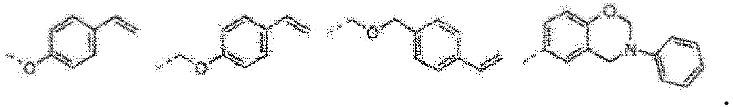

3. The compound of claim 1, wherein Chemical Formula 1 above is represented by the following Chemical Formula 2: 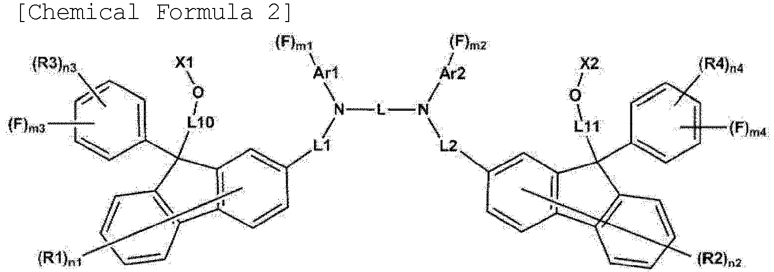 in Chemical Formula 2,
L, L1, L2, Ar1, Ar2, L10, L11, X1, X2, R1 to R4, n1 to n4, and m1 to m4 are defined as in Chemical Formula 1.

4. The compound of claim 1, wherein Chemical Formula 1 above is represented by the following Chemical Formula 3: 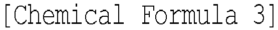 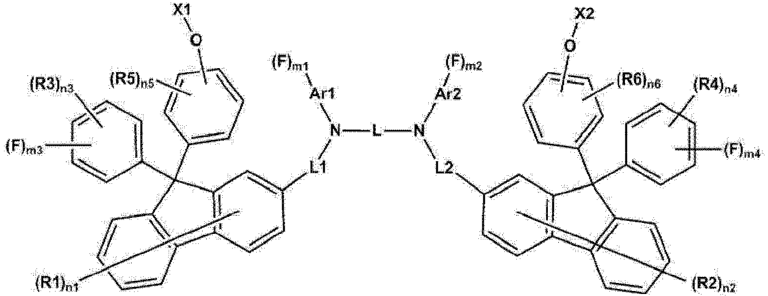 in Chemical Formula 3,
L, L1, L2, Ar1, Ar2, X1, X2, R1 to R4, n1 to n4, and m1 to m4 are defined as in Chemical Formula 1,
R5 and R6 are the same as or different from each other, and are each independently hydrogen; heavy hydrogen; a halogen group; a substituted or unsubstituted alkyl group; a substituted or unsubstituted alkoxy group; a substituted or unsubstituted aryl group; or a substituted or unsubstituted heteroaryl group,
n5 and n6 are each independently an integer of 0 to 4, and when n5 and n6 are each 2 or more, each occurrence of R5 and R6 is the same as or different from each other.

5. The compound of claim 1, wherein L is a C₆-C₃₀ divalent hydrocarbon ring group unsubstituted or substituted with a C₁-C₂₀ alkyl group.

6. The compound of claim 1, wherein L is any one selected from the following structures: 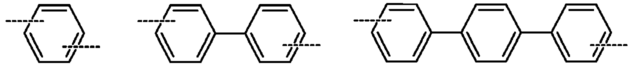 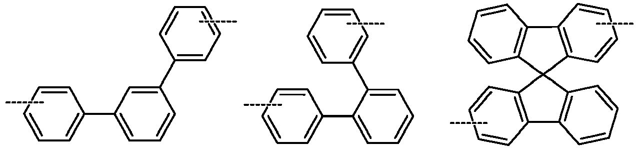 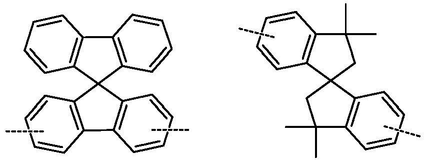
each of which is optionally substituted with one or more substituents selected from the group consisting of heavy hydrogen; a halogen group; a substituted or unsubstituted alkyl group; a substituted or unsubstituted alkoxy group; a substituted or unsubstituted aryl group; and a substituted or unsubstituted heteroaryl group, and
... denotes a bonding position.

7. The compound of claim 1, wherein Ar1 and Ar2 are the same as or different from each other, and are each independently a substituted or unsubstituted phenyl group; a substituted or unsubstituted biphenyl group; or a substituted or unsubstituted terphenyl group.

8. The compound of claim 1, wherein m1 and m2 are each independently an integer of 1 to 5.

9. The compound of claim 1, wherein Chemical Formula 1 above is represented by any one of the following compounds:

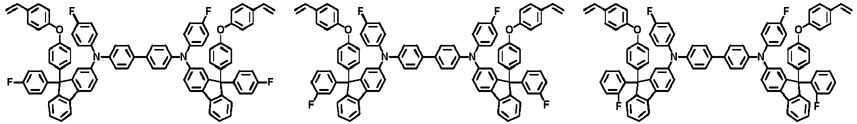

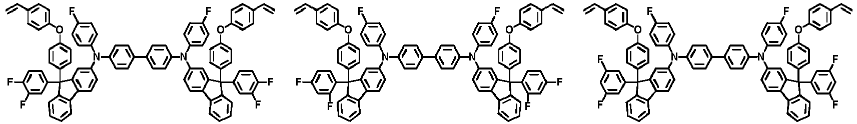

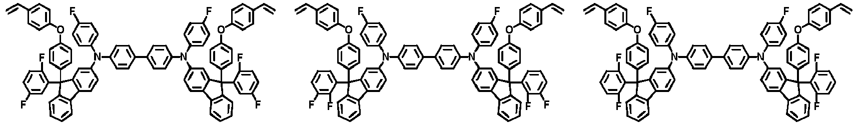

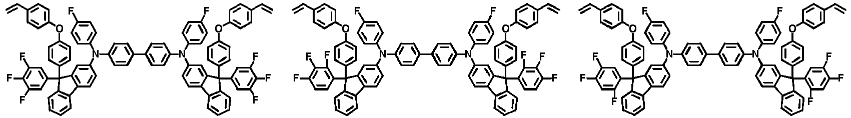

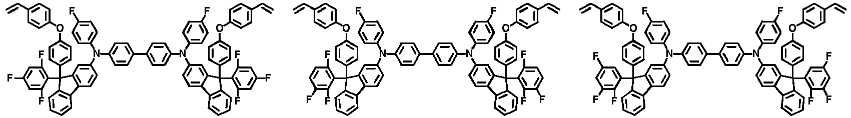

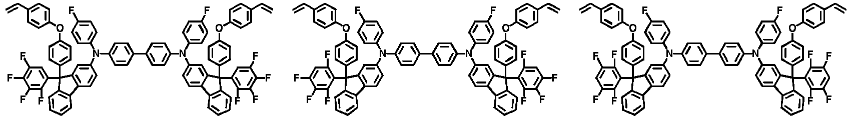

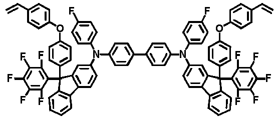

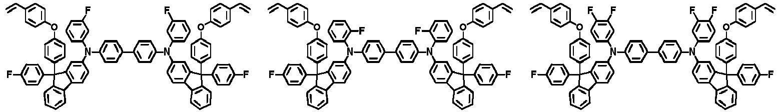

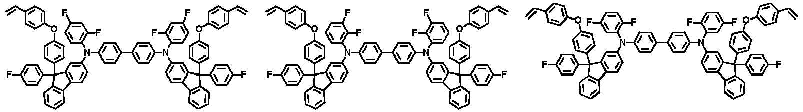

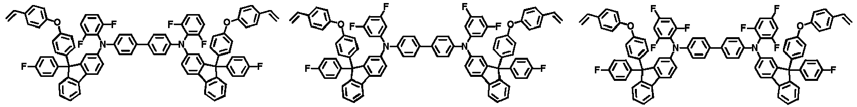

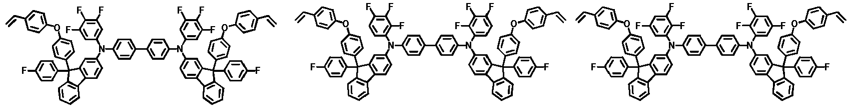

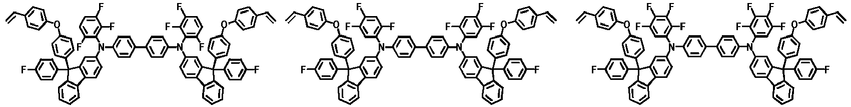

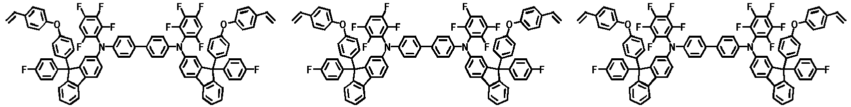

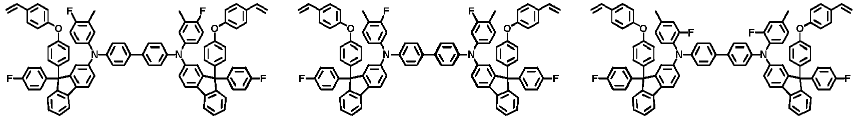

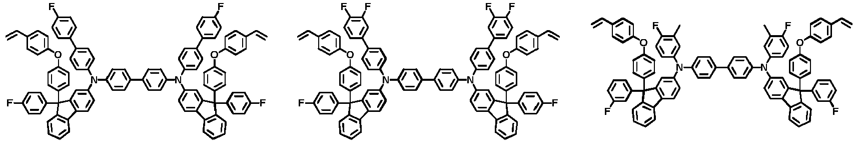

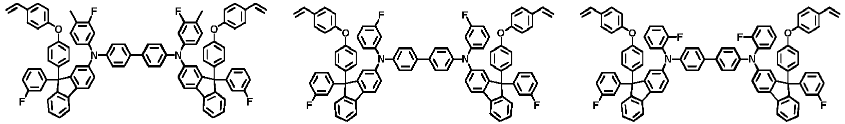

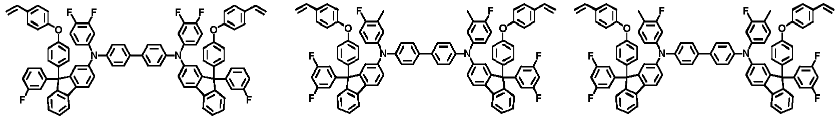

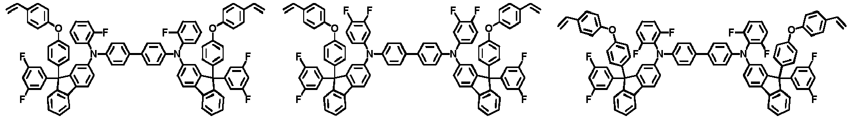

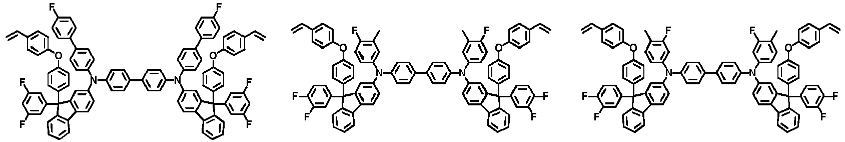

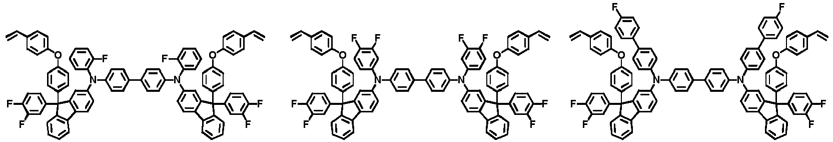

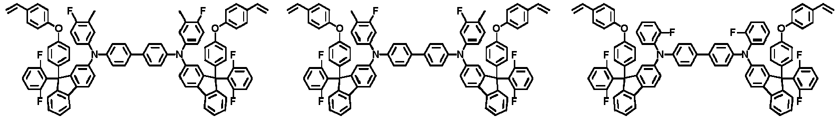

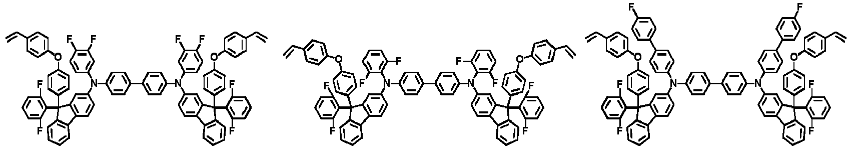

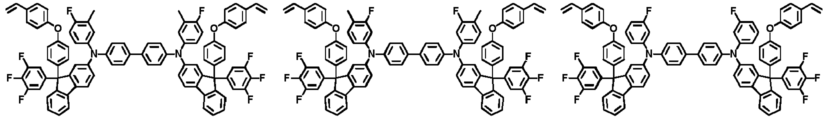

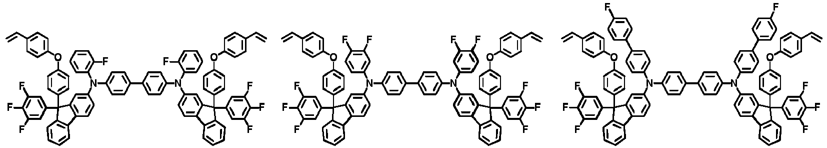

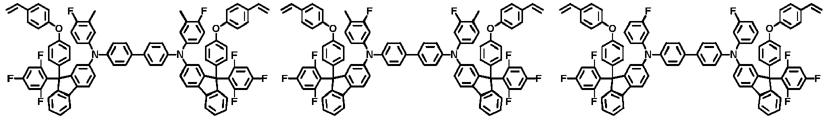

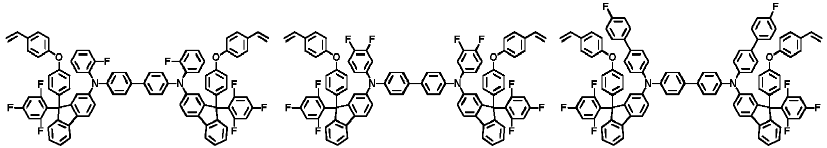

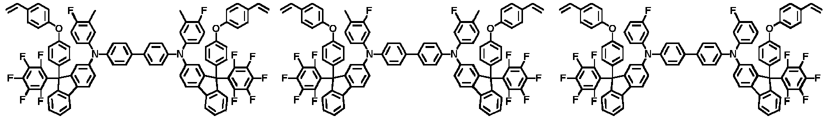

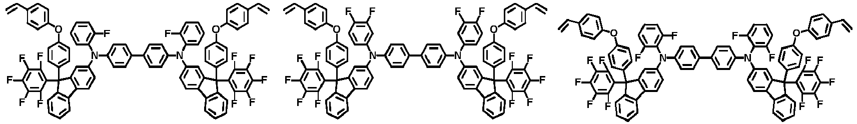

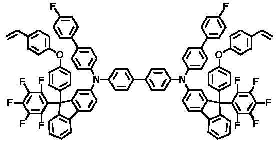

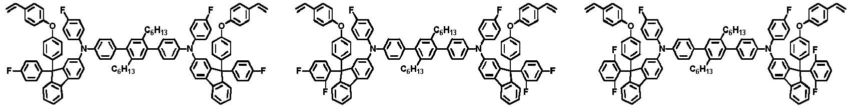

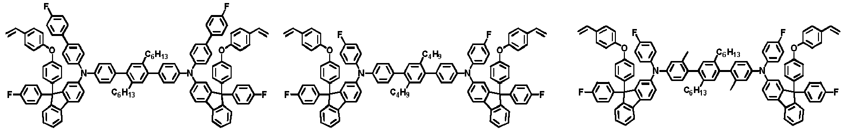

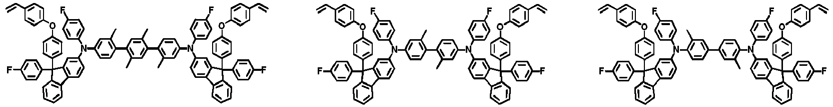

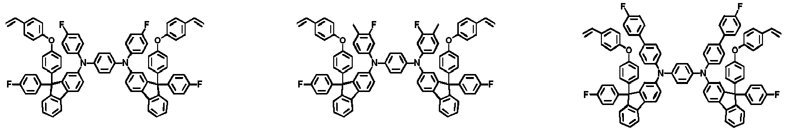

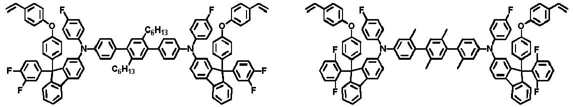

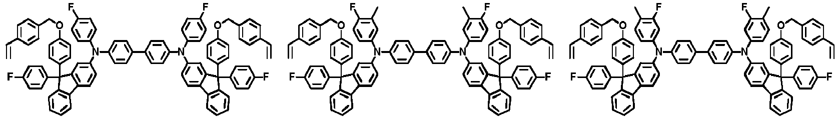

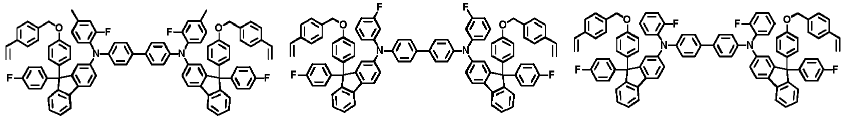

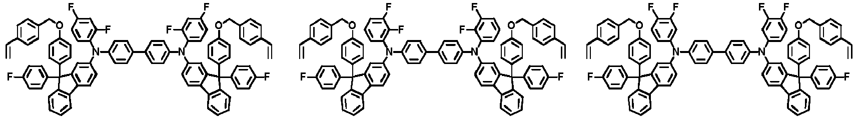

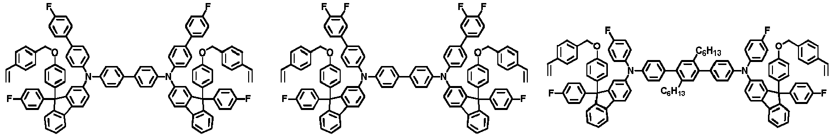

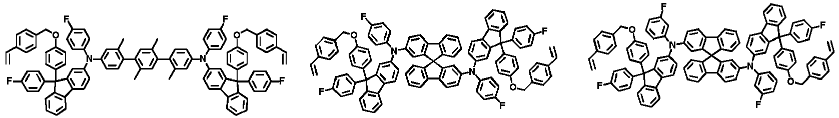

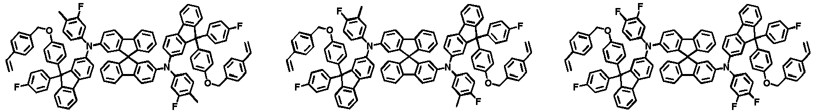

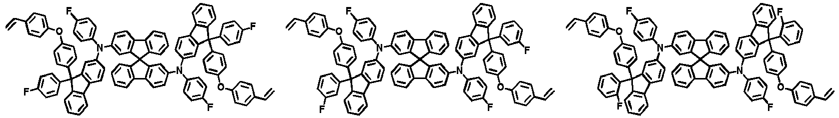

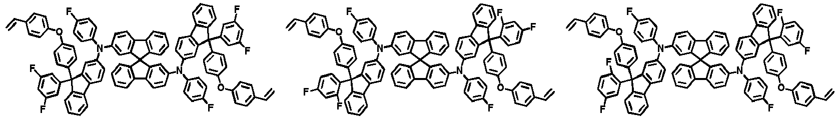

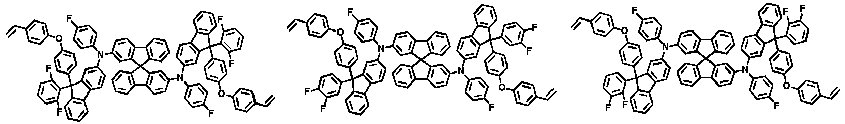

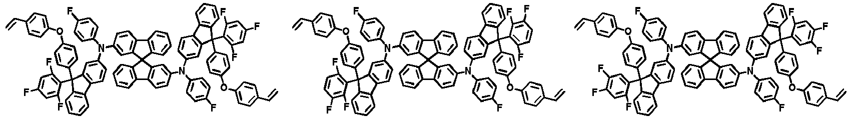

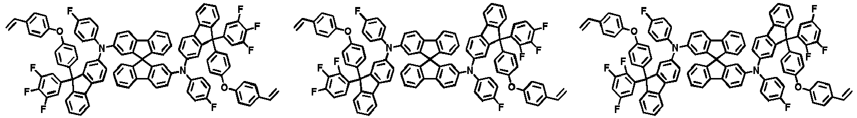

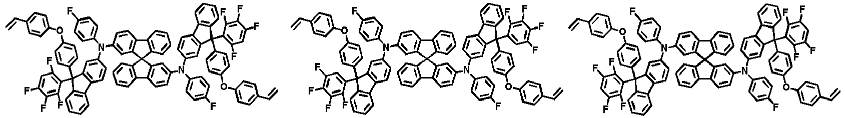

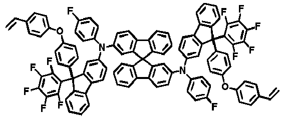

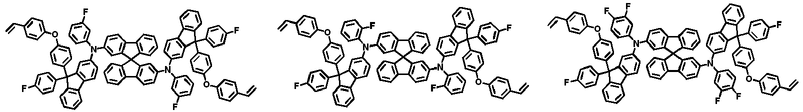

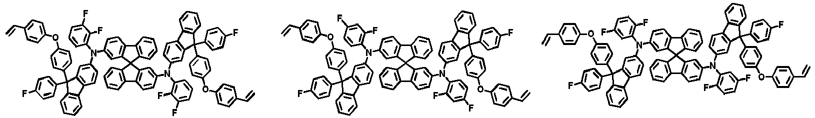

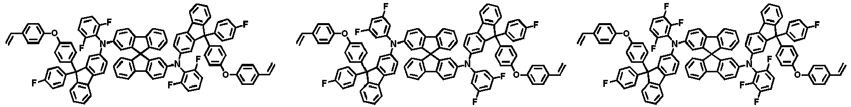

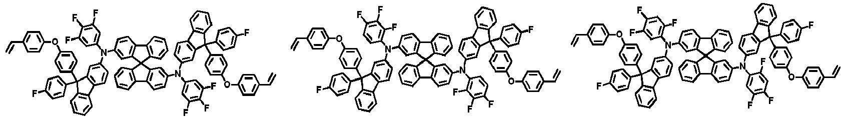

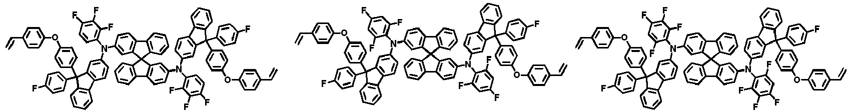

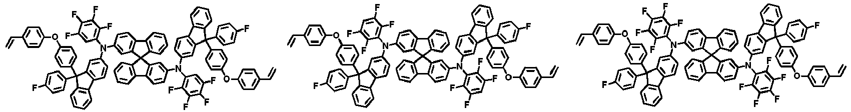

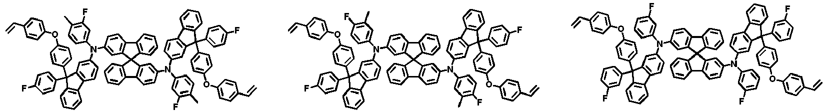

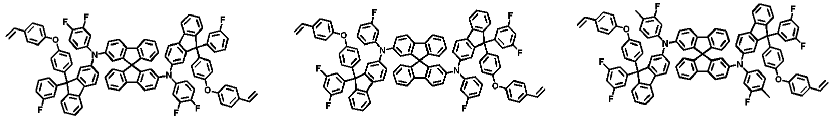

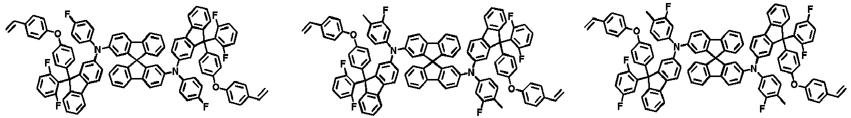

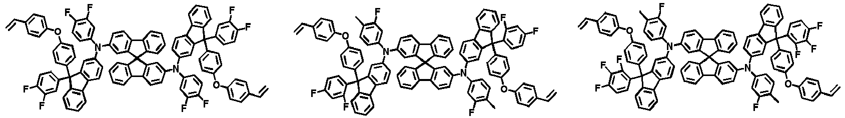

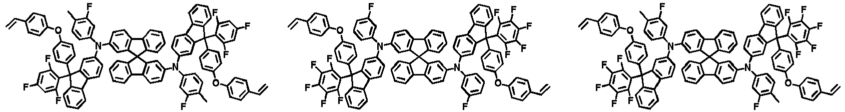

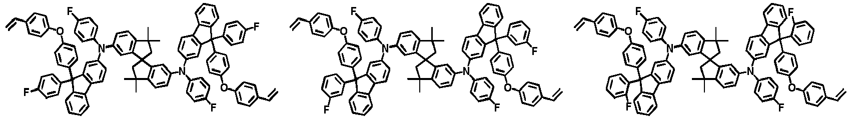

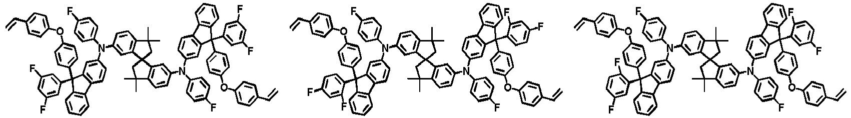

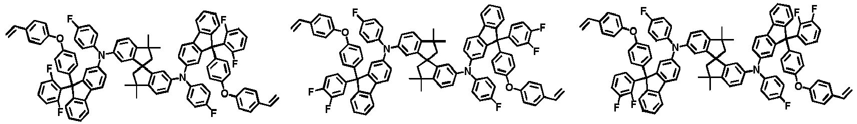

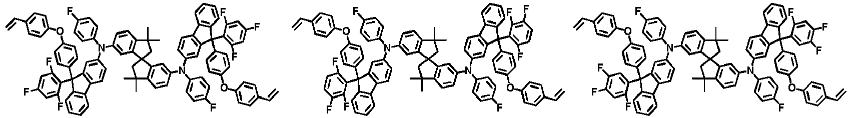

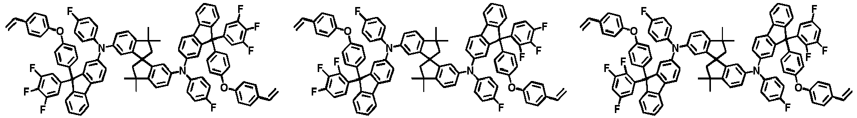

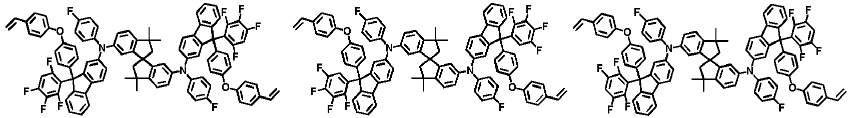

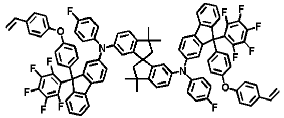

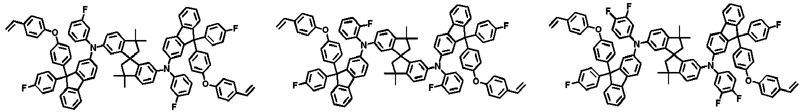

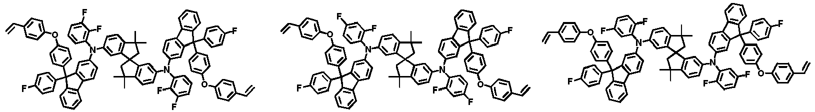

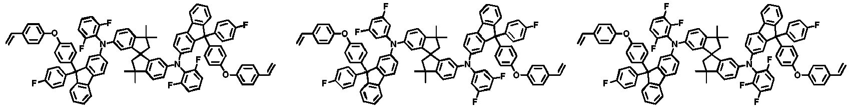

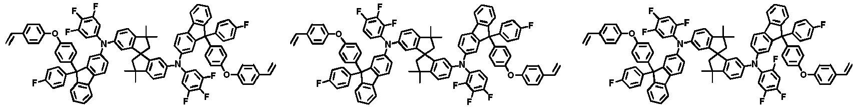

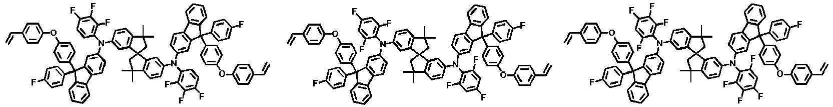

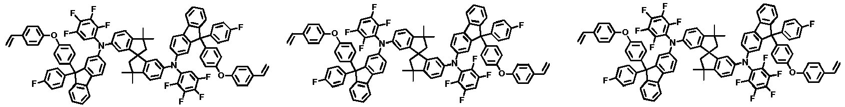

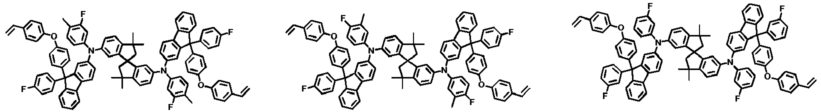

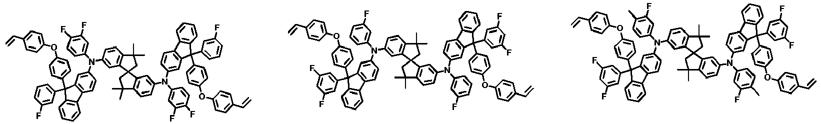

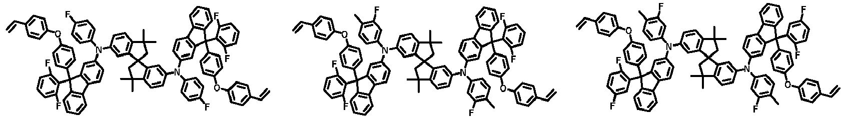

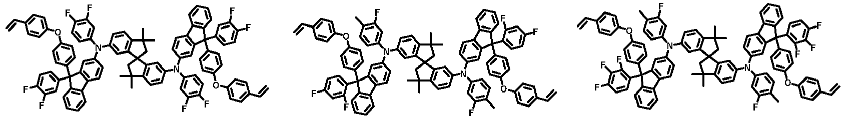

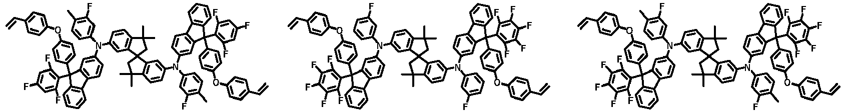

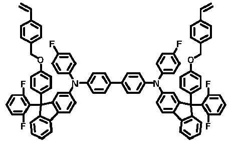

10. A coating composition comprising the compound according to any one of claims 1 to 9.

11. An organic light emitting device comprising:
a first electrode;
a second electrode; and
an organic material layer provided between the first electrode and the second electrode,
wherein one or more layers of the organic material layer contain the coating composition of claim 10 or a cured product thereof.

12. The organic light emitting device of claim 11, wherein the one or more layers containing the coating composition or a cured product thereof is a hole transport layer or a hole injection layer.

13. A method for manufacturing an organic light emitting device, the method comprising the steps of:
preparing a first electrode;
forming an organic material layer on the first electrode; and
forming a second electrode on the organic material layer,
wherein the step of forming an organic material layer comprises a step of forming one or more layers using the coating composition of claim 10.

14. The method of claim 13, wherein the step of forming the one or more layers using the coating composition comprises the steps of: coating the coating composition; and heat-treating or light-treating the coated coating composition.

## Patentansprüche

1. Verbindung, die durch die folgende chemische Formel 1 dargestellt ist: 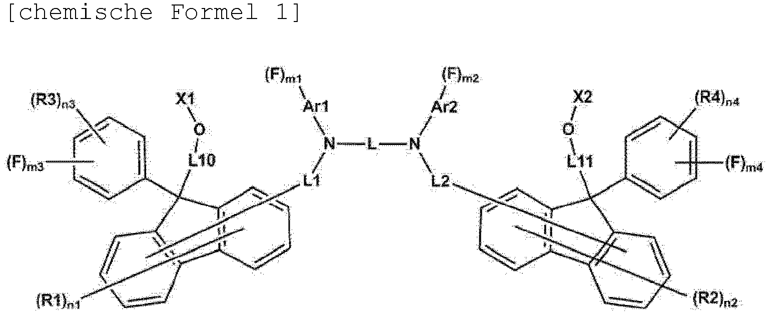 worin, in der chemischen Formel 1,
L eine substituierte oder unsubstituierte zweiwertige Kohlenwasserstoffringgruppe; oder eine substituierte oder unsubstituierte zweiwertige heterocyclische Gruppe ist,
L1 und L2 gleich sind oder sich voneinander unterscheiden und jeweils unabhängig eine direkte Bindung; eine substituierte oder unsubstituierte Arylengruppe; oder eine substituierte oder unsubstituierte Heteroarylengruppe darstellen,
Ar1 und Ar2 gleich sind oder sich voneinander unterscheiden und jeweils unabhängig eine substituierte oder unsubstituierte Arylgruppe; oder eine substituierte oder unsubstituierte Heteroarylgruppe darstellen,
L10 und L11 gleich sind oder sich voneinander unterscheiden und jeweils unabhängig eine substituierte oder unsubstituierte Arylengruppe sind,
X1 und X2 gleich sind oder sich voneinander unterscheiden und jeweils unabhängig eine härtbare Gruppe sind,
R1 bis R4 gleich sind oder sich voneinander unterscheiden und jeweils unabhängig Wasserstoff; schwerer Wasserstoff; eine Halogengruppe; eine substituierte oder unsubstituierte Alkylgruppe; eine substituierte oder unsubstituierte Alkoxygruppe; eine substituierte oder unsubstituierte Arylgruppe; oder eine substituierte oder unsubstituierte Heteroarylgruppe sind,
n1 und n2 jeweils unabhängig eine ganze Zahl von 0 bis 7 sind, und wenn n1 und n2 jeweils 2 oder mehr sind, jedes Vorkommen von R1 und R2 gleich ist oder sich voneinander unterscheidet,
m1 1 bis zur Anzahl der Bindungspositionen, an denen ein Fluor (F) an Ar1 gebunden ist, ist,
m2 1 bis zur Anzahl der Bindungspositionen, an denen ein Fluor (F) an Ar2 gebunden ist, ist,
n3 und n4 jeweils unabhängig eine ganze Zahl von 0 bis 4 sind, und wenn n3 und n4 jeweils 2 oder mehr sind, jedes Vorkommen von R3 und R4 gleich ist oder sich voneinander unterscheidet,
m3 und m4 jeweils unabhängig eine ganze Zahl von 1 bis 5 sind, n3+m3 5 oder weniger ist und n4+m4 5 oder weniger ist.

2. Verbindung gemäß Anspruch 1, worin die härtbare Gruppe eine Gruppe ist, die aus den folgenden Strukturen ausgewählt ist, und --- in den folgenden Strukturen eine Bindungsposition darstellt:

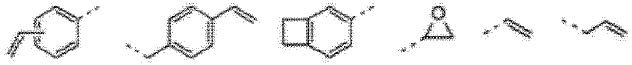

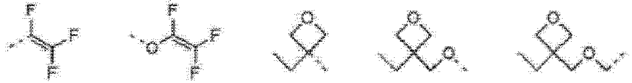

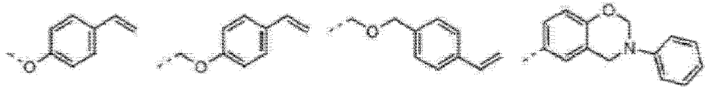

3. Verbindung gemäß Anspruch 1, worin die obige chemische Formel 1 durch die folgende chemische Formel 2 dargestellt ist: 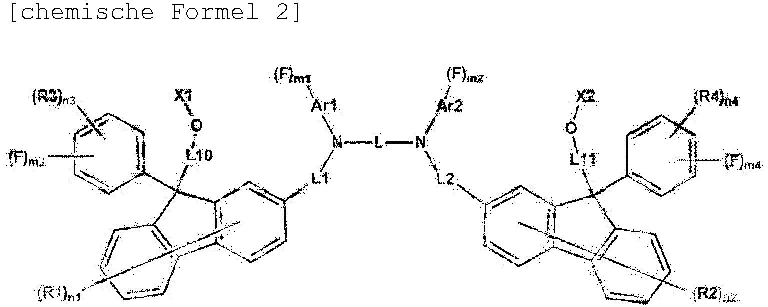 worin, in der chemischen Formel 2,
L, L1, L2, Ar1, Ar2, L10, L11, X1, X2, R1 bis R4, n1 bis n4 und m1 bis m4 wie in der chemischen Formel 1 definiert sind.

4. Verbindung gemäß Anspruch 1, worin die obige chemische Formel 1 durch die folgende chemische Formel 3 dargestellt ist: 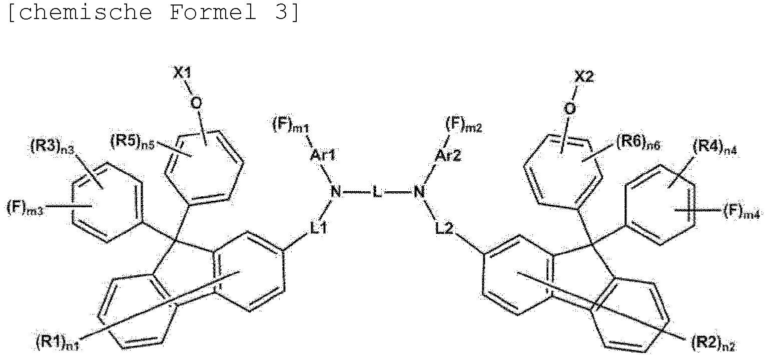 worin, in der chemischen Formel 3,
L, L1, L2, Ar1, Ar2, L10, L11, X1, X2, R1 bis R4, n1 bis n4 und m1 bis m4 wie in der chemischen Formel 1 definiert sind,
R5 und R6 gleich sind oder sich voneinander unterscheiden und jeweils unabhängig Wasserstoff; schwerer Wasserstoff; eine Halogengruppe; eine substituierte oder unsubstituierte Alkylgruppe; eine substituierte oder unsubstituierte Alkoxygruppe; eine substituierte oder unsubstituierte Arylgruppe; oder eine substituierte oder unsubstituierte Heteroarylgruppe sind,
n5 und n6 jeweils unabhängig eine ganze Zahl von 0 bis 4 sind, und wenn n5 und n6 jeweils 2 oder mehr sind, jedes Vorkommen von R5 und R6 gleich ist oder sich voneinander unterscheidet.

5. Verbindung gemäß Anspruch 1, worin L eine zweiwertige C₆-C₃₀-Kohlenwasserstoffringruppe ist, die unsubstituiert oder mit einer C₁-C₂₀-Alkylgruppe substituiert ist.

6. Verbindung gemäß Anspruch 1, worin L aus den folgenden Strukturen ausgewählt ist: 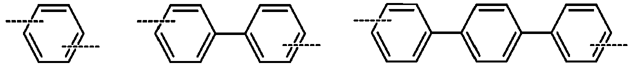 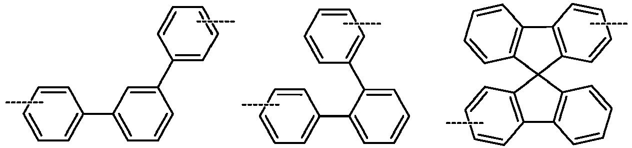 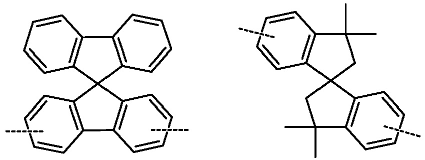
von denen jeweils jede gegebenenfalls mit einem oder mehreren Substituenten substituiert ist, ausgewählt aus der Gruppe, bestehend aus schwerem Wasserstoff; einer Halogengruppe; einer substituierten oder unsubstituierten Alkylgruppe; einer substituierten oder unsubstituierten Alkoxygruppe; einer substituierten oder unsubstituierten Arylgruppe; und einer substituierten oder unsubstituierten, und
... eine Bindungsposition angibt.

7. Verbindung gemäß Anspruch 1, worin Ar1 und Ar1 gleich sind oder sich voneinander unterscheiden und jeweils unabhängig eine substituierte oder unsubstituierte Phenylgruppe; eine substituierte oder unsubstituierte Biphenylgruppe; oder eine substituierte oder unsubstituierte Terphenylgruppe sind.

8. Verbindung gemäß Anspruch 1, worin m1 und m2 jeweils unabhängig eine ganze Zahl von 1 bis 5 sind.

9. Verbindung gemäß Anspruch 1, worin die obige chemische Formel 1 durch eine der folgenden Verbindungen dargestellt ist:

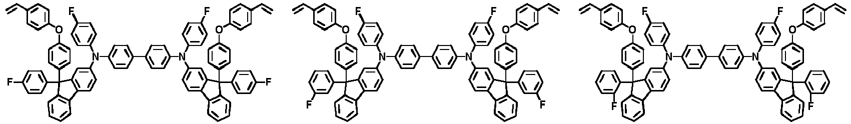

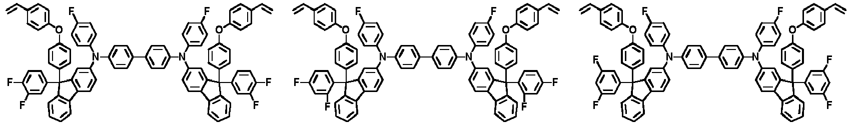

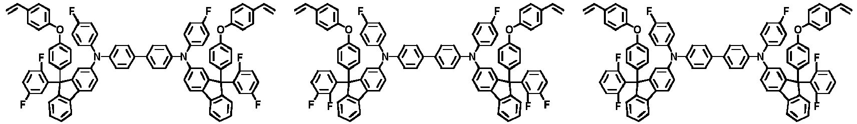

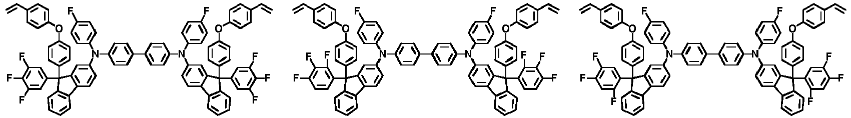

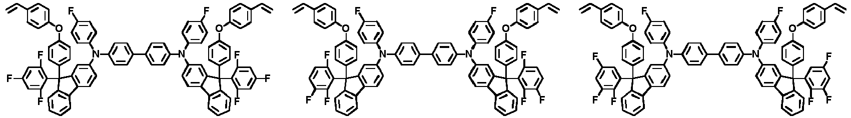

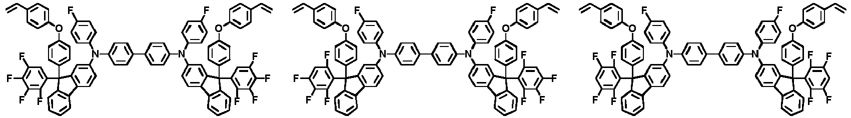

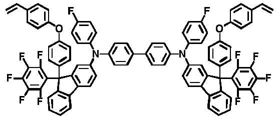

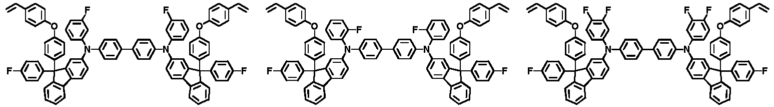

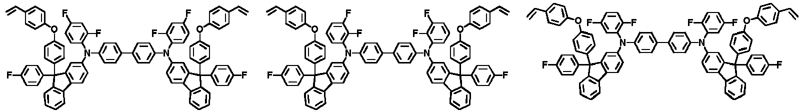

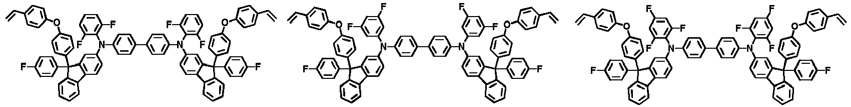

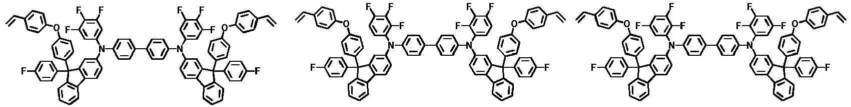

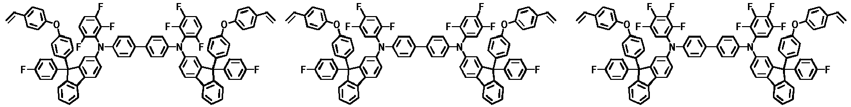

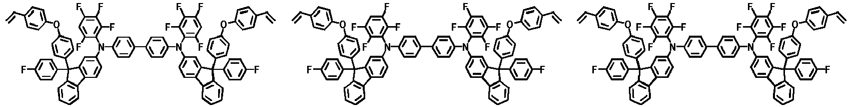

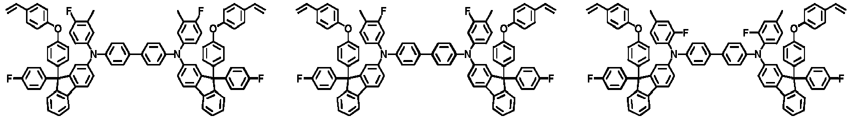

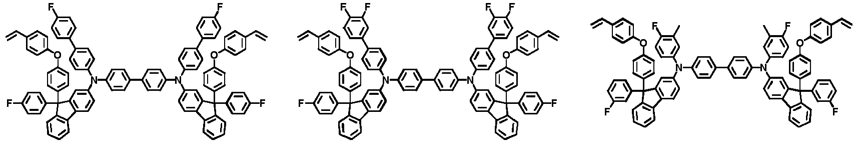

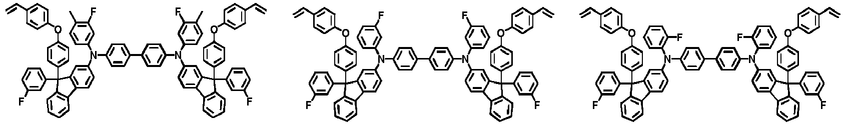

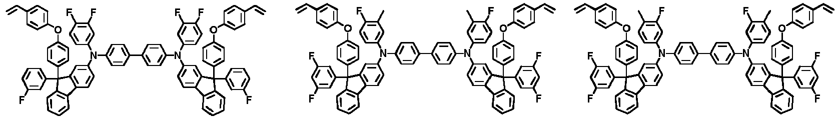

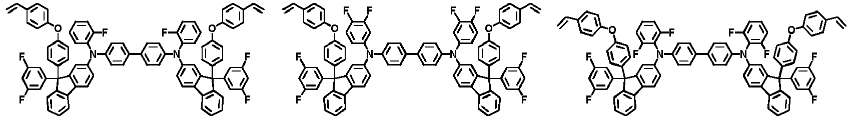

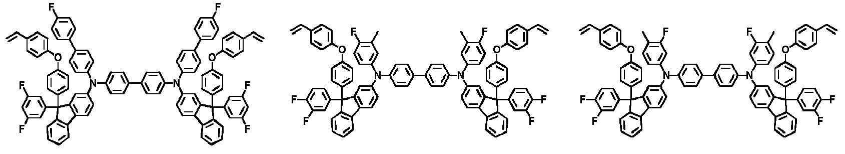

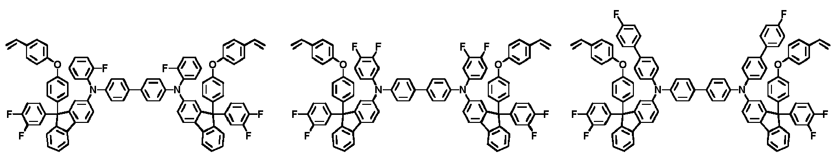

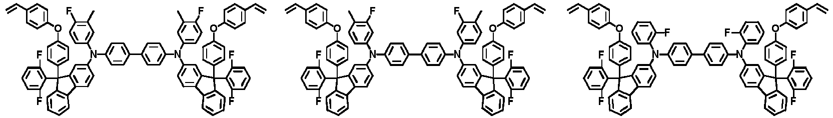

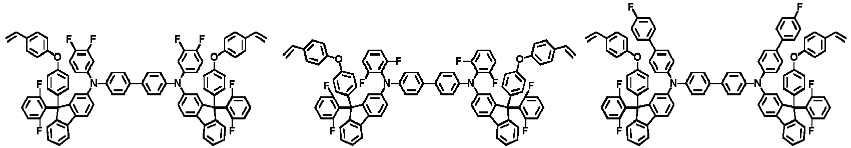

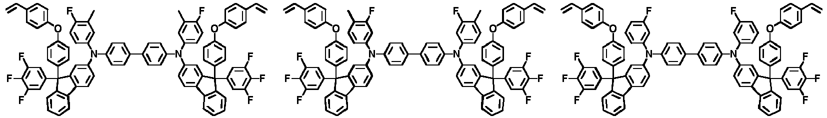

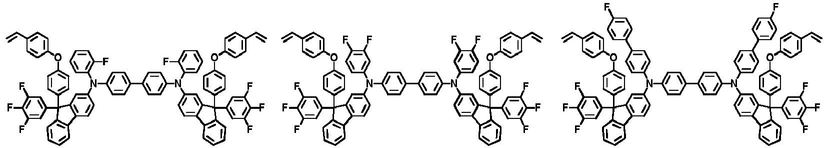

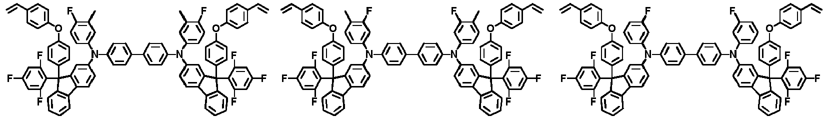

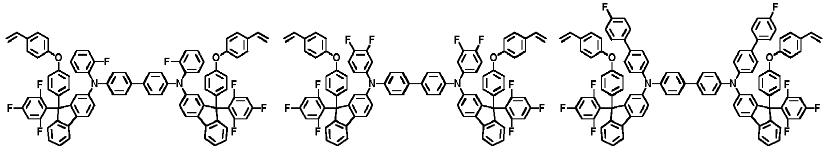

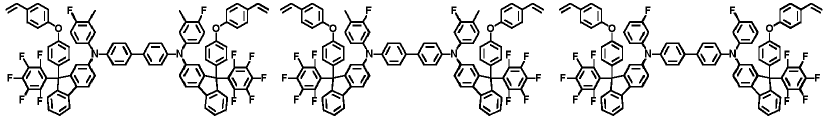

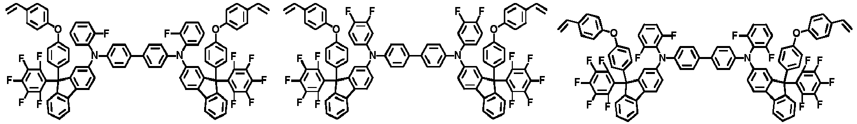

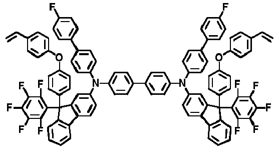

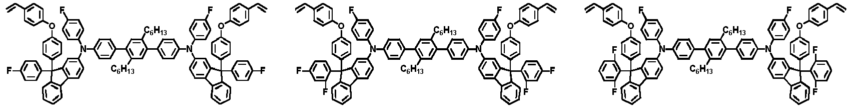

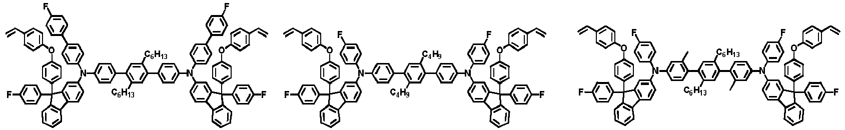

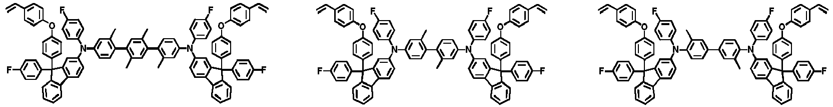

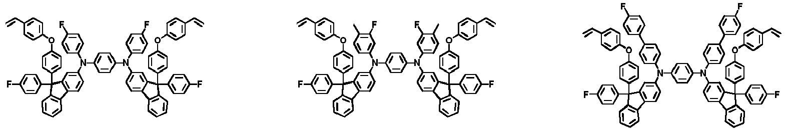

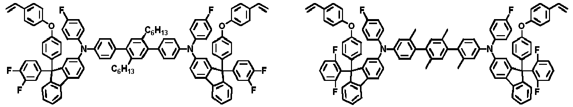

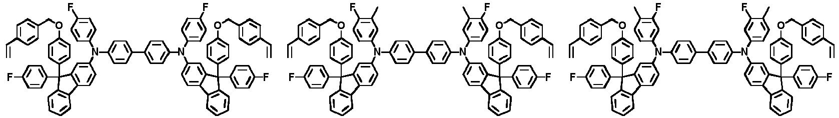

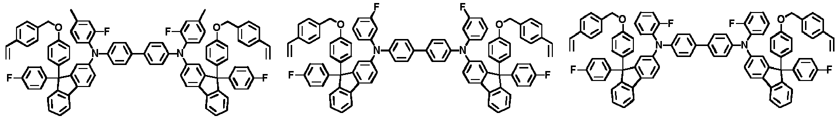

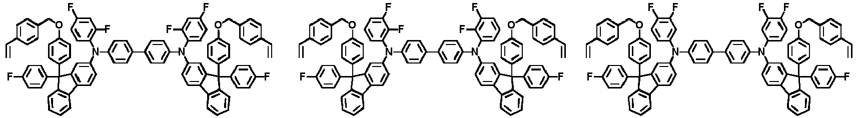

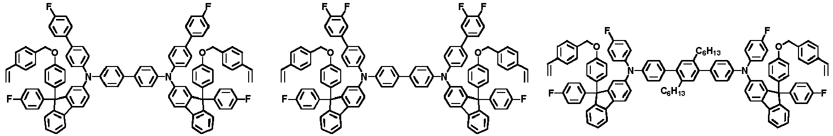

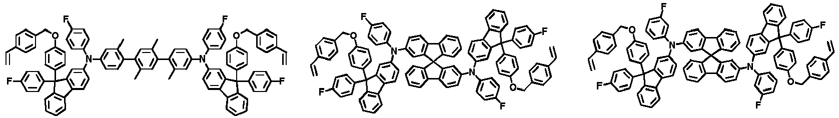

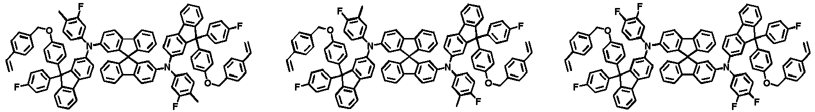

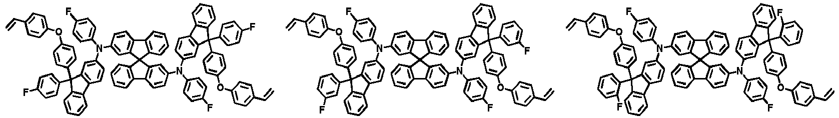

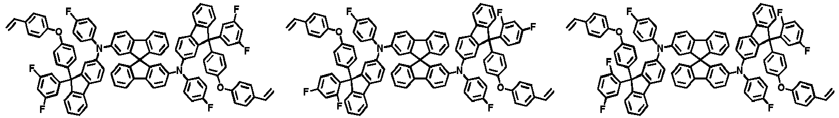

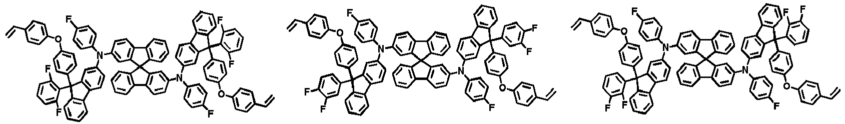

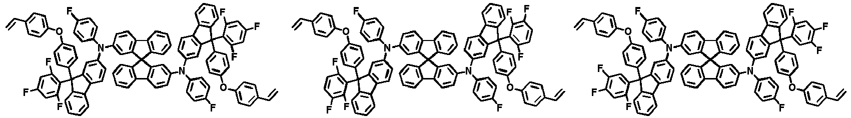

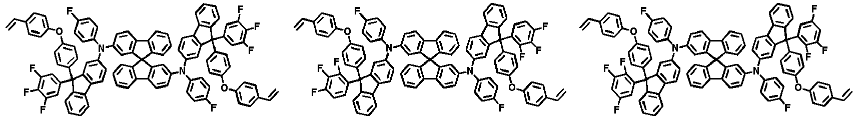

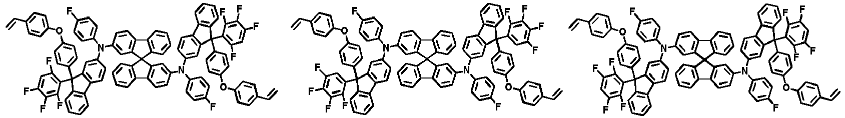

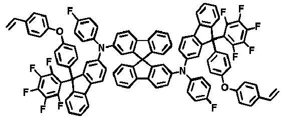

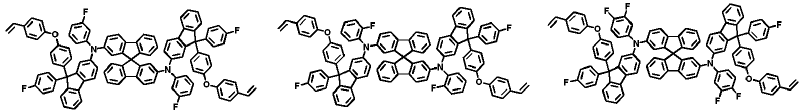

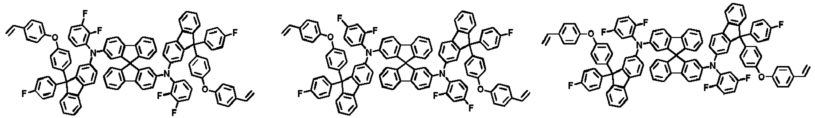

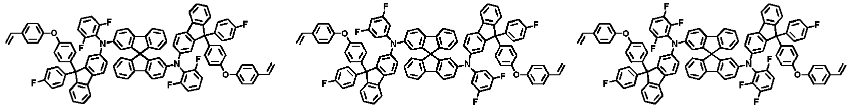

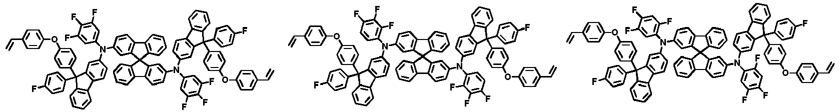

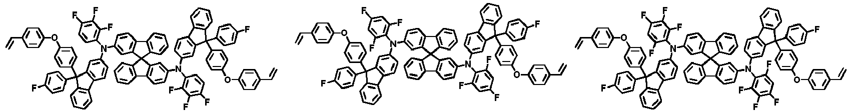

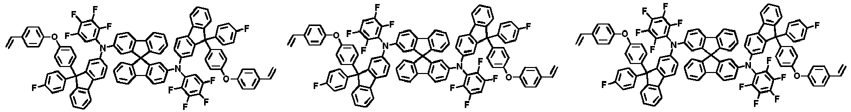

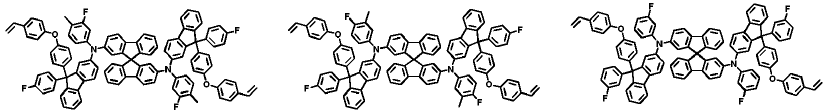

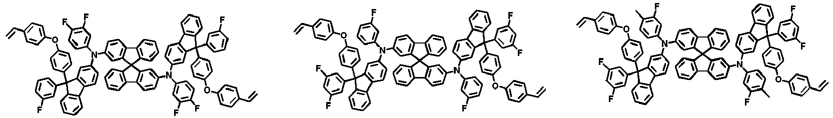

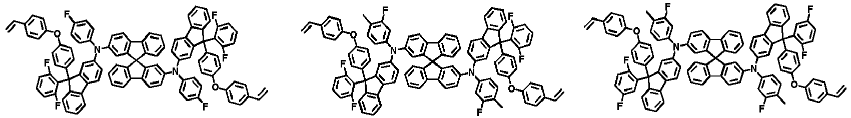

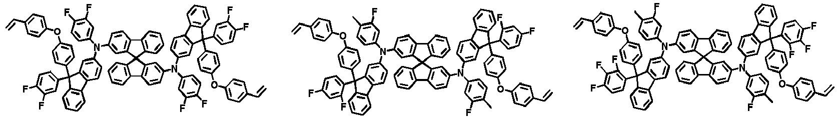

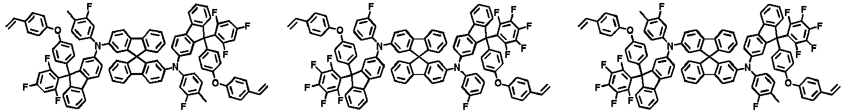

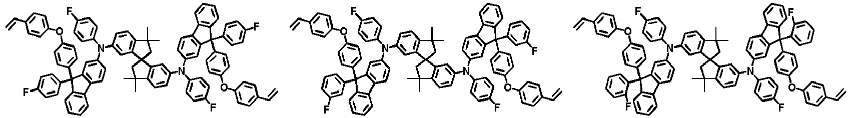

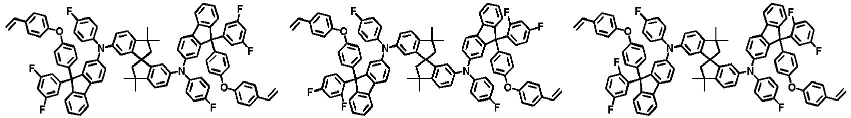

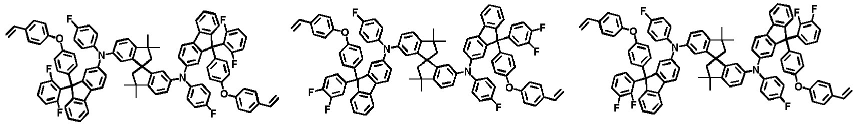

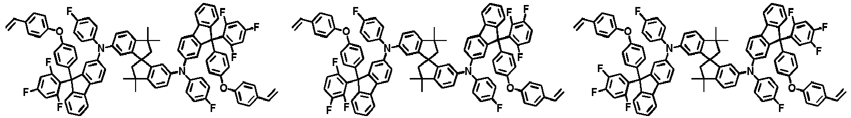

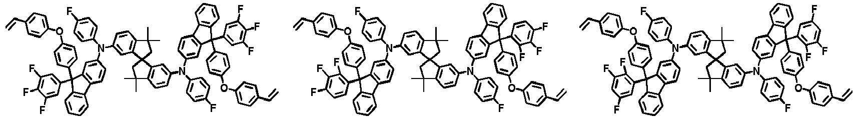

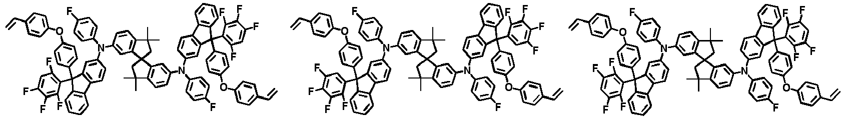

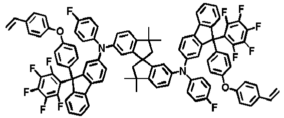

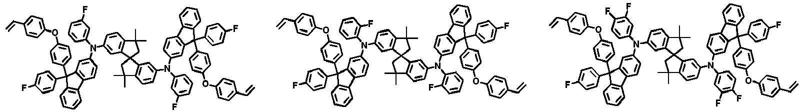

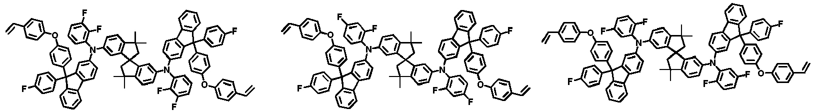

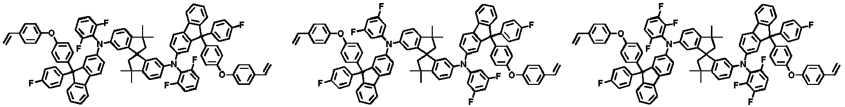

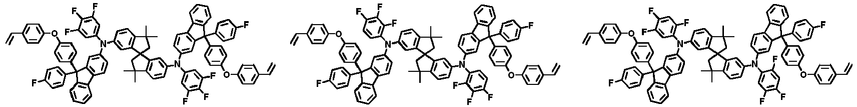

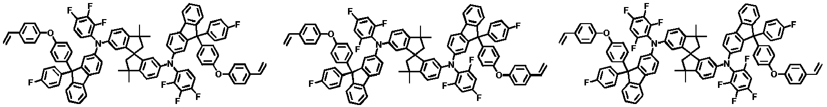

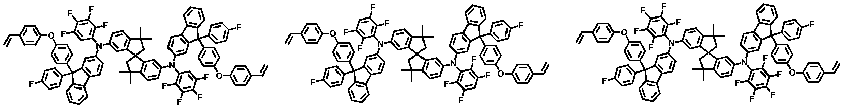

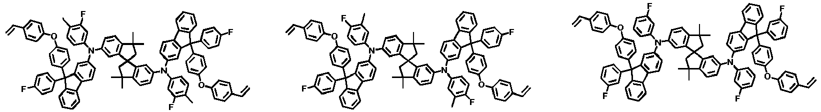

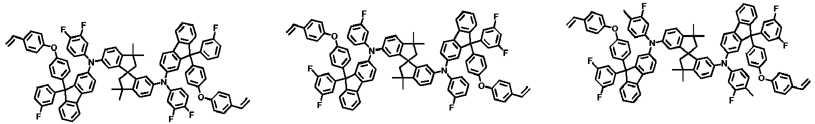

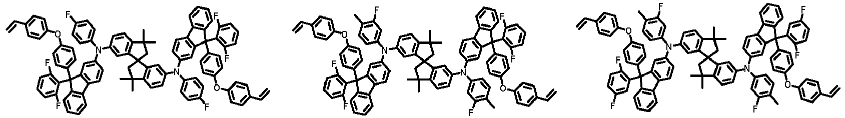

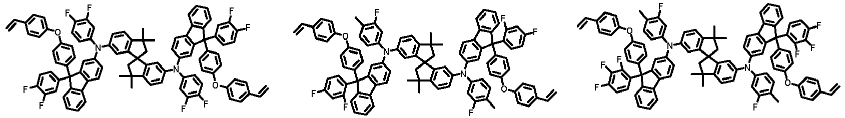

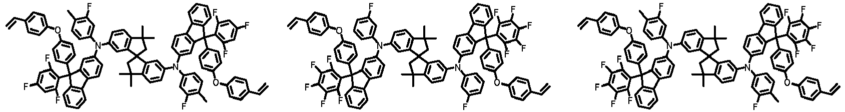

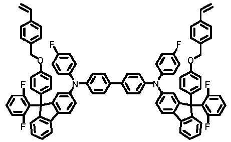

10. Beschichtungszusammensetzung, umfassend die Verbindung gemäß einem der Ansprüche 1 bis 9.

11. Organische lichtemittierende Vorrichtung, umfassend:
eine erste Elektrode;
eine zweite Elektrode; und
eine Schicht aus organischem Material, das zwischen der ersten Elektrode und der zweiten Elektrode bereitgestellt ist,
worin eine oder mehrere Schichten der Schicht aus organischem Material die Beschichtungszusammensetzung gemäß Anspruch 10 oder ein gehärtetes Produkt davon enthalten.

12. Organische lichtemittierende Vorrichtung gemäß Anspruch 11, worin die eine oder mehreren Schichten, die die Beschichtungszusammensetzung oder ein gehärtetes Produkt davon enthalten, eine Lochtransportschicht oder eine Lochinjektionsschicht ist.

13. Verfahren zur Herstellung einer organischen lichtemittierenden Vorrichtung, das Verfahren umfassend die Schritte:
des Bereitens einer ersten Elektrode;
des Ausbildens einer Schicht aus organischem Material auf der ersten Elektrode; und
des Ausbildens einer zweiten Elektrode auf der Schicht aus organischem Material,
worin der Schritt des Ausbildens einer Schicht aus organischem Material einen Schritt des Ausbildens einer oder mehrerer Schichten unter Verwendung der Beschichtungszusammensetzung gemäß Anspruch 10 umfasst.

14. Verfahren gemäß Anspruch 13, worin der Schritt des Ausbildens einer oder mehrerer Schichten unter Verwendung der Beschichtungszusammensetzung die Schritte umfasst: des Beschichtens der Beschichtungszusammensetzung; und des Wärmebehandelns oder Lichtbehandelns der beschichteten Beschichtungszusammensetzung.

## Revendications

1. Composé représenté par la Formule chimique 1 suivante : 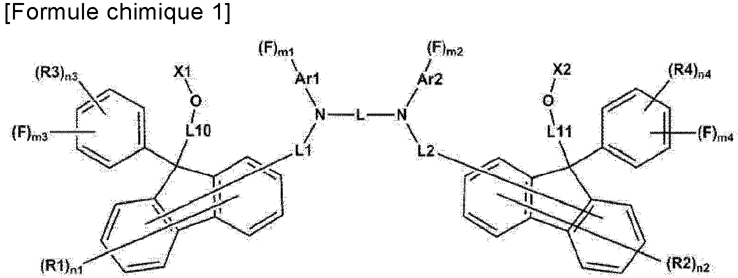 dans la Formule chimique 1,
L est un groupe de noyau hydrocarboné divalent substitué ou non substitué ; ou un groupe hétérocyclique divalent substitué ou non substitué,
L1 et L2 sont identiques l'un à l'autre ou différents l'un de l'autre, et sont chacun indépendamment une liaison directe ; un groupe arylène substitué ou non substitué ; ou un groupe hétéroarylène substitué ou non substitué,
Ar1 et Ar2 sont identiques l'un à l'autre ou différents l'un de l'autre, et sont chacun indépendamment un groupe aryle substitué ou non substitué ; ou un groupe hétéroaryle substitué ou non substitué,
L10 et L11 sont identiques l'un à l'autre ou différents l'un de l'autre, et sont chacun indépendamment un groupe arylène substitué ou non substitué,
X1 et X2 sont identiques l'un à l'autre ou différents l'un de l'autre, et sont chacun indépendamment un groupe durcissable,
R1 à R4 sont identiques les uns aux autres ou différents les uns des autres, et sont chacun indépendamment un hydrogène ; un hydrogène lourd ; un groupe halogéné ; un groupe alkyle substitué ou non substitué ; un groupe alcoxy substitué ou non substitué ; un groupe aryle substitué ou non substitué ; ou un groupe hétéroaryle substitué ou non substitué,
n1 et n2 sont chacun indépendamment un nombre entier de 0 à 7, et lorsque n1 et n2 valent chacun 2 ou plus, chaque occurrence de R1 et R2 est identique l'une à l'autre ou différente l'une de l'autre,
m1 vaut de 1 au nombre de positions de liaison au niveau desquelles un fluor (F) est lié à Ar1,
m2 vaut de 1 au nombre de positions de liaison au niveau desquelles un fluor (F) est lié à Ar2,
n3 et n4 sont chacun indépendamment un nombre entier de 0 à 4, et lorsque n3 et n4 valent chacun 2 ou plus, chaque occurrence de R3 et R4 est identique l'une à l'autre ou différente l'une de l'autre,
m3 et m4 sont chacun indépendamment un nombre entier de 1 à 5, n3+m3 vaut 5 ou moins, et n4+m₄ vaut 5 ou moins.

2. Composé selon la revendication 1, dans lequel le groupe durcissable est un quelconque, sélectionné, parmi les structures suivantes, et --- dans les structures suivantes désigne une position de liaison :

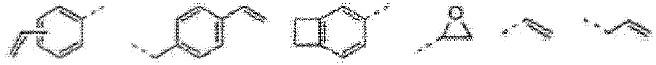

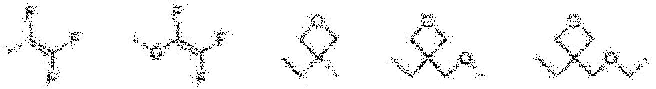

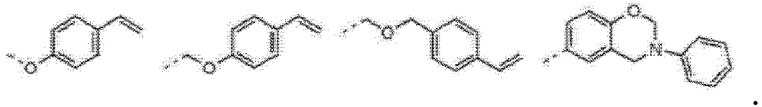

3. Composé selon la revendication 1, dans lequel la Formule chimique 1 ci-dessus est représentée par la Formule chimique 2 suivante : 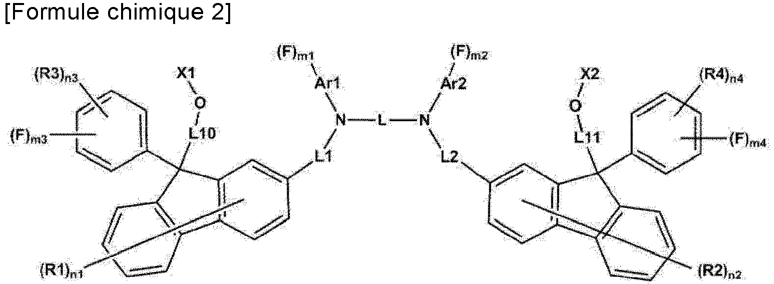 dans la Formule chimique 2,
L, L1, L2, Ar1, Ar2, L10, L11, X1, X2, R1 à R4, n1 à n4, et m1 à m4 sont tels que définis dans la Formule chimique 1.

4. Composé selon la revendication 1, dans lequel la Formule chimique 1 ci-dessus est représentée par la Formule chimique 3 suivante : 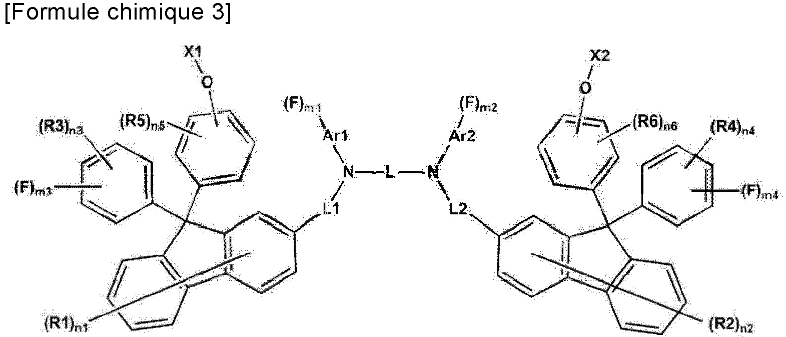 dans la Formule chimique 3,
L, L1, L2, Ar1, Ar2, X1, X2, R1 à R4, n1 à n4, et m1 à m4 sont tels que définis dans la Formule chimique 1,
R5 et R6 sont identiques les uns aux autres ou différents les uns des autres, et sont chacun indépendamment un hydrogène ; un hydrogène lourd ; un groupe halogéné ; un groupe alkyle substitué ou non substitué ; un groupe alcoxy substitué ou non substitué ; un groupe aryle substitué ou non substitué ; ou un groupe hétéroaryle substitué ou non substitué,
n5 et n6 sont chacun indépendamment un nombre entier de 0 à 4, et lorsque n5 et n6 valent chacun 2 ou plus, chaque occurrence de R5 et R6 est identique l'une à l'autre ou différente l'une de l'autre.

5. Composé selon la revendication 1, dans lequel L est un groupe de noyau hydrocarboné divalent en C₆-C₃₀ non substitué ou substitué par un groupe alkyle en C₁-C₂₀.

6. Composé selon la revendication 1, dans lequel L est un quelconque sélectionné parmi les structures suivantes : 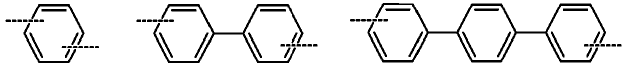 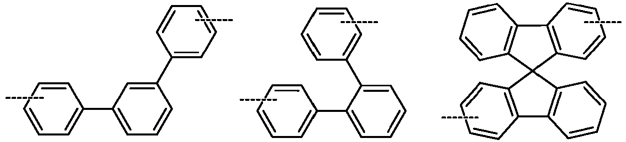 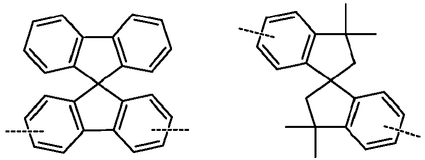
dont chacune est facultativement substituée par un ou plusieurs substituants sélectionnés dans le groupe consistant en un hydrogène lourd ; un groupe halogéné ; un groupe alkyle substitué ou non substitué ; un groupe alcoxy substitué ou non substitué ; un groupe aryle substitué ou non substitué ; et un groupe hétéroaryle substitué ou non substitué, et
... désigne une position de liaison.

7. Composé selon la revendication 1, dans lequel Ar1 et Ar2 sont identiques l'un à l'autre ou différents l'un de l'autre, et sont chacun indépendamment un groupe phényle substitué ou non substitué ; un groupe biphényle substitué ou non substitué ; ou un groupe terphényle substitué ou non substitué.

8. Composé selon la revendication 1, dans lequel m1 et m2 sont chacun indépendamment un nombre entier de 1 à 5.

9. Composé selon la revendication 1, dans lequel la Formule chimique 1 ci-dessus est représentée par l'un quelconque des composés suivants :

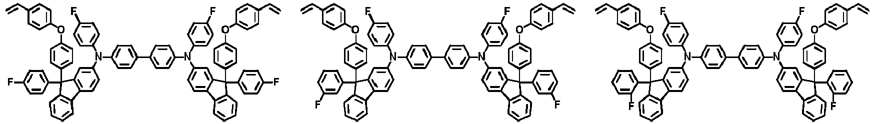

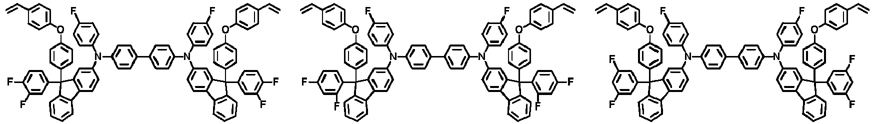

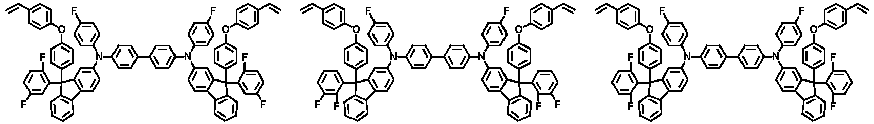

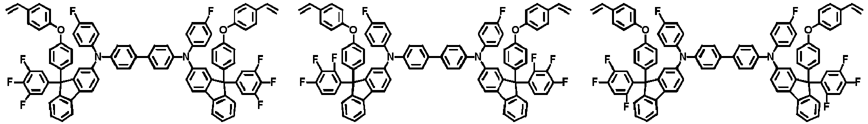

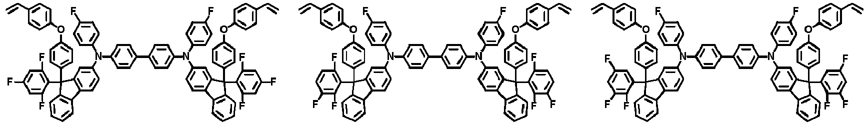

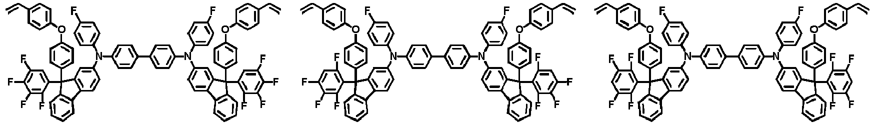

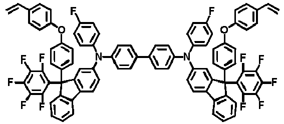

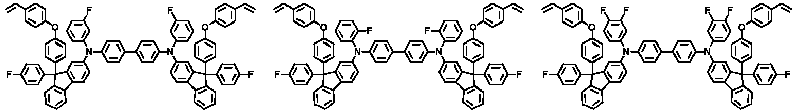

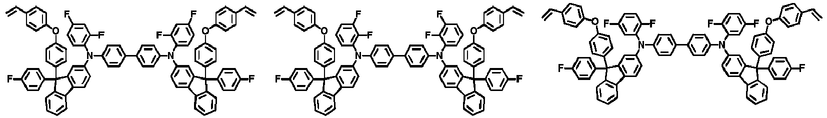

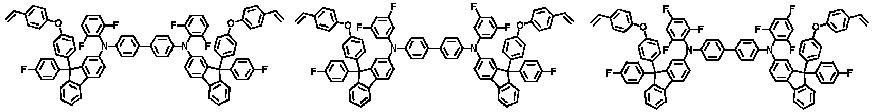

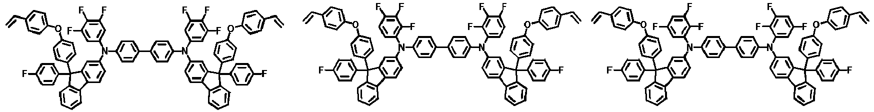

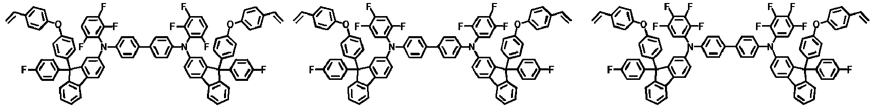

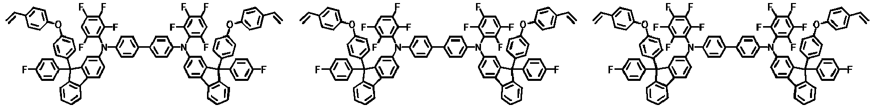

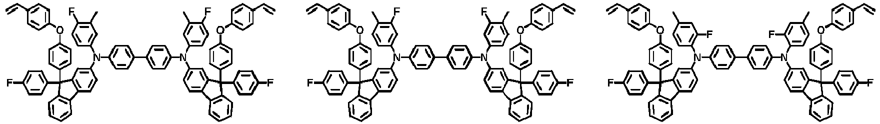

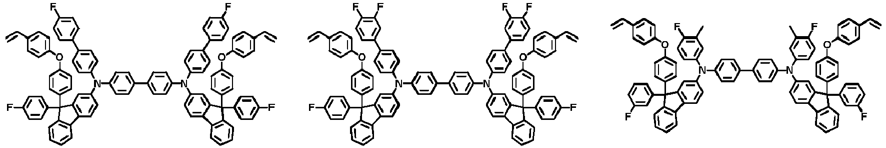

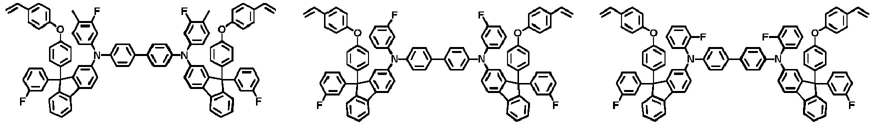

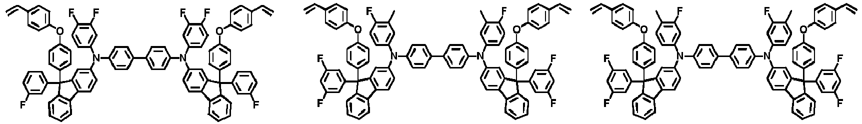

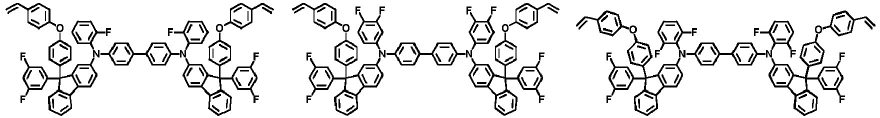

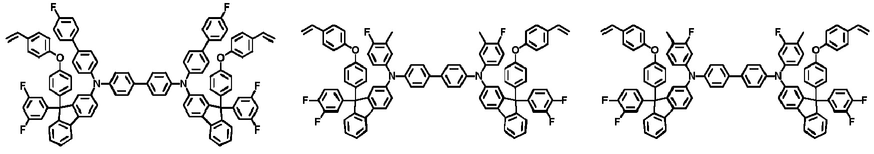

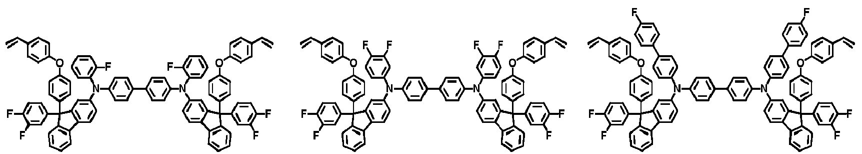

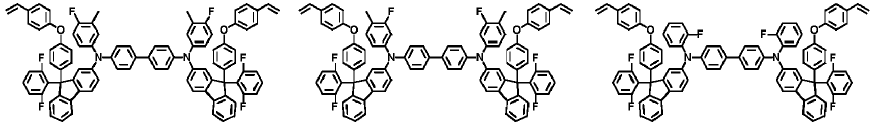

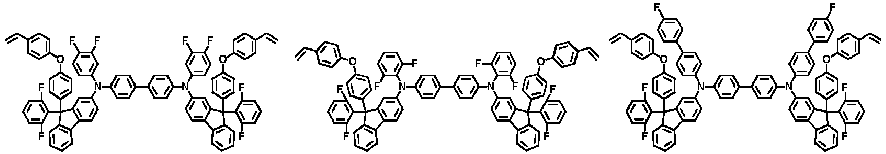

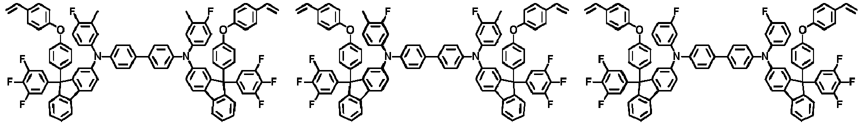

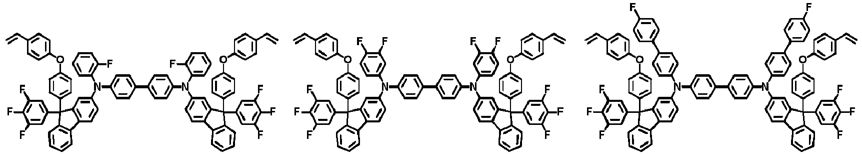

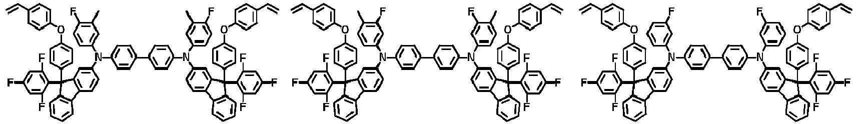

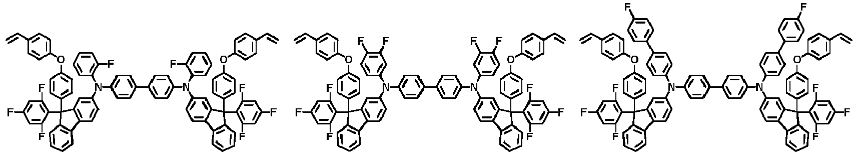

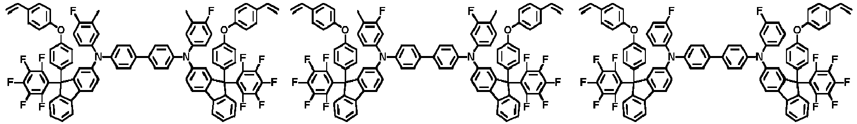

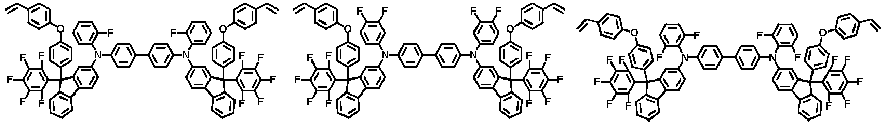

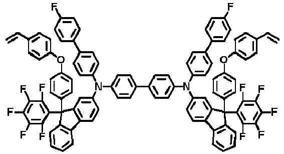

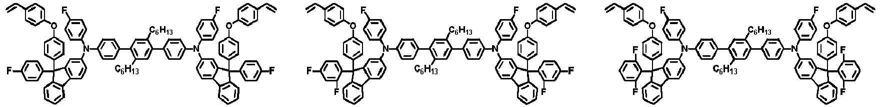

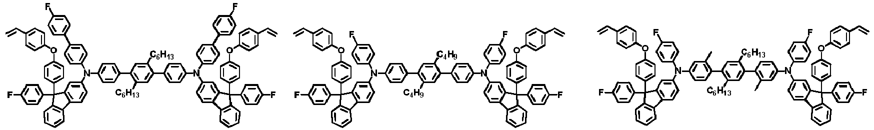

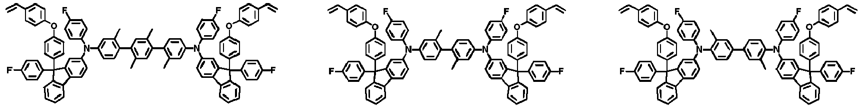

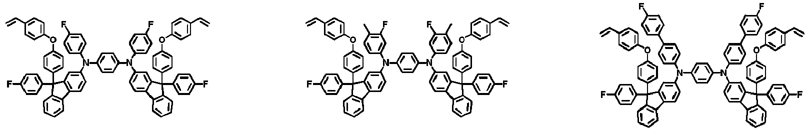

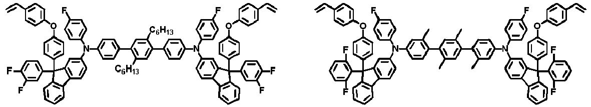

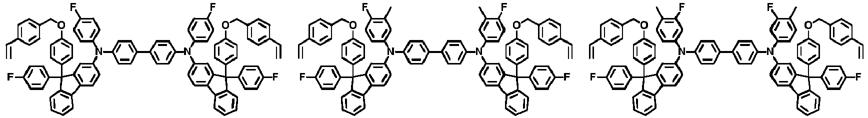

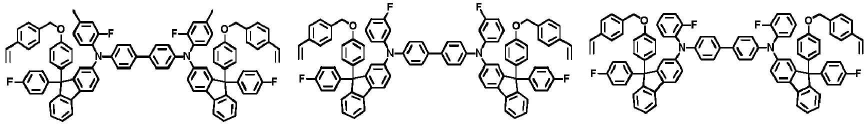

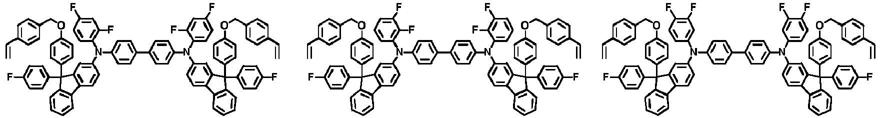

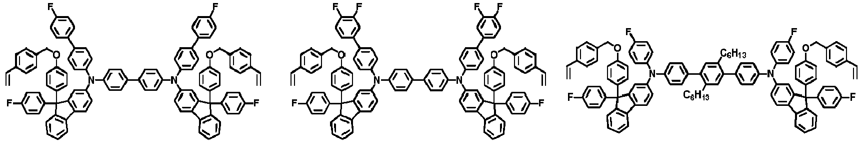

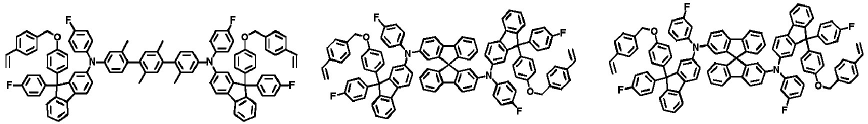

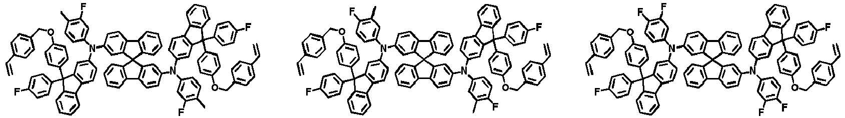

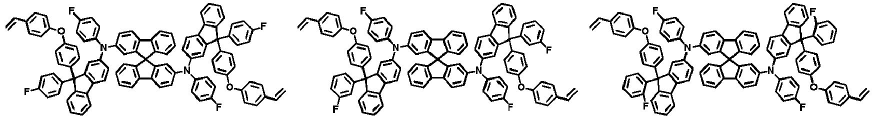

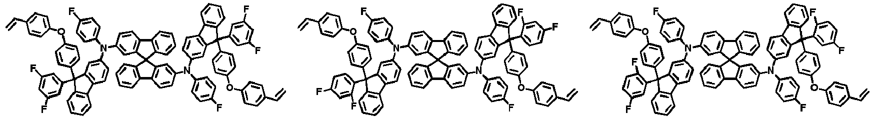

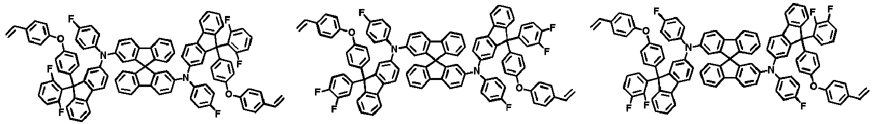

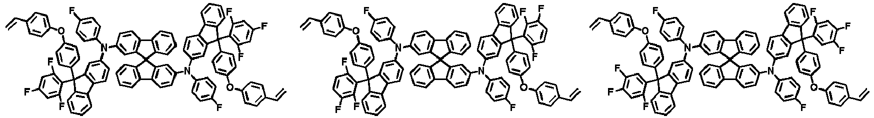

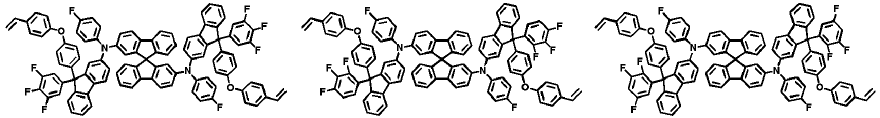

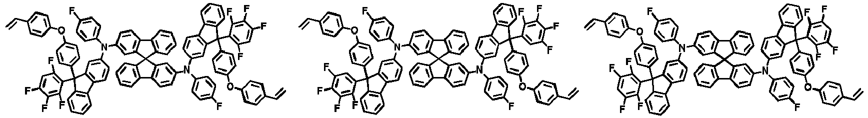

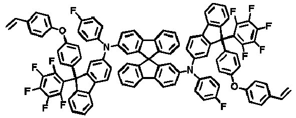

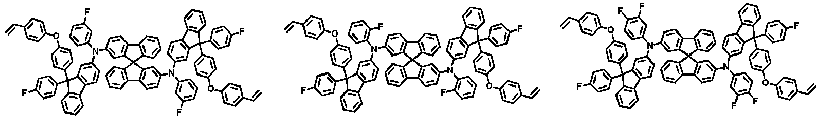

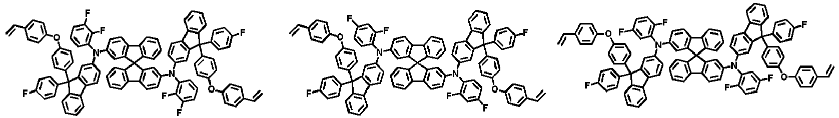

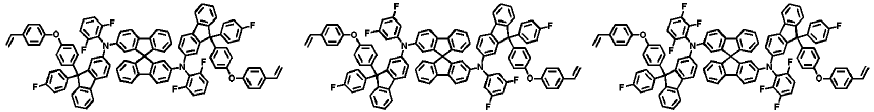

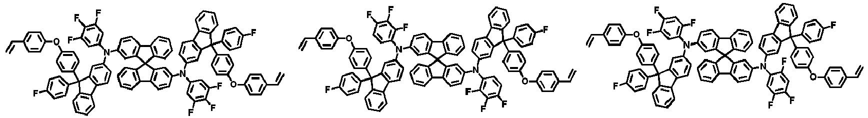

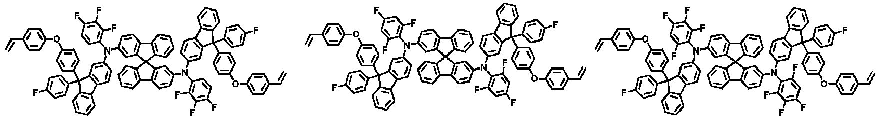

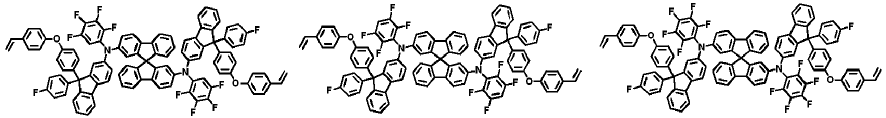

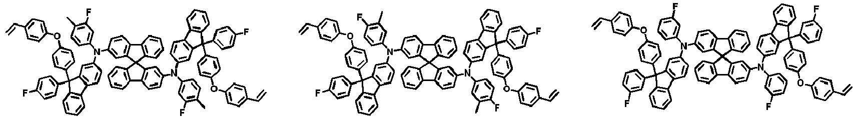

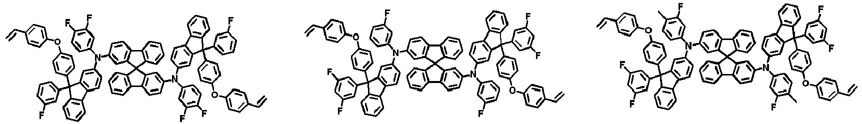

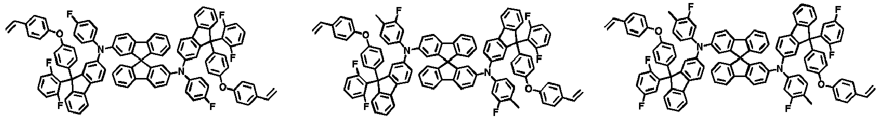

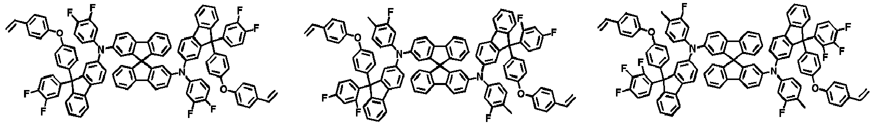

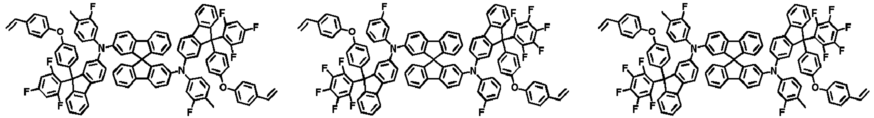

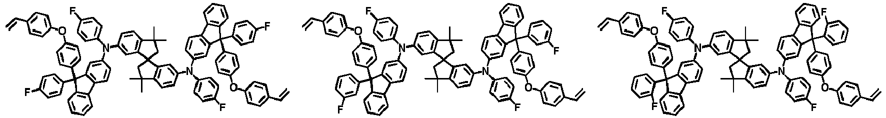

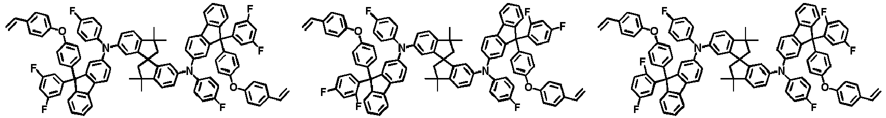

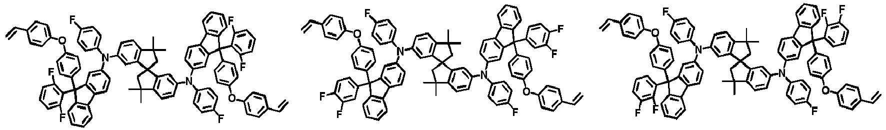

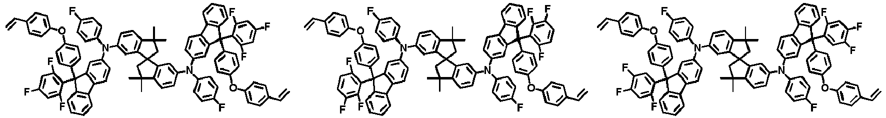

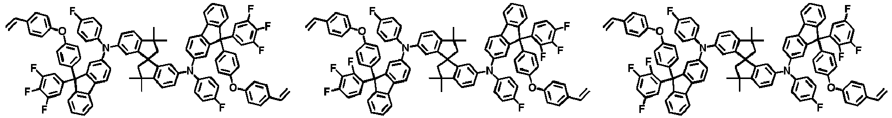

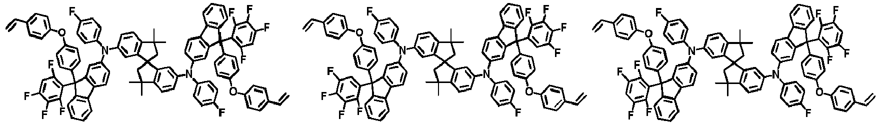

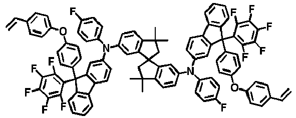

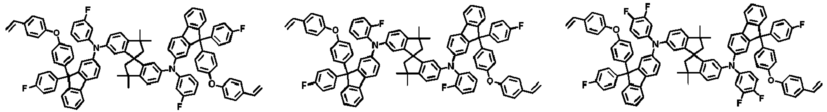

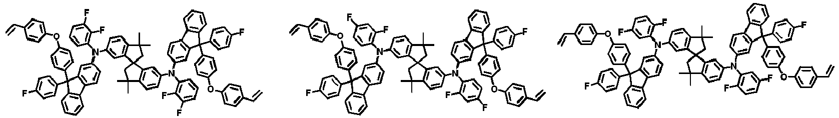

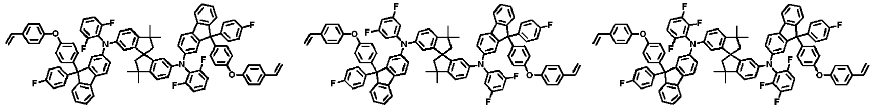

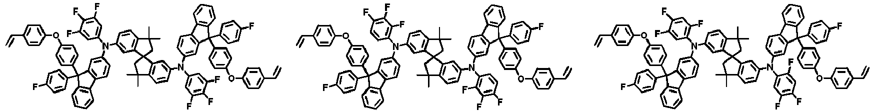

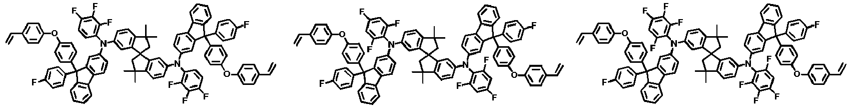

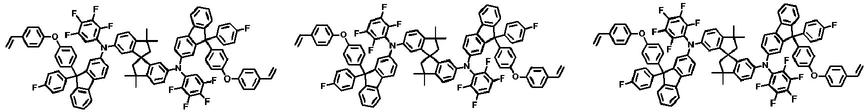

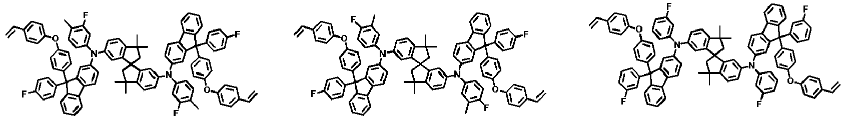

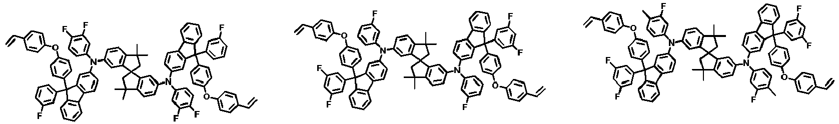

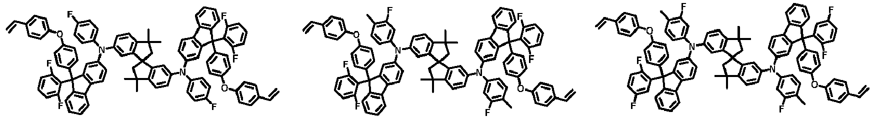

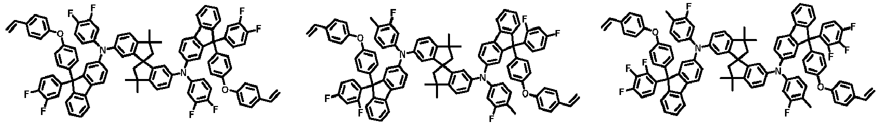

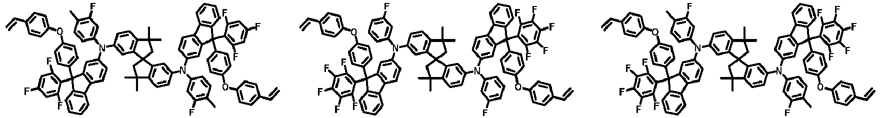

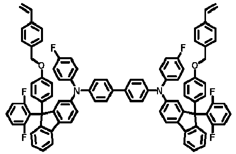

10. Composition de revêtement comprenant le composé selon l'une quelconque des revendications 1 à 9.

11. Dispositif électroluminescent organique comprenant :
une première électrode ;
une deuxième électrode ; et
une couche de matériau organique prévue entre la première électrode et la deuxième électrode,
dans lequel une ou plusieurs couches de la couche de matière organique contiennent la composition de revêtement selon la revendication 10 ou un produit durci de celle-ci.

12. Dispositif électroluminescent organique selon la revendication 11, dans lequel les une ou plusieurs couches contenant la composition de revêtement ou un produit durci de celle-ci sont une couche de transport de trous ou une couche d'injection de trous.

13. Procédé de fabrication d'un dispositif électroluminescent organique, le procédé comprenant les étapes de :
préparation d'une première électrode ;
formation d'une couche de matière organique sur la première électrode ; et
formation d'une deuxième électrode sur la couche de matière organique,
dans lequel l'étape de formation d'une couche de matière organique comprend une étape de formation d'une ou plusieurs couches en utilisant la composition de revêtement selon la revendication 10.

14. Procédé selon la revendication 13, dans lequel l'étape de formation des une ou plusieurs couches en utilisant la composition de revêtement comprend les étapes de : revêtement de la composition de revêtement ; et traitement thermique ou traitement lumineux de la composition de revêtement revêtue.
